# EUROPEAN PATENT SPECIFICATION

(11) **EP 3 107 577 B1**
(45) Date of publication and mention of the grant of the patent: **20.03.2024**
(21) Application number: 15752087.5
(22) Date of filing: 20.01.2015
(51) Int. Cl.: A61K 39/395, C07K 16/00, C07K 16/28, C07K 16/30, A61K 39/00, A61P 35/00, A61P 1/04, A61P 1/18, A61P 11/00, A61P 13/08, A61P 13/10, A61P 13/12, A61P 15/00, A61P 37/04, A61P 43/00

(54) **DISEASE THERAPY BY INDUCING IMMUNE RESPONSE TO TROP-2 EXPRESSING CELLS**
KRANKHEITSTHERAPIE DURCH INDUKTION EINER IMMUNANTWORT GEGEN TROP-2-EXPRIMIERENDE ZELLEN
TRAITEMENT D'UNE MALADIE PAR INDUCTION D'UNE RÉPONSE IMMUNE À DES CELLULES EXPRIMANT LE TROP-2

(30) Priority: 21.02.2014 US 201461942752 P; 12.09.2014 US 201462049826 P
(43) Date of publication of application: 28.12.2016
(73) Proprietor: IBC Pharmaceuticals, Inc., Morris Plains, NJ 07950 (US)
(72) Inventor: CHANG, Chien-Hsing, Morris Plains, New Jersey 07950 (US); GOLDENBERG, David M., Morris Plains, New Jersey 07950 (US); ROSSI, Edmund A., Morris Plains, New Jersey 07950 (US); ROSSI, Diane, Morris Plains, New Jersey 07950 (US)
(74) Representative: Bohmann, Armin K.
(86) International application number: PCT/US2015/012010
(87) International publication number: WO 2015/126548

(56) References cited:
- WO-A1-2014/163684
- WO-A2-2014/028560
- US-A1- 2004 001 825
- US-A1- 2008 213 256
- US-A1- 2011 165 161
- US-A1- 2014 050 660
- E. A. ROSSI ET AL: "Redirected T-Cell Killing of Solid Cancers Targeted with an Anti-CD3/Trop-2-Bispecific Antibody Is Enhanced in Combination with Interferon-", MOLECULAR CANCER THERAPEUTICS, vol. 13, no. 10, 22 July 2014 (2014-07-22) , pages 2341-2351, XP055222114, ISSN: 1535-7163, DOI: 10.1158/1535-7163.MCT-14-0345
- T. M. CARDILLO ET AL: "Humanized Anti-Trop-2 IgG-SN-38 Conjugate for Effective Treatment of Diverse Epithelial Cancers: Preclinical Studies in Human Cancer Xenograft Models and Monkeys", CLINICAL CANCER RESEARCH, vol. 17, no. 10, 3 March 2011 (2011-03-03) , pages 3157-3169, XP055398728, US ISSN: 1078-0432, DOI: 10.1158/1078-0432.CCR-10-2939
- BEUM ET AL.: 'Binding of rituximab, trastuzumab, cetuximab, or mAb T101 to cancer cells promotes trogocytosis mediated by THP-1 cells and monocytes.' J IMMUNOL. vol. 181, no. 11, 01 December 2008, pages 8120 - 8132, XP055072559
- ROSSI ET AL.: 'Redirected T- Cell Killing of Solid Cancers Targeted with an Anti- CD 3/Trop-2 Bispecific Antibody Is Enhanced in Combination with Interferon-alpha.' MOL CANCER THER. vol. 13, no. 10, October 2014, ISSN 1535-7163 pages 2341 - 2351, XP055222114
- CHANG, C-H: CANCER RES, vol. 77, no. 19, 1 October 2017 (2017-10-01), pages 5384-5394,

## Description

### FIELD

The present invention is related to a bispecific antibody for use in a method of treatment by inducing an immune response to a Trop-2 expressing cancer, wherein the method comprises administering the bispecific antibody and a checkpoint inhibitor antibody to a subject with a Trop-2 expressing cancer Preferably, the anti-Trop-2 antibody is a hRS7 antibody. The bispecific antibody redirects effector T cells, monocytes, NK cells or neutrophils to target diseased cells or tissues and induces an immune response against the target.

### BACKGROUND

Use of bispecific antibodies (bsAbs) to redirect effector T cells for the targeted killing of tumor cells has shown considerable promise both pre-clinically and clinically (see, e.g., Topp et al., 2012, Blood 120:5185-87; Bargou et al., 2008, Science 321:974-77). The bispecific antibodies developed to date contain a first binding site specific to CD3 for T-cell recruitment and activation and a second binding site for a targeted disease-associated antigen, such as CD19 (Bassan, 2012, Blood 120:5094-95). The bispecific antibody brings CD3⁺ T cells into direct contact with targeted disease cells and induces cell-mediated cytotoxicity (Bassan, 2012). Anti-CD3 X anti-CD19 bispecific antibodies have been reported to produce a complete and durable molecular remission at very low concentrations in approximately 70% of adult patients with MRD⁺ ALL (Topp et al., 2012, Blood 120:5185-87). Bispecific antibodies recognizing gliomas and the CD3 epitope on T cells have been successfully used in treating brain tumors in human patients (Nitta, et al. Lancet 1990; 355:368-371).

Leukocyte redirecting bsAbs are not limited to T cells. The bispecific killer engagers (BiKEs) comprising scFvs against the NK cell antigen CD16 and a tumor-associated antigen (e.g., CD19, CD22, CD33) have also shown potent anti-cancer activity (e.g., Miller, Hematology Soc Hematol Educ Pogram 2013:247-53). Other alternatives include trispecific killer engagers (TriKEs), such as anti-CD 16 x anti-CD 19 x anti-CD22 (Miller, 2013; Gleason et al., 2012, Mol Cancer Ther 11:2674-84). An anti-CD16 x anti-CD33 BiKE was used to treat AML and myelodysplastic syndrome (Miller, 2013; Wiernik et al., 2013, Clin Cancer Res 19:3844-55). In refractory AML, a CD16 x CD33 BiKE led to potent tumor cell killing and cytokine production by NK cells. Inhibition of ADAM17 enhanced the CD16 x CD33 BiKE response (Miller, 2013). Other trispecific, trivalent constructs, for example against CD16/CD19/HLA-DR, have been reported (Schubert et al., 2012, mAbs 4:45-56).

Numerous methods to produce bispecific antibodies are known (see, e.g. U.S. Patent No. 7,405,320). Bispecific antibodies can be produced by the quadroma method, which involves the fusion of two different hybridomas, each producing a monoclonal antibody recognizing a different antigenic site (Milstein and Cuello, Nature 1983; 305:537-540). The fused hybridomas are capable of synthesizing two different heavy chains and two different light chains, which can associate randomly to give a heterogeneous population of 10 different antibody structures of which only one of them, amounting to 1/8 of the total antibody molecules, will be bispecific, and therefore must be further purified from the other forms. Fused hybridomas are often less stable cytogenetically than the parent hybridomas, making the generation of a production cell line more problematic.

Another method for producing bispecific antibodies uses heterobifunctional crosslinkers to chemically tether two different monoclonal antibodies, so that the resulting hybrid conjugate will bind to two different targets (Staerz, et al. Nature 1985; 314:628-631; Perez, et al. Nature 1985; 316:354-356). Bispecific antibodies generated by this approach are essentially heteroconjugates of two IgG molecules, which diffuse slowly into tissues and are rapidly removed from the circulation. Bispecific antibodies can also be produced by reduction of each of two parental monoclonal antibodies to the respective half molecules, which are then mixed and allowed to reoxidize to obtain the hybrid structure (Staerz and Bevan. Proc Natl Acad Sci USA 1986; 83:1453-1457). An alternative approach involves chemically cross-linking two or three separately purified Fab' fragments using appropriate linkers. All these chemical methods are undesirable for commercial development due to high manufacturing cost, laborious production process, extensive purification steps, low yields (<20%), and heterogeneous products.

Discrete V_{H} and V_{L} domains of antibodies produced by recombinant DNA technology may pair with each other to form a dimer (recombinant Fv fragment) with binding capability (U.S. Pat. No. 4,642,334). However, such non-covalently associated molecules are not sufficiently stable under physiological conditions to have any practical use. Cognate V_{H} and V_{L} domains can be joined with a peptide linker of appropriate composition and length (usually consisting of more than 12 amino acid residues) to form a single-chain Fv (scFv) with binding activity. Methods of manufacturing scFv-based agents of multivalency and multi specificity by varying the linker length were disclosed in U.S. Pat. No. 5,844,094, U.S. Pat. No. 5,837,242 and WO 98/44001. Common problems that have been frequently associated with generating scFv-based agents of multivalency and multi specificity are low expression levels, heterogeneous products, instability in solution leading to aggregates, instability in serum, and impaired affinity.

Several bispecific antibodies targeting CD3 and CD19 are in clinical development. An scFv-based bispecific antibody construct, known as BITE^{®} (Bispecific T-cell Engager), employs a single polypeptide containing 2 antigen-binding specificities, each contributed by a cognate VH and VL, linked in tandem via a flexible linker (see, e.g., Nagorsen et al., 2009, Leukemia & Lymphoma 50:886-91; Amann et al., 2009, J Immunother 32:453-64; Baeuerle and Reinhardt, 2009, Cancer Res 69:4941-44). Another bispecific antibody called DART^{®} (Dual-Affinity Re-Targeting) utilizes a disulfide-stabilized diabody design (see, e.g., Moore et al., 2011, Blood 117:4542-51; Veri et al., 2010, Arthritis Rheum 62:1933-43). Both BITE^{®} and DART^{®} exhibit fast blood clearance due to their small size (~55 kDa), which requires frequent administration to maintain therapeutic levels of the bispecific antibodies.

Interferons are critical role players in the antitumor and antimicrobial host defense, and have been extensively explored as therapeutic agents for cancer and infectious disease (Billiau et al., 2006, Cytokine Growth Factor Rev 17:381-409; Pestka et al., 2004, Immunol Rev 202:8-32). Despite considerable efforts with type I and II interferons (IFN-α/β and γ), their use in clinic settings have been limited because of the short circulation half-life, systemic toxicity, and suboptimal responses in patients (Pestka et al., 2004, Immunol Rev 202:8-32; Miller et al., 2009, Ann N Y Acad Sci 1182:69-79). The discovery of the IFN-λ family in early 2003 brought an exciting new opportunity to develop alternative IFN agents for these unmet clinical indications (Kotenko et al., 2003, Nat Immunol 4:69-77; Sheppard et al., 2003, Nat Immunol 4:63-8).

The therapeutic effectiveness of IFNs has been validated to date by the approval of IFN-α2 for treating hairy cell leukemia, chronic myelogenous leukemia, malignant melanoma, follicular lymphoma, condylomata acuminata, AIDs-related Kaposi sarcoma, and chronic hepatitis B and C; IFN-β for treating multiple sclerosis; and IFN-γ for treating chronic granulomatous disease and malignant osteopetrosis. Despite a vast literature on this group of autocrine and paracrine cytokines, their functions in health and disease are still being elucidated, including more effective and novel forms being introduced clinically (Pestka, 2007, J. Biol. Chem 282:20047-51; Vilcek, 2006, Immunity 25:343-48). The effects of combination of various interferons with antibody-based therapies also remain under investigation.

Antibody-drug conjugates (ADCs) are a potent class of therapeutic constructs that allow targeted delivery of cytotoxic agents to target cells, such as cancer cells. Because of the targeting function, these compounds show a much higher therapeutic index compared to the same systemically delivered agents. ADCs have been developed as intact antibodies or antibody fragments, such as scFvs. The antibody or fragment is linked to one or more copies of drug via a linker that is stable under physiological conditions, but that may be cleaved once inside the target cell. ADCs approved for therapeutic use include gemtuzumab ozogamicin for AML (subsequently withdrawn from the market), brentuximab vedotin for ALCL and Hodgkin lymphoma, and trastuzumab emtansine for HER2-positive metastatic breast cancer (Verma et al., 2012, N Engl J Med 367: 1783-91; Bross et al., 2001, Clin Cancer Res 7:1490-96; Francisco et al., 2003, Blood 102:1458-65). Numerous other candidate ADCs are currently in clinical testing, such as inotuzumab ozogamicin (Pfizer), glembatumomab vedotin (Celldex Therapeutics), SAR3419 (Sanofi-Aventis), SAR56658 (Sanofi-Aventis), AMG-172 (Amgen), AMG-595 (Amgen), BAY-94-9343 (Bayer), BIIB015 (Biogen Idec), BT062 (Biotest), SGN-75 (Seattle Genetics), SGN-CD19A (Seattle Genetics), vorsetuzumab mafodotin (Seattle Genetics), ABT-414 (AbbVie), ASG-SME (Agensys), ASG-22ME (Agensys), ASG-16M8F (Agensys), IMGN-529 (ImmunoGen), IMGN-853 (ImmunoGen), MDX-1203 (Medarex), MLN-0264 (Millenium), RG-7450 (Roche/Genentech), RG-7458 (Roche/Genentech), RG-7593 (Roche/Genentech), RG-7596 (Roche/Genentech), RG-7598 (Roche/Genentech), RG-7599 (Roche/Genentech), RG-7600 (Roche/Genentech), RG-7636 (Roche/Genentech), anti-PSMA ADC (Progenics), lorvotuzumab mertansine (ImmunoGen), milatuzumab-doxorubicin (Immunomedics), IMMU-130 (Immunomedics), IMMU-132 (Immunomedics) and antibody conjugates of pro-2-pyrrolinodoxorubicin. (See, e.g., Li et al., 2013, Drug Disc Ther 7:178-84; Firer & Gellerman, J Hematol Oncol 5:70; Beck et al., 2010, Discov Med 10:329-39; Mullard, 2013, Nature Rev Drug Discovery 12:329, Provisional U.S. Patent Application 61/761,845.) Because of the potential of ADCs to act as potent anti-cancer agents with reduced systemic toxicity, they may be used either alone or as an adjunct therapy to reduce tumor burden.

Another promising approach to immunotherapy concerns use of antagonistic antibodies against immune checkpoint proteins (e.g., Pardoll, 2012, Nature Reviews Cancer 12:252-64). Immune checkpoints function as endogenous inhibitory pathways for immune system function that act to maintain self-tolerance and to modulate the duration and extent of immune response to antigenic stimulation (Pardoll, 2012). However, it appears that tumor tissues and possibly certain pathogens may co-opt the checkpoint system to reduce the effectiveness of host immune response, resulting in tumor growth and/or chronic infection (see, e.g., Pardoll, 2012, Nature Reviews Cancer 12:252-64; Nirschl & Drake, 2013, Clin Cancer Res 19:4917-24). Checkpoint molecules include CTLA4 (cytotoxic T lymphocyte antigen-4), PD1 (programmed cell death protein 1), PD-L1 (programmed cell death ligand 1), LAG-3 (lymphocyte activation gene-3), TIM-3 (T cell immunoglobulin and mucin protein-3) and several others (Pardoll, 2012, Nature Reviews Cancer 12:252-64; Nirschl & Drake, 2013, Clin Cancer Res 19:4917-24). Antibodies against several of the checkpoint proteins (CTLA4, PD1, PD-L1) are in clinical trials and has shown unexpected efficacy againts tumors that were resistant to standard treatments.

WO 2014/163684 A1 and WO 2014/028560 are related to a combination therapy for inducing immune response to a disease. Suitable agents may include leukocyte redirecting complexes, antibody-drug conjugates, interferons and/or checkpoint inhibitor antibodies.

Chang C-H et al. (Cancer Res; 77(19) October 1, 2017, pp. 5384-5394) report on combination therapy with bispecific antibodies and PD-1 blockade which enhances the antitumor potency of T cells.

A need exists for methods and compositions to generate improved bispecific antibody complexes with longer T_{1/2}, better pharmacokinetic properties, increased *in vivo* stability and/or improved *in vivo* efficacy. A further need exists for combination therapies to improve efficacy of treatments directed to inducing immune response against various diseases, such as Trop-2⁺ cancer.

### SUMMARY

The problem underlying the present invention is solved by the subject matter of the attached independent claim. Preferred embodiments may be taken from the attached dependent claims.

More specifically, the problem underlying the present invention is solved by a bispecific antibody for use in a method of treatment by inducing an immune response to a Trop-2 expressing cancer, wherein the method comprises administering the bispecific antibody and a checkpoint inhibitor antibody to a subject with a Trop-2 expressing cancer, wherein the bispecific antibody comprises a first antibody fragment and a second antibody fragment, wherein both the first antibody fragment and the second antibody fragment are each an scFv and, wherein the bispecific antibody comprises one binding site for Trop-2 and one binding site for CD3, and wherein the bispecific antibody comprises the amino acid sequence of SEQ ID NO: 107 binding to Trop-2.

In an embodiment, the method further comprises administering to the subject at least one therapeutic agent selected from the group consisting of (i) an interferon selected from the group consisting of interferon-α, interferon-β, interferon-λ1, interferon-λ2 and interferon-λ3; and (ii) an antibody-drug conjugate (ADC).

In a preferred embodiment, the interferon is interferon-α.

In an embodiment, the checkpoint inhibitor antibody is selected from the group consisting of lambrolizumab (MK-3475), nivolumab (BMS-936558), pidilizumab (CT-011), AMP-224, MDX-1105, MEDI4736, MPDL3280A, BMS-936559, ipilimumab, lirlumab, IPH2101 and tremelimumab.

In an embodiment, the checkpoint inhibitor antibody binds to an antigen selected from the group consisting of CTLA4, PD1, PD-L1, LAG3, B7-H3, B7-H4, KIR and TIM3.

In an embodiment, the antibody-drug conjugate is selected from the group consisting of hLL1-doxorubicin, hRS7-SN-38, hMN-14-SN-38, hLL2-SN-38, hA20-SN-38, hPAM4-SN-38, hLL1-SN-38, hRS7-Pro-2-P-Dox, hMN-14-Pro-2-P-Dox, hLL2-Pro-2-P-Dox, hA20-Pro-2-P-Dox, hPAM4-Pro-2-P-Dox, hLL1-Pro-2-P-Dox, P4/D10-doxorubicin, gemtuzumab ozogamicin, brentuximab vedotin, trastuzumab emtansine, inotuzumab ozogamicin, glembatumomab vedotin, SAR3419, SAR566658, BIIB015, BT062, SGN-75, SGN-CD19A, AMG-172, AMG-595, BAY-94-9343, ASG-5ME, ASG-22ME, ASG-16M8F, MDX-1203, MLN-0264, anti-PSMA ADC, RG-7450, RG-7458, RG-7593, RG-7596, RG-7598, RG-7599, RG-7600, RG-7636, ABT-414, IMGN-853, IMGN-529, vorsetuzumab mafodotin, and lorvotuzumab mertansine.

In an embodiment, an ADC is administered prior to any other agent.

In an embodiment, the interferon is administered as free interferon, PEGylated interferon, an interferon fusion protein or interferon conjugated to an antibody.

In an embodiment, the Trop-2 expressing cancer is a carcinoma of the esophagus, pancreas, lung, stomach, colon, rectum, urinary bladder, breast, ovary, uterus, kidney or prostate.

In an embodiment, the method further comprises administering to the subject a therapeutic agent selected from the group consisting of a second antibody or antigen-binding fragment thereof, a drug, a toxin, an enzyme, a cytotoxic agent, an anti-angiogenic agent, a pro-apoptotic agent, an antibiotic, a hormone, an immunomodulator, a cytokine, a chemokine, an antisense oligonucleotide, a small interfering RNA (siRNA), a boron compound and a radioisotope.

In an embodiment, the bispecific antibody induces an immune response to a Trop-2 expressing cancer without increasing cytokine production to levels capable of inducing cytokine release syndrome (CRS).

The present invention as defined in the claims relates to bispecific antibodies for use in the treatment of Trop-2⁺ cancer cells by inducing an immune response to said Trop-2⁺ cancer cells.. Trop-2 is overexpressed in numerous types of solid tumors, such as carcinomas of the esophagus, pancreas, lung, stomach, colon and rectum, urinary bladder, breast, ovary, uterus, cervix, kidney and prostate. Preferably, the bispecific antibody is of use to treat gastric cancer or pancreatic cancer. Administration of the bispecific antibody induces an immune response to cells that are Trop-2⁺. Although Trop-2 is also expressed in some normal tissues (e.g., Stepan et al., 2011, J Histochem Cytochem 59:701-10), the Examples below demonstrate that anti-Trop-2 antibodies may be administered *in vivo* in both animal model systems and human subjects, with only tolerable toxicities. In other preferred embodiments, administration of bispecific antibody to a subject induces an immune response against Trop-2⁺ cancer cells without elevating levels of cytokines that would induce cytokine release syndrome (CRS). In alternative preferred embodiments, the bispecific antibody induces trogocytosis of cell surface antigens between Trop-2⁺ cancer cells and T cells.

Also disclosed is a the bispecific antibody targeting besides CD3 other T cell or leukocyte antigens. Exemplary T-cell antigens are selected from the group consisting of CD2, CD3, CD4, CD5, CD6, CD8, CD25, CD28, CD30, CD40, CD40L, CD44, CD45, CD69 and CD90. Exemplary antigens expressed on NK cells are selected from the group consisting of CD8, CD16, CD56, CD57, ADAM17, KIR and CD137. Exemplary monocyte antigens are selected from the group consisting of CD74, HLA-DR alpha chain, CD14, CD16, CD64 and CD89. Exemplary neutrophil antigens are selected from the group consisting of CEACAM6, CEACAM8, CD16b, CD32a, CD89, CD177, CD11a, CD11b and SLC44A2. Preferably the T-cell antigen is CD3, or the NK cell antigen is CD16.

Also disclosed is a bispecific antibody targeting besides Trop-2 other tumor-associated antigens. Tumor-associated antigens that may be targeted include, but are not limited to, alpha-fetoprotein (AFP), α-actinin-4, A3, antigen specific for A33 antibody, ART-4, B7, Ba 733, BAGE, BrE3-antigen, CA125, CAMEL, CAP-1, carbonic anhydrase IX, CASP-8/m, CCCL19, CCCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD44, CD45, CD46, CD52, CD54, CD55, CD59, CD64, CD66a-e, CD67, CD70, CD70L, CD74, CD79a, CD79b, CD80, CD83, CD95, CD126, CD132, CD133, CD138, CD147, CD154, CDC27, CDK-4/m, CDKN2A, CTLA4, CXCR4, CXCR7, CXCL12, HIF-1α, colon-specific antigen-p (CSAp), CEA (CEACAM5), CEACAM6, c-Met, DAM, EGFR, EGFRvIII, EGP-1 (TROP-2), EGP-2, ELF2-M, Ep-CAM, fibroblast growth factor (FGF), Flt-1, Flt-3, folate receptor, G250 antigen, GAGE, gp100, GRO-β, HLA-DR, HM1.24, human chorionic gonadotropin (HCG) and its subunits, HER2/neu, HMGB-1, hypoxia inducible factor (HIF-1), HSP70-2M, HST-2, Ia, IGF-1R, IFN-γ, IFN-α, IFN-β, IFN-λ, IL-4R, IL-6R, IL-13R, IL-15R, IL-17R, IL-18R, IL-2, IL-6, IL-8, IL-12, IL-15, IL-17, IL-18, IL-23, IL-25, insulin-like growth factor-1 (IGF-1), KC4-antigen, KS-1-antigen, KS1-4, Le-Y, LDR/FUT, macrophage migration inhibitory factor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5ac, MUC13, MUC16, MUM-1/2, MUM-3, NCA66, NCA95, NCA90, PAM4 antigen, pancreatic cancer mucin, PD1 receptor, placental growth factor, p53, PLAGL2, prostatic acid phosphatase, PSA, PRAME, PSMA, PlGF, ILGF, ILGF-1R, IL-6, IL-25, RS5, RANTES, T101, SAGE, S100, survivin, survivin-2B, TAC, TAG-72, tenascin, TRAIL receptors, TNF-α, Tn antigen, Thomson-Friedenreich antigens, tumor necrosis antigens, VEGFR, ED-B fibronectin, WT-1, 17-1A-antigen, complement factors C3, C3a, C3b, C5a, C5, an angiogenesis marker, bcl-2, bcl-6, Kras, an oncogene marker and an oncogene product (see, e.g., Sensi et al., Clin Cancer Res 2006, 12:5023-32; Parmiani et al., J Immunol 2007, 178:1975-79; Novellino et al. Cancer Immunol Immunother 2005, 54:187-207).

Exemplary anti-TAA antibodies that may be used include, but are not limited to, hA19 (anti-CD19, U.S. Patent No. 7,109,304), hR1 (anti-IGF-1R, U.S. Patent Application Serial No. 12/722,645, filed 3/12/10), hPAM4 (anti-MUC5ac, U.S. Patent No. 7,282,567), hA20 (anti-CD20, U.S. Patent No. 7,251,164), hIMMU31 (anti-AFP, U.S. Patent No. 7,300,655), hLL1 (anti-CD74, U.S. Patent No. 7,312,318), hLL2 (anti-CD22, U.S. Patent No. 7,074,403), hMu-9 (anti-CSAp, U.S. Patent No. 7,387,773), hL243 (anti-HLA-DR, U.S. Patent No. 7,612,180), hMN-14 (anti-CEACAM5, U.S. Patent No. 6,676,924), hMN-15 (anti-CEACAM6, U.S. Patent No. 7,541,440), hRS7 (anti-EGP-1, U.S. Patent No. 7,238,785), hMN-3 (anti-CEACAM6, U.S. Patent No. 7,541,440), Ab124 and Ab125 (anti-CXCR4, U.S. Patent No. 7,138,496).

Alternative antibodies that may be used for treatment of various disease states include, but are not limited to, abciximab (anti-glycoprotein IIb/IIIa), alemtuzumab (anti-CD52), bevacizumab (anti-VEGF), cetuximab (anti-EGFR), gemtuzumab (anti-CD33), ibritumomab (anti-CD20), panitumumab (anti-EGFR), rituximab (anti-CD20), tositumomab (anti-CD20), trastuzumab (anti-ErbB2), lambrolizumab (anti-PD1 receptor), nivolumab (anti-PD1 receptor), ipilimumab (anti-CTLA4), abagovomab (anti-CA-125), adecatumumab (anti-EpCAM), atlizumab (anti-IL-6 receptor), benralizumab (anti-CD125), obinutuzumab (GA101, anti-CD20), CC49 (anti-TAG-72), AB-PG1-XG1-026 (anti-PSMA, U.S. Patent Application 11/983,372, deposited as ATCC PTA-4405 and PTA-4406), D2/B (anti-PSMA, WO 2009/130575), tocilizumab (anti-IL-6 receptor), basiliximab (anti-CD25), daclizumab (anti-CD25), efalizumab (anti-CD11a), GA101 (anti-CD20; Glycart Roche), atalizumab (anti-α4 integrin), omalizumab (anti-IgE); anti-TNF-α antibodies such as CDP571 (Ofei et al., 2011, Diabetes 45:881-85), MTNFAI, M2TNFAI, M3TNFAI, M3TNFABI, M302B, M303 (Thermo Scientific, Rockford, IL), infliximab (Centocor, Malvern, PA), certolizumab pegol (UCB, Brussels, Belgium), anti-CD40L (UCB, Brussels, Belgium), adalimumab (Abbott, Abbott Park, IL), BENLYSTA^{®} (Human Genome Sciences); anti-CD38 antibodies such as MOR03087 (MorphoSys AG), MOR202 (Celgene), HuMax-CD38 (Genmab) or daratumumab (Johnson & Johnson).

An exemplary design for a leukocyte redirecting bsAb disclosed in the Examples below combined an anti-CD3 scFv with an anti-CD19 F(ab)₂ to form a construct designated (19)-3s, which specifically targeted B cells. Other bsAbs combining anti-CD3 with antibody fragments against other tumor-associated antigens, discussed in more detail below, are of use in targeted leukocyte immunotherapy of various solid tumors. The advantages of this design include bivalent binding to tumor cells, a larger size (~130 kDa) to preclude rapid renal clearance, and potent leukocyte mediated cytotoxicity. The bsAbs mediate the formation of immunological synapses between leukocytes and cognate target cells, induce leukocyte activation and proliferation in the presence of target cells, redirect potent leukocyte mediated killing of target cells *in vitro* and inhibit growth of human tumors *in vivo.*

Leukocyte redirecting bispecific antibodies may be produced as trivalent DNL^{™} complexes, with longer T_{1/2}, better pharmacokinetic properties and increased *in vivo* stability. Methods for production and use of DNL^{™} complexes, comprising dimers of DDD moieties from human PKA regulatory subunits RIα, RIβ, RIIα or RIIβ, bound to AD moieties from AKAPs, are well known (see, e.g., U.S. Patent Nos. 7,550,143; 7,521,056; 7,534,866; 7,527,787; 7,666,400; 7,906,118; 7,901,680; 8,003,111 and 8,034,352.) By attaching different effector moieties, such as antibodies or antibody fragments, to the DDD and AD moieties, DNL^{™} complexes comprising virtually any combination of effectors may be constructed and used.

The antibodies can be of various isotypes, preferably human IgG1, IgG2, IgG3 or IgG4, more preferably comprising human IgG1 hinge and constant region sequences. The antibodies or fragments thereof can be chimeric human-mouse, humanized (human framework and murine hypervariable (CDR) regions), or fully human, as well as variations thereof, such as half-IgG4 antibodies (referred to as "unibodies"), as described by van der Neut Kolfschoten et al. (Science 2007; 317:1554-1557). The antibodies or fragments thereof may be designed or selected to comprise human constant region sequences that belong to specific allotypes, which may result in reduced immunogenicity when administered to a human subject. Preferred allotypes for administration include a non-G1m1 allotype (nG1m1), such as G1m3, G1m3,1, G1m3,2 or G1m3,1,2. More preferably, the allotype is selected from the group consisting of the nG1m1, G1m3, nG1m1,2 and Km3 allotypes.

According to the present invention as defined in the claims, the method comprises administering a checkpoint inhibitor antibody. Such antibody is antagonistic for checkpoint inhibitor function. Many such antibodies are known in the art, such as lambrolizumab (MK-3475, Merck), nivolumab (BMS-936558, Bristol-Myers Squibb), pidilizumab (CT-011, CureTech Ltd.), AMP-224 (Merck), MDX-1105 (Medarex), MEDI4736 (MedImmune), MPDL3280A (Genentech), BMS-936559 (Bristol-Myers Squibb), ipilimumab (Bristol-Myers Squibb) and tremelimumab (Pfizer). Anti-KIR antibodies such as lirlumab (Innate Pharma) and IPH2101 (Innate Pharma) may perform similar functions in NK cells. Any known checkpoint inhibitor antibody may be used in combination with one or more of the other agents. Combination therapy with immunostimulatory antibodies has been reported to enhance efficacy, for example against tumor cells. Morales-Kastresana et al. (2013, Clin Cancer Res 19:6151-62) showed that the combination of anti-PD-L1 (10B5) antibody with anti-CD137 (1D8) and anti-OX40 (OX86) antibodies provided enhanced efficacy in a transgenic mouse model of hepatocellular carcinoma. Combination of anti-CTLA4 and anti-PD1 antibodies has also been reported to be highly efficacious (Wolchok et al., 2013, N Engl J Med 369:122-33). Combination of rituximab with anti-KIR antibody, such as lirlumab (Innate Pharma) or IPH2101 (Innate Pharma), was also more efficacious against hematopoietic tumors (Kohrt et al., 2012). The person of ordinary skill will realize that the subject combination therapy may include combinations with multiple antibodies that are immunostimulatory, anti-tumor or anti-infectious agent.

Another agent that may be used in the practicing of the invention as defined in the claims is an interferon. Interferons of use are known in the art and may include interferon-α, interferon-β, interferon-λ1, interferon-λ2 or interferon-λ3. Preferably, the interferon is interferon-α. The subject interferon may be administered as free interferon, PEGylated interferon, an interferon fusion protein or interferon conjugated to an antibody.

In alternative embodiments of the invention as defined in the claims, one or more of the immunomodulatory agents discussed above may be used in combination with an antibody-drug conjugate (ADC). ADCs are particularly effective for reducing tumor burden without significant systemic toxicity and may act to improve the effectiveness of the immune response induced by leukocyte retargeting bsAb, interferon and/or checkpoint inhibitor antibody. Exemplary ADCs of use may include ADCs approved for therapeutic use include gemtuzumab ozogamicin for AML (subsequently withdrawn from the market), brentuximab vedotin for ALCL and Hodgkin lymphoma, and trastuzumab emtansine for HER2-positive metastatic breast cancer (Verma et al., 2012, N Engl J Med 367:1783-91; Bross et al., 2001, Clin Cancer Res 7:1490-96; Francisco et al., 2003, Blood 102:1458-65). Numerous other candidate ADCs are currently in clinical testing, such as inotuzumab ozogamicin (Pfizer), glembatumomab vedotin (Celldex Therapeutics), SAR3419 (Sanofi-Aventis), SAR56658 (Sanofi-Aventis), AMG-172 (Amgen), AMG-595 (Amgen), BAY-94-9343 (Bayer), BIIB015 (Biogen Idec), BT062 (Biotest), SGN-75 (Seattle Genetics), SGN-CD19A (Seattle Genetics), vorsetuzumab mafodotin (Seattle Genetics), ABT-414 (AbbVie), ASG-5ME (Agensys), ASG-22ME (Agensys), ASG-16M8F (Agensys), IMGN-529 (ImmunoGen), IMGN-853 (ImmunoGen), MDX-1203 (Medarex), MLN-0264 (Millenium), RG-7450 (Roche/Genentech), RG-7458 (Roche/Genentech), RG-7593 (Roche/Genentech), RG-7596 (Roche/Genentech), RG-7598 (Roche/Genentech), RG-7599 (Roche/Genentech), RG-7600 (Roche/Genentech), RG-7636 (Roche/Genentech), anti-PSMA ADC (Progenics), lorvotuzumab mertansine (ImmunoGen), milatuzumab-doxorubicin (Immunomedics), IMMU-130 (Immunomedics) and IMMU-132 (Immunomedics). (See, e.g., Li et al., 2013, Drug Disc Ther 7:178-84; Firer & Gellerman, J Hematol Oncol 5:70; Beck et al., 2010, Discov Med 10:329-39; Mullard, 2013, Nature Rev Drug Discovery 12:329.) Preferably, where an ADC is used in combination with an immunomodulator, the ADC is administered prior to the immunomodulator.

The subject agents may be administered in combination with one or more other immunomodulators to enhance the immune response. Immunomodulators may include, but are not limited to, a cytokine, a chemokine, a stem cell growth factor, a lymphotoxin, an hematopoietic factor, a colony stimulating factor (CSF), erythropoietin, thrombopoietin, tumor necrosis factor-α (TNF), TNF-β, granulocyte-colony stimulating factor (G-CSF), granulocyte macrophage-colony stimulating factor (GM-CSF), interferon-α, interferon-β, interferon-γ, interferon-λ, stem cell growth factor designated "S1 factor", human growth hormone, N-methionyl human growth hormone, bovine growth hormone, parathyroid hormone, thyroxine, insulin, proinsulin, relaxin, prorelaxin, follicle stimulating hormone (FSH), thyroid stimulating hormone (TSH), luteinizing hormone (LH), hepatic growth factor, prostaglandin, fibroblast growth factor, prolactin, placental lactogen, OB protein, mullerian-inhibiting substance, mouse gonadotropin-associated peptide, inhibin, activin, vascular endothelial growth factor, integrin, NGF-β, platelet-growth factor, TGF-α, TGF-β, insulin-like growth factor-I, insulin-like growth factor-II, macrophage-CSF (M-CSF), IL-1, IL-1α, IL-2, IL-3, IL-4, IL-5, IL-6, IL-7, IL-8, IL-9, IL-10, IL-11, IL-12, IL-13, IL-14, IL-15, IL-16, IL-17, IL-18, IL-21, IL-25, LIF, FLT-3, angiostatin, thrombospondin, endostatin, or lymphotoxin. In certain embodiments, a leukocyte- redirecting bispecific antibody or antibody fragment may be attached to an immunomodulator, such as a cytokine. Cytokine complexes are disclosed, for example, in U.S. Patent Nos. 7,906,118 and 8,034,3522.

### BRIEF DESCRIPTION OF THE DRAWINGS

**FIG. 1**. Schematic diagram of formation of DOCK-AND-LOCK^{™} complex comprising anti-CD 19 F(ab)₂ x anti-CD3 scFv.
**FIG.** 2A. Immune synapse formation between Daudi Burkitt lymphoma and T cells, mediated by (19)-3s. Freshly isolated T cells were combined with Daudi cells at an E:T ratio of 2.5:1. Cells were treated with 0, 1 or 5 µg/mL of (19)-3s for 30 min at room temperature prior to analysis by flow cytometry. Anti-CD20-FITC and anti-CD7-APC were used to identify Daudi and T cells, respectively. Co-binding was indicated as the % of CD20⁺/CD7⁺ events. After treatment with (19)-3s, 45.5% of flow events were CD20/CD7 dual-positive, indicating synapsed Daudi and T cells.
**FIG. 2B****.** Conditions were as in FIG. 2(A), except for the absence of (19)-3s antibody. Compared to FIG. 2(A), only 2% of flow events were CD20/CD7 dual-positive without antibody.
**FIG. 2C****.** Addition of (19)-3s resulted in association of >90% of the Daudi with T cells.
**FIG. 3A****.** Jurkat (T cells) and Daudi (B cells) were combined at a 1:1 ratio, treated with 0.1 µg/mL (19)-3s for 30 minutes and stained with anti-CD20-FITC, prior to analysis by fluorescence microscopy.
**FIG. 3B****.** Jurkat (T cells) and Daudi (B cells) were combined at a 1:1 ratio, treated with 0.1 µg/mL (19)-3s for 30 minutes and stained with anti-CD20-FITC and anti-CD3-PE, prior to analysis by fluorescence microscopy.
**FIG. 3C****.** The merged image of FIG. 3A and 3B reveals synapse formation between green-stained Daudi and red-stained Jurkat cells.
**FIG. 3D****.** Synapse formation was not evident in the absence of (19)-3s.
**FIG. 4****.** Dose response analysis of (19)-3s mediated cell-to-cell association of Daudi and Jurkat cells as a function of increasing concentrations of (19)-3s.
**FIG. 5A****.** Comparison of cell-to-cell association mediated by BITE^{®} and DART^{™}. The data for BITE^{®} and DART^{™} was taken from Moore et al. (2011, Blood 117:4542-4551.
**FIG. 5B****.** Comparison of cell-to-cell association mediated by (19)-3s.
**FIG. 6A****.** Synapse formation between T cells and Capan-1 pancreatic cancer cells mediated by (19)-3s control bsAb. CFSE-labeled Capan-1 cells were coincubated with PKH26-labeled Jurkat in the presence of the bsAb.
**FIG. 6B****.** Synapse formation between T cells and Capan-1 pancreatic cancer cells mediated by (M1)-3s MUC5AC bsAb. CFSE-labeled Capan-1 cells were coincubated with PKH26-labeled Jurkat in the presence of the bsAb.
**FIG. 6C****.** Synapse formation between T cells and Capan-1 pancreatic cancer cells mediated by (E1)-3s TROP-2 targeting bsAb. CFSE-labeled Capan-1 cells were coincubated with PKH26-labeled Jurkat in the presence of the bsAb.

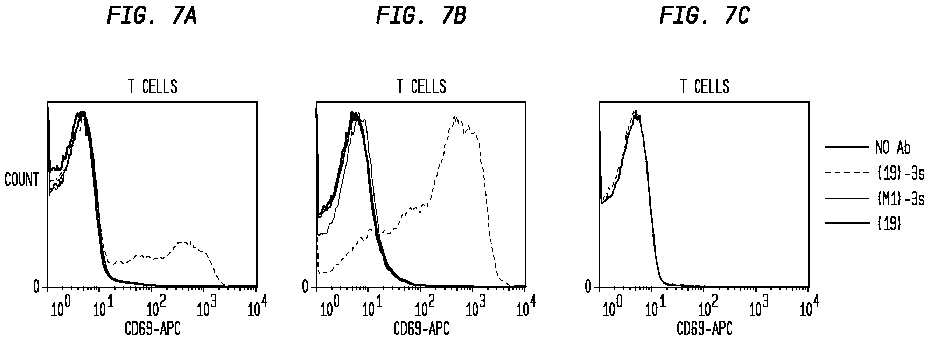

**FIG. 7A****.** T**-**cell activation by (19)-3s. Upregulation of CD69 expression is an early event in T-cell activation. Daudi cells combined with PBMCs were treated overnight with the indicated antibodies, and stained with anti-CD3-PE and anti-CD69-APC, prior to analysis by flow cytometry. CD69 expression was evaluated following gating of T cells by forward vs. side scattering and anti-CD3 staining. Combination of Daudi cells with an equal number of PBMCs resulted in 1.6% CD69+ T cells. Addition of 3 ng/mL (19)-3s induced 27% CD69+ T cells. Neither a control construct [(M1)-3s], which comprises the Okt3-scFv-AD2 module fused with a non-targeting F(ab)₂, nor the hA19-Fab-DDD2 module, induced T-cell activation.
**FIG. 7B****.** T-cell activation by (19)-3s. Daudi cells combined with purified T cells were treated overnight with the indicated antibodies, and stained with anti-CD3-PE and anti-CD69-APC, prior to analysis by flow cytometry. CD69 expression was evaluated following gating of T cells by forward vs. side scattering and anti-CD3 staining. Treatment of Daudi and purified T cells with (M1)-3s or hA19-Fab-DDD2 did not increase the number of CD69+ T cells (<4%), compared to the untreated cell mixture. Alternatively, (19)-3s induced robust T-cell activation, producing 80% CD69+ cells.
**FIG. 7C****.** T-cell activation by (19)-3s. Purified T cells alone were treated overnight with the indicated antibodies, and stained with anti-CD3-PE and anti-CD69-APC, prior to analysis by flow cytometry. CD69 expression was evaluated following gating of T cells by forward vs. side scattering and anti-CD3 staining. Without the addition of Daudi (target) cells, (19)-3 s did not induce CD69 expression and T-cell activation. These results demonstrate that (19)-3s-mediated synapse formation between T cells and target cells is both required and sufficient for T-cell activation.
**FIG. 8A****.** Induction of T-cell proliferation by (19)-3s. PBMCs were incubated with 3 nM or 30pM of (19)-3s, compared to IL-2/PHA positive control and (14)-3s (non-target-binding control).
**FIG. 8B****.** Induction of T-cell proliferation by (19)-3s. T cell proliferation was not observed in PBMCs depleted of B cells, indicating that target cells (B cells) are required for T-cell activation and proliferation.
**FIG. 9A****.** *In vitro* cytotoxicity of (19)-3s T-cell redirecting bsAbs. Dose-response curves for cytotoxicity to Nalm-6, Raji, Ramos and Namalwa cancer cells were determined for the (19)-3s DNL^{™} bsAb complex.
**FIG. 9B****.** *In vitro* cytotoxicity of (19)-3s T-cell redirecting bsAbs. Dose-response curves for cytotoxicity to Nalm-6, Raji, Ramos and Namalwa cancer cells were determined for the (14)-3s (non-targeting) DNL^{™} bsAb complex.
**FIG. 9C****.** Consistent results were observed using PBMCs, or T cells, obtained from two different donors and Nalm-6 cancer cells.
**FIG. 10A****.** *In vitro* cytotoxicity of (20)-3 s, (22)-3 s and (C2)-3s T-cell redirecting bsAbs. Dose-response curves were determined for cytotoxicity to Namalwa cells induced by (20)-3s, (22)-3s and (C2)-3s T-cell redirecting bsAbs.
**FIG. 10B****.** *In vitro* cytotoxicity of (20)-3 s, (22)-3 s and (C2)-3s T-cell redirecting bsAbs. Dose-response curves were determined for cytotoxicity to Jeko cells induced by (20)-3s, (22)-3s and (C2)-3s T-cell redirecting bsAbs.
**FIG. 10C****.** *In vitro* cytotoxicity of (20)-3 s, (22)-3 s and (C2)-3s T-cell redirecting bsAbs. Dose-response curves were determined for cytotoxicity to Daudi cells induced by (20)-3s, (22)-3s and (C2)-3s T-cell redirecting bsAbs.
**FIG. 11A****.** *In vitro* cytotoxicity of T-cell redirecting bsAbs in solid tumor cell lines. Dose-response curves were determined for cytotoxicity to the LS174T colon adenocarcinoma cell line for the (14)-3s bsAb, compared to non-targeting (19)-3s bsAb.
**FIG. 11B****.** *In vitro* cytotoxicity of T-cell redirecting bsAbs in solid tumor cell lines. Dose-response curves were determined for cytotoxicity to the Capan-1 pancreatic adenocarcinoma cell line for the (E1)-3s bsAb, compared to non-targeting (19)-3s bsAb.
**FIG. 11C****.** *In vitro* cytotoxicity of T-cell redirecting bsAbs in solid tumor cell lines. Dose-response curves were determined for cytotoxicity to the NCI-N87 gastric carcinoma cell line for the (E1)-3s and (15)-3s bsAbs, compared to non-targeting (19)-3s bsAb.
**FIG. 12****.** Summary of *in vitro* cytotoxicity data for T-cell redirecting bsAbs in cancer cell lines.
**FIG. 13A****.** *In vivo* retargeting of Raji lymphoma xenografts using (19)-3s bsAb. NOD/SCID mice bearing Raji Burkitt lymphoma (1 x 10⁶ cells) xenografts, reconstituted with human PBMCs (5 x 10⁶ cells) and treated with (19)-3s for only 1 week, administered as indicated by the arrows. Control with untreated cells.
**FIG. 13B****.** *In vivo* retargeting of Raji lymphoma xenografts using (19)-3s bsAb. NOD/SCID mice bearing Raji Burkitt lymphoma (1 x 10⁶ cells) xenografts, reconstituted with human PBMCs (5 x 10⁶ cells) and treated with (19)-3s for only 1 week, administered as indicated by the arrows. Cells were treated with a single dose of 130 µg.
**FIG. 13C****.** *In vivo* retargeting of Raji lymphoma xenografts using (19)-3s bsAb. NOD/SCID mice bearing Raji Burkitt lymphoma (1 x 10⁶ cells) xenografts, reconstituted with human PBMCs (5 x 10⁶ cells) and treated with (19)-3s for only 1 week, administered as indicated by the arrows. Cells were treated 3x with 43 µg per dose.
**FIG. 13D****.** *In vivo* retargeting of Raji lymphoma xenografts using (19)-3s bsAb. NOD/SCID mice bearing Raji Burkitt lymphoma (1 x 10⁶ cells) xenografts, reconstituted with human PBMCs (5 x 10⁶ cells) and treated with (19)-3s for only 1 week, administered as indicated by the arrows. Cells were treated 5x with 26 µg per dose.
**FIG. 14A****.** Effect of repeated dosing on *in vivo* retargeting of Raji lymphoma xenografts using (19)-3s bsAb. NOD/SCID mouse xenografts were prepared as indicated in the legend to FIG. 13. The (19)-3s was administered as indicated by the arrows. FIG. 14A shows untreated control.
**FIG. 14B****.** Effect of repeated dosing on *in vivo* retargeting of Raji lymphoma xenografts using (19)-3s bsAb. The (19)-3s was administered as indicated by the arrows. Cells were treated 2x with 130 µg per dose of (19)-3s administered i.v.
**FIG. 14C****.** Effect of repeated dosing on *in vivo* retargeting of Raji lymphoma xenografts using (19)-3s bsAb. The (19)-3s was administered as indicated by the arrows. Cells were treated treated 2x with 130 µg per dose of (19)-3s administered s.c.
**FIG. 14D****.** Effect of repeated dosing on *in vivo* retargeting of Raji lymphoma xenografts using (19)-3s bsAb. The (19)-3s was administered as indicated by the arrows. Cells were treated treated 4x with 65 µg per dose of (19)-3s administered i.v.
**FIG. 14E****.** Effect of repeated dosing on *in vivo* retargeting of Raji lymphoma xenografts using (19)-3s bsAb. The (19)-3s was administered as indicated by the arrows. Cells were treated treated 6x with 43 µg per dose of (19)-3s administered i.v.
**FIG. 14F****.** Effect of repeated dosing on *in vivo* retargeting of Raji lymphoma xenografts using (19)-3s bsAb. The (19)-3s was administered as indicated by the arrows. Cells were treated treated 6x with 43 µg per dose of control (M1)-3s administered i.v.
**FIG. 15A****.** *In vivo* efficacy of T-cell retargeting bsAbs in solid tumor xenografts. NOD/SCID mouse xenografts were prepared with LS174T colon adenocarcinoma. Mice were administered T cells only without bsAb.
**FIG. 15B****.** *In vivo* efficacy of T-cell retargeting bsAbs in solid tumor xenografts. NOD/SCID mouse xenografts were prepared with LS174T colon adenocarcinoma. Mice were treated with (E1)-3s bsAb as indicated.
**FIG. 15C****.** *In vivo* efficacy of T-cell retargeting bsAbs in solid tumor xenografts. NOD/SCID mouse xenografts were prepared with Capan-1 pancreatic carcinoma. Mice were administered PBMCs only without bsAb.
**FIG. 15D****.** *In vivo* efficacy of T-cell retargeting bsAbs in solid tumor xenografts. NOD/SCID mouse xenografts were prepared with Capan-1 pancreatic carcinoma. Mice were treated with (14)-3s bsAb as indicated.
**FIG. 16A****.** *In vivo* inhibition of tumor growth by (E1)-3s DNL^{™} complex in the presence or absence of interferon-α. Capan-1 pancreatic carcinoma xenografts in NOD/SCID mice were treated with anti-TROP-2 x anti-CD3 bsAb with or without added interferon-α. The interferon-α was added in the form of a TROP-2 targeting DNL^{™} complex.
**FIG. 16B****.** *In vivo* inhibition of tumor growth by (E1)-3s DNL^{™} complex in the presence or absence of interferon-α. Capan-1 pancreatic carcinoma xenografts in NOD/SCID mice were treated with anti-TROP-2 x anti-CD3 bsAb with or without added interferon-α. The interferon-α was added as the commercially available PEGASYS^{®} (peginterferon alfa-2a).
**FIG. 17****.** Survival curves for NOD/SCID mice treated with (E1)-3s with or without interferon-α. Controls were untreated or treated with interferon-α alone.
**FIG. 18****.** *In vivo* inhibition of tumor growth by (E1)-3s DNL^{™} complex in the presence or absence of interferon-α, compared to TF12 control. Capan-1 pancreatic carcinoma xenografts in NOD/SCID mice were treated with anti-TROP-2 x anti-CD3 bsAb with or without added interferon-α, added as PEGASYS^{®}, compared to untreated control, TF12 control or PEGASYS^{®} alone.
**FIG. 19****.** Survival curves for NOD/SCID mice treated with (E1)-3s with or without interferon-α (PEGASYS^{®}). Controls were untreated or treated with PEGASYS^{®} alone or TF12 alone.
**FIG. 20****.** *In vivo* inhibition of tumor growth by (E1)-3s DNL^{™} complex in the presence or absence of interferon-α, compared to TF12 control. NCI-N87 human gastric cancer xenografts in NOD/SCID mice were treated with anti-TROP-2 x anti-CD3 bsAb with or without added interferon-α, added as PEGASYS^{®}, compared to untreated control, TF12 control or PEGASYS^{®} alone.
**FIG. 21****.** Survival curves for NOD/SCID mice with NCI-N87 gastric cancer xenografts treated with (E1)-3s with or without interferon-α (PEGASYS^{®}). Controls were untreated or treated with PEGASYS^{®} alone or TF12 alone.
**FIG. 22****.** Schematic representation of the nascent E1-3 polypeptide. LP, leader peptide that is removed in mature protein; VH, heavy chain variable domain, VK, kappa light chain variable domain, L1, linker peptide 1; L2, linker peptide 2; L3, linker pepide 3; 6H, hexa-histidine.
**FIG. 23A****.** Ex vivo T cell redirected killing of BxPC3 human pancreatic cancer solid tumor cell line.
**FIG. 23B****.** Ex vivo T cell redirected killing of Capan-1 human pancreatic cancer solid tumor cell line.
**FIG. 23C****.** Ex vivo T cell redirected killing of NCI-N87 human gastric cancer solid tumor cell line.
**FIG. 24****.** *In vivo* T cell redirected therapy of NCI-N87 gastric carcinoma in NOD-SCID mice.

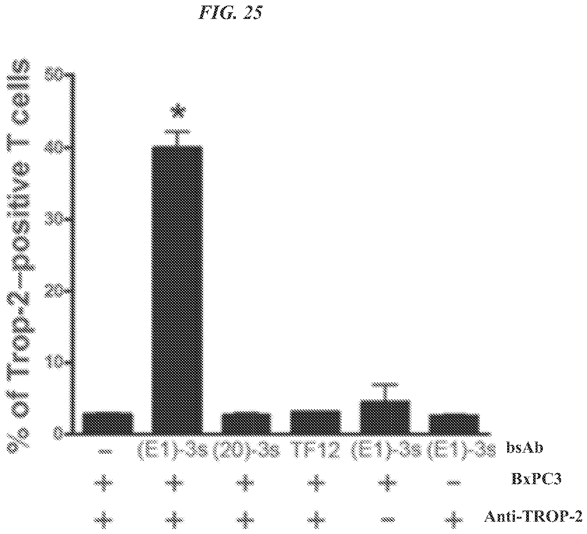

**FIG. 25****.** Immunologic synapse formation and bidirectional trogocytosis mediated by (E1)-3s. Purified T cells were mixed with BxPC3 cells at a 5:1 ratio and incubated for 60 minutes with 0.1 nmol/L of the indicated bsAb before staining with anti-Trop-2 MAb C518 and GAM-Fc-FITC. The cells were analyzed by flow cytometry, with nonconjugated T cells and BxPC3 cells first gated by forward versus side scattering. Trogocytosis of Trop-2 from BxPC3 cells to T cells was evident by detection of Trop-2 on T cells, specifically in cell mixtures with (E1)-3s, shown as the percentage of Trop-2-positive unconjugated T cells.
**FIG. 26****.** Immunologic synapse formation and bidirectional trogocytosis mediated by (E1)-3s. Purified T cells were mixed with BxPC3 cells at a 5:1 ratio and incubated for 60 minutes with 0.1 nmol/L of the indicated bsAb before staining with anti-Trop-2 MAb C518 and GAM-Fc-FITC. The cells were analyzed by flow cytometry, with nonconjugated T cells and BxPC3 cells first gated by forward versus side scattering. Trogocytosis resulted in a reduction of Trop-2 on BxPC3 cells, shown as the geometric MFI.
**FIG. 27A****.** Cytokine induction. (**A**) PBMCs (6 x 10⁶ cells/well) were combined with Raji (5 × 10⁵ cells/well) and treated for 20 h with 0.1 nM 19-3 BiTE (checkered), (19)-3s (black), or incubated without bsAb (white, not tested for D-5). Concentrations of TNF-α, IFN-γ, IL-2, IL-6, and IL-10 in the supernatant fluids were determined using commercial ELISA kits. D-1 through D-8 are independent blood donors, where only D-5 was used in both A and B at the same time.
**FIG. 27B****.** NCI-N87 cells (5 x 10⁵ cells/0.5 mL/well) were cultured overnight in 24-well plates to allow cell attachment. PBMCs were added to wells containing attached NCI-N87 cells (10:1 ratio) and treated for 20 h with 0.1 nM of (E1)-3s (black), peginterferonalfa-2a (white), (E1)-3s plus peginterferonalfa-2a (checkered), or untreated (gray). Concentrations of TNF-α, IFN-γ, IL-2, IL-6, and IL-10 in the supernatant fluids were determined using commercial ELISA kits. D-1 through D-8 are independent blood donors, where only D-5 was used in both A and B at the same time.

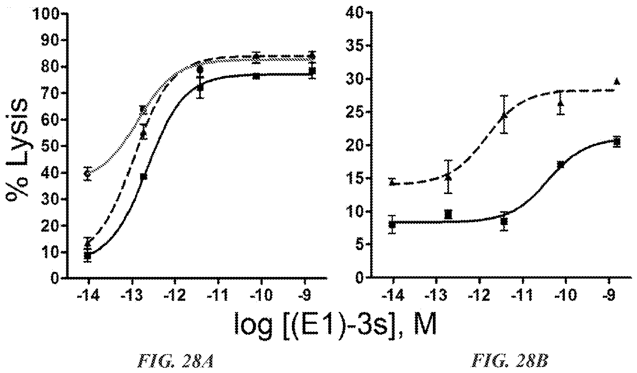

**FIG. 28A****.** *In-vitro* cytotoxicity. Purified CD8⁺ T cells isolated from a first donor were pre-treated for 24 h with 0.1 nM peginterferonalfa-2a (▲, dashed), 0.1 nM 20*-2b (●, grey) or media (■, black) before combining with PKH-67 green fluorescent labeled NCI-N87 cells at a 5:1 ratio. The cell mixtures were treated with titrations of (E1)-3s for two days before counting the number of live NCI-N87 cells by flow cytometry. Non-linear regression analysis (sigmoidal dose-response) of the percent lysis, which was calculated for each sample using the formula: [1-(A₁/A₂)] x 100, where A₁ and A₂ represent the number of viable target cells in the test and untreated samples, respectively, vs the log of the molar concentration of (E1)-3 s.
**FIG. 28B****.** *In-vitro* cytotoxicity. Purified CD8⁺ T cells isolated from a second donor were pre-treated for 24 h with 0.1 nM peginterferonalfa-2a (▲, dashed), 0.1 nM 20*-2b (●, grey) or media (■, black) before combining with PKH-67 green fluorescent labeled NCI-N87 cells at a 5:1 ratio. The cell mixtures were treated with titrations of (E1)-3s for two days before counting the number of live NCI-N87 cells by flow cytometry. Non-linear regression analysis (sigmoidal dose-response) of the percent lysis, which was calculated for each sample using the formula: [1-(A₁/A₂)] x 100, where A₁ and A₂ represent the number of viable target cells in the test and untreated samples, respectively, vs the log of the molar concentration of (E1)-3 s.
**FIG. 29A****.** T-cell activation. Purified T cells were mixed 5:1 with NCI-N87 cells and treated for 18 h with (E1)-3s before measuring CD69 expression by flow cytometry. Non-linear regression analysis (sigmoidal dose-response) of the percent CD69-positive CD4⁺ (●) or CD8⁺ (■) T cells *vs* the log of the molar concentration of (E1)-3s, in the presence (dashed line) or absence (solid line) of 0.1 nM peginterferonalfa-2a.
**FIG. 29B****.** T-cell activation. Purified T cells were mixed 5:1 with NCI-N87 cells and treated for 18 h with (E1)-3s before measuring CD69 expression by flow cytometry. Histogram showing anti-CD69-APC staining of CD8⁺ T cells following treatment with 0.1 nM (E1)-3s (dotted), 0.1 nM peginterferonalfa-2a (gray), or a combination of both agents (black), in the presence of NCI-N87 cells.
**FIG. 29C****.** T-cell activation. Purified T cells were mixed 5:1 with NCI-N87 cells and treated for 18 h with (E1)-3s before measuring CD69 expression by flow cytometry. Percent CD69-positive CD8⁺ T cells after incubation with 0.1 nM (E1)-3s (E) and/or 0.1 nM peginterferonalfa-2a (P), in the absence or presence of NCI-N87 target cells (T). Each treatment was assayed in triplicate. Error bars, S.D. *, P<0.001.
**FIG. 29D****.** T-cell activation. Purified T cells were mixed 5:1 with NCI-N87 cells and treated for 18 h with (E1)-3s before measuring CD69 expression by flow cytometry. Geometric mean fluorescence of the CD69⁺ cells after incubation with 0.1 nM (E1)-3s (E) and/or 0.1 nM peginterferonalfa-2a (P), in the absence or presence of NCI-N87 target cells (T). Each treatment was assayed in triplicate. Error bars, S.D. *, P<0.001.

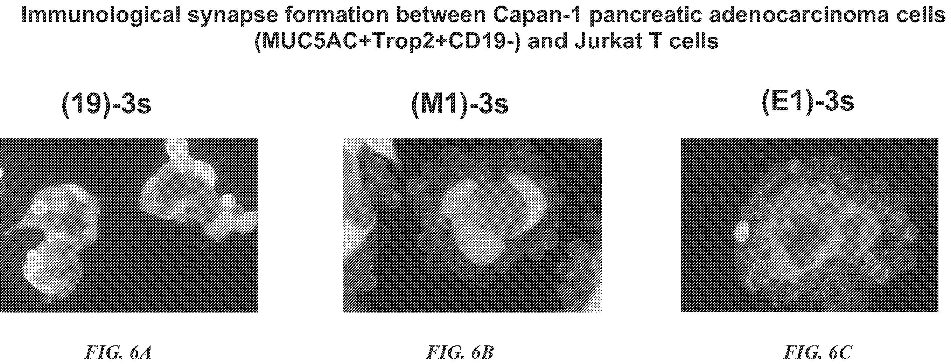

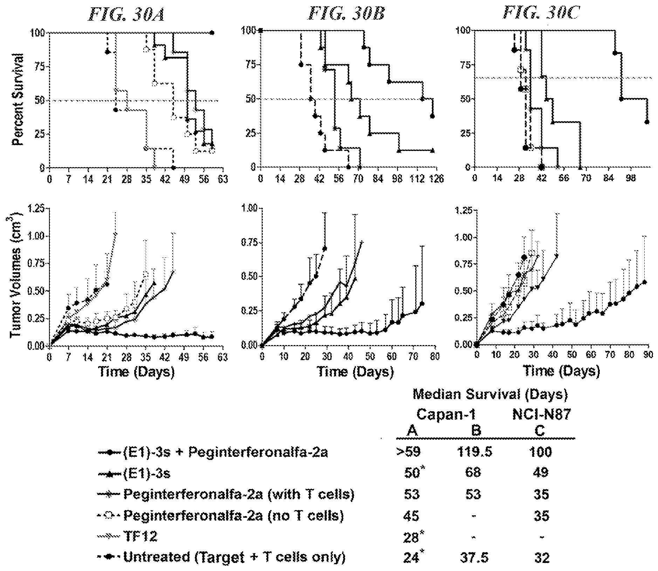

**FIG. 30A****.** In-vivo efficacy with human pancreatic and gastric cancer xenografts. Groups of 8 mice inoculated with human T cells and Capan-1 pancreatic cancer cells were treated daily for five days with 50 µg of (E1)-3s (▲, solid black) or 60 µg TF12 (▼, gray), once weekly for four weeks with 0.6 µg of peginterferonalfa-2a (*, solid black), a combination of (E1)-3s and peginterferonalfa-2a regimens (●, solid black) or with saline (●, dashed black). An additional group was inoculated with Capan-1, but not T cells, and treated with peginterferonalfa-2a (0, dashed black). Top panel, Kaplan-Meyer survival plots. Bottom panel, mean tumor volumes (+S.D.) vs days. Data marked with an asterisk were adapted from Fig. 6C in Rossi et al. (2014, MAbs 6:381-91).
**FIG. 30B****.** In-vivo efficacy with human pancreatic and gastric cancer xenografts. Groups of 8 mice inoculated with human T cells and Capan-1 pancreatic cancer cells were treated daily for five days with 50 µg of (E1)-3s (A, solid black) or 60 µg TF12 (▼, gray), once weekly for four weeks with 0.6 µg of peginterferonalfa-2a (*, solid black), a combination of (E1)-3s and peginterferonalfa-2a regimens (●, solid black) or with saline (●, dashed black). An additional group was inoculated with Capan-1, but not T cells, and treated with peginterferonalfa-2a (0, dashed black). Top panel, Kaplan-Meyer survival plots. Bottom panel, mean tumor volumes (+S.D.) vs days. Data marked with an asterisk were adapted from Fig. 6C in Rossi et al. (2014, MAbs 6:381-91).
**FIG. 30C****.** In-vivo efficacy with human pancreatic and gastric cancer xenografts. Groups of 8 mice inoculated with NCI-N87 gastric cancer cells were treated daily for five days with 50 µg of (E1)-3s (A, solid black) or 60 µg TF12 (▼, gray), once weekly for four weeks with 0.6 µg of peginterferonalfa-2a (*, solid black), a combination of (E1)-3s and peginterferonalfa-2a regimens (●, solid black) or with saline (●, dashed black). An additional group was inoculated with Capan-1, but not T cells, and treated with peginterferonalfa-2a (0, dashed black). Top panel, Kaplan-Meyer survival plots. Bottom panel, mean tumor volumes (+S.D.) vs days. Data marked with an asterisk were adapted from Fig. 6C in Rossi et al. (2014, MAbs 6:381-91).
**FIG. 31****.** Cytokine production induced by E1-3. PBMCs were combined at a 5:1 ratio with BxPC-3 cells and treated with a titration of E1-3 for 24 h. Cytokine concentrations were measured using Single-Analyte ELISArray kits (Qiagen). All cytokine levels were <10 pg/mL in the absence of E1-3.
**FIG. 32****.** *In vitro* redirected T cell killing of pancreatic and gastric cancer cell lines. Purified CD8⁺ T cells (1.2 x 10⁵/well) were mixed 6:1 with target cells (2 x 10⁴/well) and treated with titrations of E1-3 in a 96-well plate. After 48 h, wells were washed to remove T cells and the viable target cell densities were determined with an MTS assay. Example of results for one of several T cell donors.
**FIG. 33A****.** *In vivo* therapy of human gastric tumor xenografts. PBMCs were mixed 2:1 with NCI-N87 cells and injected s.c. with matrigel in NOD-SCID mice. Animals were given 50 µg E1-3 i.v. on Days 0 and 3. Mice were monitored daily for signs of tumor outgrowth, after which tumors were measured twice weekly with an endpoint measurement of >1.0 cm³. After 176 days, 7 of 8 mice in the E1-3 treatment group had not reached the endpoint with 6 animals remaining tumor free.
**FIG. 33B****.** *In vivo* therapy of human gastric tumor xenografts. PBMCs were mixed 2:1 with NCI-N87 cells and injected s.c. with matrigel in NOD-SCID mice. Animals were given 50 µg E1-3 i.v. on Days 0 and 3. Mice were monitored daily for signs of tumor outgrowth, after which tumors were measured twice weekly with an endpoint measurement of >1.0 cm³. Tumors in the control group comprising only PBMCs and NCI-87 reached the end point with a median time of 39.5 days.

### DETAILED DESCRIPTION

### Definitions

Unless otherwise specified, "a" or "an" means "one or more".

As used herein, the terms "and" and "or" may be used to mean either the conjunctive or disjunctive. That is, both terms should be understood as equivalent to "and/or" unless otherwise stated.

A "therapeutic agent" is an atom, molecule, or compound that is useful in the treatment of a disease. Examples of therapeutic agents include antibodies, antibody fragments, peptides, drugs, toxins, enzymes, nucleases, hormones, immunomodulators, antisense oligonucleotides, small interfering RNA (siRNA), chelators, boron compounds, photoactive agents, dyes, and radioisotopes.

An "antibody" as used herein refers to a full-length (i.e., naturally occurring or formed by normal immunoglobulin gene fragment recombinatorial processes) immunoglobulin molecule *(e.g.,* an IgG antibody) or an immunologically active (i.e., specifically binding) portion of an immunoglobulin molecule, like an antibody fragment. An "antibody" includes monoclonal, polyclonal, bispecific, multispecific, murine, chimeric, humanized and human antibodies.

A "naked antibody" is an antibody or antigen binding fragment thereof that is not attached to a therapeutic or diagnostic agent. The Fc portion of an intact naked antibody can provide effector functions, such as complement fixation and ADCC (see, *e.g.*, Markrides, Pharmacol Rev 50:59-87, 1998). Other mechanisms by which naked antibodies induce cell death may include apoptosis. (Vaswani and Hamilton, Ann Allergy Asthma Immunol 81: 105-119, 1998.)

An "antibody fragment" is a portion of an intact antibody such as F(ab')₂, F(ab)₂, Fab', Fab, Fv, scFv, dAb and the like. Regardless of structure, an antibody fragment binds with the same antigen that is recognized by the full-length antibody. For example, antibody fragments include isolated fragments consisting of the variable regions, such as the "Fv" fragments consisting of the variable regions of the heavy and light chains or recombinant single chain polypeptide molecules in which light and heavy variable regions are connected by a peptide linker ("scFv proteins"). "Single-chain antibodies", often abbreviated as "scFv" consist of a polypeptide chain that comprises both a V_{H} and a V_{L} domain which interact to form an antigen- binding site. The V_{H} and V_{L} domains are usually linked by a peptide of 1 to 25 amino acid residues. Antibody fragments also include diabodies, triabodies and single domain antibodies (dAb).

A "chimeric antibody" is a recombinant protein that contains the variable domains including the complementarity determining regions (CDRs) of an antibody derived from one species, preferably a rodent antibody, while the constant domains of the antibody molecule are derived from those of a human antibody. For veterinary applications, the constant domains of the chimeric antibody may be derived from that of other species, such as a cat or dog.

A "humanized antibody" is a recombinant protein in which the CDRs from an antibody from one species; e.g., a rodent antibody, are transferred from the heavy and light variable chains of the rodent antibody into human heavy and light variable domains, including human framework region (FR) sequences. The constant domains of the antibody molecule are derived from those of a human antibody. To maintain binding activity, a limited number of FR amino acid residues from the parent (e.g., murine) antibody may be substituted for the corresponding human FR residues.

A "human antibody" is an antibody obtained from transgenic mice that have been genetically engineered to produce specific human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described by Green et al., Nature Genet. 7:13 (1994), Lonberg et al., Nature 368:856 (1994), and Taylor et al., Int. Immun. 6:579 (1994). A human antibody also can be constructed by genetic or chromosomal transfection methods, as well as phage display technology, all of which are known in the art. (See, e.g., McCafferty et al., 1990, Nature 348:552-553 for the production of human antibodies and fragments thereof *in vitro,* from immunoglobulin variable domain gene repertoires from unimmunized donors). In this technique, antibody variable domain genes are cloned in-frame into either a major or minor coat protein gene of a filamentous bacteriophage, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. In this way, the phage mimics some of the properties of the B cell. Phage display can be performed in a variety of formats, for their review, see, e.g. Johnson and Chiswell, Current Opinion in Structural Biology 3:5564-571 (1993). Human antibodies may also be generated by *in vitro* activated B cells. (See, U.S. Pat. Nos. 5,567,610 and 5,229,275).

As used herein, the term "antibody fusion protein" is a recombinantly produced antigen-binding molecule in which an antibody or antibody fragment is linked to another protein or peptide, such as the same or different antibody or antibody fragment or a DDD or AD peptide. The fusion protein may comprise a single antibody component, a multivalent or multispecific combination of different antibody components or multiple copies of the same antibody component. The fusion protein may additionally comprise an antibody or an antibody fragment and a therapeutic agent. Examples of therapeutic agents suitable for such fusion proteins include immunomodulators and toxins. One preferred toxin comprises a ribonuclease (RNase), preferably a recombinant RNase. A preferred immunomodulator might be an interferon, such as interferon-α, interferon-β or interferon-λ.

A "multispecific antibody" is an antibody that can bind simultaneously to at least two targets that are of different structure, e.g., two different antigens, two different epitopes on the same antigen, or a hapten and/or an antigen or epitope. A "multivalent antibody" is an antibody that can bind simultaneously to at least two targets that are of the same or different structure. Valency indicates how many binding arms or sites the antibody has to a single antigen or epitope; i.e., monovalent, bivalent, trivalent or multivalent. The multivalency of the antibody means that it can take advantage of multiple interactions in binding to an antigen, thus increasing the avidity of binding to the antigen. Specificity indicates how many antigens or epitopes an antibody is able to bind; i.e., monospecific, bispecific, trispecific, multispecific. Using these definitions, a natural antibody, e.g., an IgG, is bivalent because it has two binding arms but is monospecific because it binds to one epitope. Multispecific, multivalent antibodies are constructs that have more than one binding site of different specificity.

A "bispecific antibody" is an antibody that can bind simultaneously to two targets which are of different structure. Bispecific antibodies (bsAb) and bispecific antibody fragments (bsFab) may have at least one arm that specifically binds to, for example, a T cell, an NK cell, a monocyte or a neutrophil, and at least one other arm that specifically binds to an antigen produced by or associated with a diseased cell, tissue, organ or pathogen, for example a tumor-associated antigen. A variety of bispecific antibodies can be produced using molecular engineering.

An antibody preparation, or a composition described herein, is said to be administered in a "therapeutically effective amount" if the amount administered is physiologically significant. An agent is physiologically significant if its presence results in a detectable change in the physiology of a recipient subject. In particular embodiments, an antibody preparation is physiologically significant if its presence invokes an antitumor response or mitigates the signs and symptoms of an infectious disease state. A physiologically significant effect could also be the evocation of a humoral and/or cellular immune response in the recipient subject leading to growth inhibition or death of target cells.

### Anti-Trop-2 Antibodies

. In a preferred embodiment of the invention as defined in the claims, the anti-Trop-2 antibody may is a humanized RS7 antibody (see, e.g., U.S. Patent No. 7,238,785), comprising the light chain CDR sequences CDR1 (KASQDVSIAVA, SEQ ID NO:115); CDR2 (SASYRYT, SEQ ID NO:116); and CDR3 (QQHYITPLT, SEQ ID NO:117) and the heavy chain CDR sequences CDR1 (NYGMN, SEQ ID NO:118); CDR2 (WINTYTGEPTYTDDFKG, SEQ ID NO:119) and CDR3 (GGFGSSYWYFDV, SEQ ID NO:120).

The RS7 antibody was a murine IgG₁ raised against a crude membrane preparation of a human primary squamous cell lung carcinoma. (Stein et al., Cancer Res. 50: 1330, 1990) The RS7 antibody recognizes a 46-48 kDa glycoprotein, characterized as cluster 13 (Stein et al., Int. J. Cancer Supp. 8:98-102, 1994). The antigen was designated as EGP-1 (epithelial glycoprotein-1), but is also referred to as Trop-2.

Trop-2 is a type-I transmembrane protein and has been cloned from both human (Fornaro et al., Int J Cancer 1995; 62:610-8) and mouse cells (Sewedy et al., Int J Cancer 1998; 75:324-30). In addition to its role as a tumor-associated calcium signal transducer (Ripani et al., Int J Cancer 1998;76:671-6), the expression of human Trop-2 was shown to be necessary for tumorigenesis and invasiveness of colon cancer cells, which could be effectively reduced with a polyclonal antibody against the extracellular domain of Trop-2 (Wang et al., Mol Cancer Ther 2008;7:280-5).

The growing interest in Trop-2 as a therapeutic target for solid cancers (Cubas et al., Biochim Biophys Acta 2009;1796:309-14) is attested by further reports that documented the clinical significance of overexpressed Trop-2 in breast (Huang et al., Clin Cancer Res 2005;11:4357-64), colorectal (Ohmachi et al., Clin Cancer Res 2006;12:3057-63; Fang et al., Int J Colorectal Dis 2009;24:875-84), and oral squamous cell (Fong et al., Modern Pathol 2008;21:186-91) carcinomas. The latest evidence that prostate basal cells expressing high levels of Trop-2 are enriched for *in vitro* and *in vivo* stem-like activity is particularly noteworthy (Goldstein et al., Proc Natl Acad Sci USA 2008;105:20882-7).

Flow cytometry and immunohistochemical staining studies have shown that the RS7 MAb detects antigen on a variety of tumor types, with limited binding to normal human tissue (Stein et al., 1990). Trop-2 is expressed primarily by carcinomas such as carcinomas of the lung, stomach, urinary bladder, breast, ovary, uterus, and prostate. Localization and therapy studies using radiolabeled murine RS7 MAb in animal models have demonstrated tumor targeting and therapeutic efficacy (Stein et al., 1990; Stein et al., 1991).

Strong RS7 staining has been demonstrated in tumors from the lung, breast, bladder, ovary, uterus, stomach, and prostate. (Stein et al., Int. J. Cancer 55:938, 1993) The lung cancer cases comprised both squamous cell carcinomas and adenocarcinomas. (Stein et al., Int. J. Cancer 55:938, 1993) Both cell types stained strongly, indicating that the RS7 antibody does not distinguish between histologic classes of non-small-cell carcinoma of the lung.

The RS7 MAb is rapidly internalized into target cells (Stein et al., 1993). The internalization rate constant for RS7 MAb is intermediate between the internalization rate constants of two other rapidly internalizing MAbs, which have been demonstrated to be useful for immunotoxin production. (*Id.*) It is well documented that internalization of immunotoxin conjugates is a requirement for anti-tumor activity. (Pastan et al., Cell 47:641, 1986) Internalization of drug immunoconjugates has been described as a major factor in anti-tumor efficacy (Yang et al., Proc. Nat'l Acad. Sci. USA 85: 1189, 1988). Thus, the RS7 antibody exhibits several important properties for therapeutic applications.

While the hRS7 antibody is preferred, other anti-Trop-2 antibodies are known and/or publicly available and in alternative embodiments may be utilized in the subject ADCs. While humanized or human antibodies are preferred for reduced immunogenicity, in alternative embodiments a chimeric antibody may be of use. As discussed below, methods of antibody humanization are well known in the art and may be utilized to convert an available murine or chimeric antibody into a humanized form.

Anti-Trop-2 antibodies are commercially available from a number of sources and include LS-C126418, LS-C178765, LS-C126416, LS-C126417 (LifeSpan BioSciences, Inc., Seattle, WA); 10428-MM01, 10428-MM02, 10428-R001, 10428-R030 (Sino Biological Inc., Beijing, China); MR54 (eBioscience, San Diego, CA); sc-376181, sc-376746, Santa Cruz Biotechnology (Santa Cruz, CA); MM0588-49D6, (Novus Biologicals, Littleton, CO); ab79976, and ab89928 (ABCAM^{®}, Cambridge, MA).

Other anti-Trop-2 antibodies have been disclosed in the patent literature. For example, U.S. Publ. No. 2013/0089872 discloses anti-Trop-2 antibodies K5-70 (Accession No. FERM BP-11251), K5-107 (Accession No. FERM BP-11252), K5-116-2-1 (Accession No. FERM BP-11253), T6-16 (Accession No. FERM BP-11346), and T5-86 (Accession No. FERM BP-11254), deposited with the International Patent Organism Depositary, Tsukuba, Japan. U.S. Patent No. 5,840,854 disclosed the anti-Trop-2 monoclonal antibody BR110 (ATCC No. HB11698). U.S. Patent No. 7,420,040 disclosed an anti-Trop-2 antibody produced by hybridoma cell line AR47A6.4.2, deposited with the IDAC (International Depository Authority of Canada, Winnipeg, Canada) as accession number 141205-05. U.S. Patent No. 7,420,041 disclosed an anti-Trop-2 antibody produced by hybridoma cell line AR52A301.5, deposited with the IDAC as accession number 141205-03. U.S. Publ. No. 2013/0122020 disclosed anti-Trop-2 antibodies 3E9, 6G11, 7E6, 15E2, 18B1. Hybridomas encoding a representative antibody were deposited with the American Type Culture Collection (ATCC), Accession Nos. PTA-12871 and PTA-12872. U.S. Patent No. 8,715,662 discloses anti-Trop-2 antibodies produced by hybridomas deposited at the AID-ICLC (Genoa, Italy) with deposit numbers PD 08019, PD 08020 and PD 08021. U.S. Patent Application Publ. No. 20120237518 discloses anti-Trop-2 antibodies 77220, KM4097 and KM4590. U.S. Patent No. 8,309,094 (Wyeth) discloses antibodies A1 and A3, identified by sequence listing. Non-patent publication Lipinski et al. (1981, Proc Natl. Acad Sci USA, 78:5147-50) disclosed anti-Trop-2 antibodies 162-25.3 and 162-46.2.

Numerous anti-Trop-2 antibodies are known in the art and/or publicly available. As discussed below, methods for preparing antibodies against known antigens were routine in the art. The sequence of the human Trop-2 protein was also known in the art (see, e.g., GenBank Accession No. CAA54801.1). Methods for producing humanized, human or chimeric antibodies were also known. The person of ordinary skill, reading the instant disclosure in light of general knowledge in the art, would have been able to make and use the genus of anti-Trop-2 antibodies in the subject ADCs.

### Anti-CD3 Antibodies

A variety of antibodies against CD3 that may be used in the practing of the invention as defined in the claims are publicly known and/or commercially available, such as from LSBio (catalog Nos. LS-B6698, LS-B8669; LS-B8765, LS-C96311, LS-C58677, etc.); ABCAM^{®} (catalog Nos. ab5690, ab16669, ab699, ab828, ab8671, etc.); Santa Cruz Biotechnology (catalog Nos.sc-20047, sc-20080, sc-19590, sc-59008, sc-101442, etc.); and many other suppliers.

The amino acid sequence of the anti-CD3 moiety, used as part of a DNL^{™} complex, is as disclosed below in SEQ ID NO:96 to SEQ ID NO:101. However, the person of ordinary skill will realize that any known anti-CD3 antibody may be utilized in the in the practing of the invention as defined in the coaims. Preferably, the antibody moieties of use are humanized or human.

### Leukocyte Redirecting Bispecific Antibody Complexes

According to the invention as defined in the claims , the bispecific antibody comprises an anti-CD3 x anti-Trop-2 antibody. As discussed above, various antibodies against CD3 or Trop-2 are known in the art and any such known antibody may be utilized. Howeveras disclosed herein, antibodies against other leukocyte antigens than CD3 or against other disease-associated antigens than Trop-2 may be utilized.

Exemplary T-cell antigens include CD2, CD4, CD5, CD6, CD8, CD25, CD28, CD30, CD40, CD40L, CD44, CD45, CD69 and CD90. Other exemplary antigens may be selected from CD8, CD16, CD56, CD57, ADAM17, and CD137 for NK cells; CD74, HLA-DR alpha chain, CD14, CD16, CD64 and CD89 for monocytes; and CEACAM6, CEACAM8, CD16b, CD32a, CD89, CD177, CD11a, CD11b and SLC44A2 for neutrophils. Preferably, the anti-T-cell antibody binds to CD3, or the anti-NK antibody binds to CD16. As discussed below, many examples of disease-associated antigens, such as tumor-associated antigens (TAAs) are known. An exemplary preferred TAA is Trop-2.

The instant disclosure concerns an anti-CD3 x anti-CD19 bispecific antibody. Various bispecific anti-CD3 x anti-CD19 antibodies are known in the art and presently in clinical development, such as BITE^{®} (Bispecific T-cell Engager) (e.g., Nagorsen et al., 2009, Leukemia & Lymphoma 50:886-91; Amann et al., 2009, J Immunother 32:453-64; Baeuerle and Reinhardt, 2009, Cancer Res 69:4941-44) and DART^{®} (see, e.g., Moore et al., 2011, Blood 117:4542-51; Veri et al., 2010, Arthritis Rheum 62:1933-43). Blinatumomab is a BITE^{®} antibody comprising V_{H} and V_{L} domains of anti-CD3 and anti-CD19 antibody fragments, connected with a 5-amino acid linker and expressed as a single polypeptide chain that anneals to itself to form the antigen-binding sites. It is thought that blinatumomab acts by bringing the T-cell-specific CD3 and B-cell specific CD19 antigens into close proximity, to initiate a T-cell cytotoxic response against the juxtaposed B cells, which does not require T-cell specificity to the cancer cells (e.g., Portell et al., 2013, Clin Pharmacol 5(Suppl 1): 5-11). Due to its short half-life, blinatumomab requires continuous intravenous infusion to be effective, (Portell et al., 2013). A phase II trial of B-cell ALL patients with persistent or relapsed minimal residual disease reported an approximately 80% rate of complete remission (Portell et al., 2013).

Doses of blinatumomab as low as 0.005 mg/m²/day were reported to be effective to eliminate cancer cells in non-Hodgkin's lymphoma patients (Bargou et al., 2008, Science 321:974-77). Partial and complete remissions were observed starting at a dose level of 0.015 mg and all six patients tested at a dose of 0.06 mg experienced a tumor regression (Bargou et al., 2008). *In vitro,* blinatumomab induced 50% cell lysis of MEC-1 cells at a concentration of 10 pg/mL (Topp et al., 2012, Blood 120:5185-87; Bassan et al., 2012, Blood 120:5094-95).

The anti-CD19 portion of blinatumomab was derived from the HD37 hybridoma (see, e.g., U.S. Patent No. 7,575,923), which is publicly available (e.g., Santa Cruz Biotechnology Cat. No. sc-18894). The anti-CD3 portion of blinatumomab was derived from the TR66 hybridoma (U.S. Patent No. 7,575,923; Traunecker et al., 1991, EMBO J. 10:3655-59), also publicly available (e.g., Enzo Life Sciences, catalog No. ALX-804-822-C100).

A variety of antibodies against CD19 are publicly known and/or commercially available, such as from Santa Cruz Biotechnology (catalog Nos. sc-390244, sc-373897, sc-18894, sc-18896, etc.); ABCAM^{®} (catalog Nos. ab25232, ab134114, ab140981, ab1255, etc.); ABBIOTEC^{™} (catalog Nos. 252262, 252248, 250585, 251063, etc.) and many other vendors.

In an example of the instant disclosure, the anti-CD19 antibody moiety is a humanized A19 antibody, comprising the light chain CDR sequences CDR1 KASQSVDYDGDSYLN (SEQ ID NO:90); CDR2 DASNLVS (SEQ ID NO:91); and CDR3 QQSTEDPWT (SEQ ID NO:92) and the heavy chain CDR sequences CDR1 SYWMN (SEQ ID NO:93); CDR2 QIWPGDGDTNYNGKFKG (SEQ ID NO:94) and CDR3 RETTTVGRYYYAMDY (SEQ ID NO:95).

Other anti-CD3 x anti-CD19 bispecific antibodies are known, such as DART^{®}, which also incorporates the anti-CD19 Fv sequences of HD37 and the anti-CD3 Fv sequences of TR66 (Moore et al., 2011, Blood 117:4542-51; Veri et al., 2010, Arthritis Rheum 62:1933-43). Moore et al. (2011) reported that DART^{®} bispecific antibodies were more potent at inducing B cell lysis than single-chain, bispecific antibodies (BITE^{®}) bearing identical anti-CD19 and anti-CD3 variable region sequences, with EC₅₀ values in the pg/mL range (Moore et al., 2011). Other anti-CD3 x anti-CD19 bispecific antibodies besides DART^{®} and BITE^{®} have been reported (see, e.g., Wei et al., 2012, Cell Oncol 35:423-34; Portner et al., 2012, Cancer Immunol Immunother 61:1869-75; Zhou et al., 2012, Biotechnol Lett. 34:1183-91). In certain embodiments, any known anti-CD3 x anti-CD19 bispecific antibody may be used to induce an immune response against disease-associated cells.

Catumaxomab is an anti-CD3 x anti-EpCAM bispecific antibody that has been approved in Europe for treatment of malignant ascites associated with metastasizing cancer (Chames & Baty, 2009, MAbs 1:539-47). In a mouse model system, catumaxomab was able to kill tumor cells at a concentration range of 10 pM and was reported to lead to total eradication of melanoma tumors (Chames & Baty, 2009). Human clinical trials with ovarian cancer patients with malignant ascites also showed a statistically significant efficacy (Chames & Baty, 2009). However, the high immunogenicity of the rat/mouse hybrid bsAb may limit i.v. administration of the antibody (Chames & Baty, 2009). The use of anti-tumor bsAbs is not limited to anti-CD3 x anti-CD19, but has also included anti-HER2 x anti-CD64 (MDX-210, MDX-H210), anti-EGFR x anti-CD64 (MDX-447), anti-CD30 x anti-CD16 (HRS-3/A9), anti-HER2 x anti-CD3 (Her2Bi), anti-CD20 x anti-CD3 (CD20Bi, Bi20), anti-EpCAM x anti-CD3 (catumaxomab, MT110), anti-HER2 x anti-CD3 (ertumaxomab), and anti-NG2 x anti-CD28 (rM28) (Chames & Baty, 2009).

The person of ordinary skill will realize that leukocyte redirecting bispecific antibodies are not limited to anti-CD3 x anti-Trop-2 constructs, but may comprise antibodies against any known disease-associated antigens attached to an anti-CD3 antibody moiety. Alternatively, antibodies against other T-cell antigens besides CD3, or other antigens expressed on NK cells, monocytes or neutrophils may also be used. Exemplary T-cell antigens include, but are not limited to, CD2, CD4, CD5, CD6, CD8, CD25, CD28, CD30, CD40, CD40L, CD44, CD45, CD69 and CD90. Other exemplary antigens may be selected from CD8, CD16, CD56, CD57, ADAM17, KIR and CD137 for NK cells; CD74, HLA-DR alpha chain, CD14, CD16, CD64 and CD89 for monocytes; and CEACAM6, CEACAM8, CD16b, CD32a, CD89, CD177, CD11a, CD11b and SLC44A2 for neutrophils. Antibodies against each of the leukocyte antigens are publicly known and/or publicly available (see, e.g., ABCAM^{®} catalog numbers ab131276, ab139266, ab8360, ab51312, ab846, ab133616, ab75877, ab133255, ab109217, ab93278, ab17147, ab115851, ab128955, ab13463, ab85986; Santa Cruz Biotechnology catalog numbers sc-46683, sc-59047; Enzo Life Sciences, Inc. catalog number ALX-805-037-C100; Sino Biological Inc. catalog numbers 12211-RP02, 11150-R074; Millipore catalog numbers 04-1102, 04-1102, MAB1406). These and numerous other anti-leukocyte antibodies were publicly available and could have been used in the subject leukocyte redirecting bsAbs. As discussed below, numerous antibodies against a wide variety of disease-associated antigens were publicly known and/or commercially available and could have been used in the subject leukocyte redirecting bispecific antibodies. Other exemplary leukocyte redirecting bsAbs of potential use include FBTA05 (anti-CD20 x anti-CD3) and TRBS07 (anti-GD2 x anti-CD3).

### Interferon Therapy

In various embodiments of the invention as defined in the claims, the subject bispecific antibodies may be used in combination with one or more interferons, such as interferon-α, interferon-β or interferon-λ, preferably interferon-α. Human interferons are well known in the art and the amino acid sequences of human interferons may be readily obtained from public databases (e.g., GenBank Accession Nos. AAA52716.1; AAA52724; AAC41702.1; EAW56871.1; EAW56870.1; EAW56869.1). Human interferons may also be commercially obtained from a variety of vendors (e.g., Cell Signaling Technology, Inc., Danvers, MA; Genentech, South San Francisco, CA; EMD Millipore, Billerica, MA).

Interferon-α (IFNα) has been reported to have anti-tumor activity in animal models of cancer (Ferrantini et al., 1994, J Immunol 153:4604-15) and human cancer patients (Gutterman et al., 1980, Ann Intern Med 93:399-406). IFNα can exert a variety of direct anti-tumor effects, including down-regulation of oncogenes, up-regulation of tumor suppressors, enhancement of immune recognition via increased expression of tumor surface MHC class I proteins, potentiation of apoptosis, and sensitization to chemotherapeutic agents (Gutterman et al., 1994, PNAS USA 91:1198-205; Matarrese et al., 2002, Am J Pathol 160:1507-20; Mecchia et al., 2000, Gene Ther 7:167-79; Sabaawy et al., 1999, Int J Oncol 14:1143-51; Takaoka et al, 2003, Nature 424:516-23). For some tumors, IFNα can have a direct and potent anti-proliferative effect through activation of STAT1 (Grimley et al., 1998 Blood 91:3017-27). Interferon-α2b has been conjugated to anti-tumor antibodies, such as the hL243 anti-HLA-DR antibody and depletes lymphoma and myeloma cells *in vitro* and *in vivo* (Rossi et al., 2011, Blood 118:1877-84).

Indirectly, IFNα can inhibit angiogenesis (Sidky and Borden, 1987, Cancer Res 47:5155-61) and stimulate host immune cells, which may be vital to the overall antitumor response but has been largely under-appreciated (Belardelli et al., 1996, Immunol Today 17:369-72). IFNα has a pleiotropic influence on immune responses through effects on myeloid cells (Raefsky et al, 1985, J Immunol 135:2507-12; Luft et al, 1998, J Immunol 161:1947-53), T-cells (Carrero et al, 2006, J Exp Med 203:933-40; Pilling et al., 1999, Eur J Immunol 29:1041-50), and B-cells (Le et al, 2001, Immunity 14:461-70). As an important modulator of the innate immune system, IFNα induces the rapid differentiation and activation of dendritic cells (Belardelli et al, 2004, Cancer Res 64:6827-30; Paquette et al., 1998, J Leukoc Biol 64:358-67; Santini et al., 2000, J Exp Med 191:1777-88) and enhances the cytotoxicity, migration, cytokine production and antibody-dependent cellular cytotoxicity (ADCC) of NK cells (Biron et al., 1999, Ann Rev Immunol 17:189-220; Brunda et al. 1984, Cancer Res 44:597-601).

Interferon-β has been reported to be efficacious for therapy of a variety of solid tumors. Patients treated with 6 million units of IFN-β twice a week for 36 months showed a decreased recurrence of hepatocellular carcinoma after complete resection or ablation of the primary tumor in patients with HCV-related liver cancer (Ikeda et al., 2000, Hepatology 32:228-32). Gene therapy with interferon-β induced apoptosis of glioma, melanoma and renal cell carcinoma (Yoshida et al., 2004, Cancer Sci 95:858-65). Endogenous IFN-β has been observed to inhibit tumor growth by inhibiting angiogenesis *in vivo* (Jablonska et al., 2010, J Clin Invest. 120:1151-64.)

IFN-ks, designated as type III interferons, are a newly described group of cytokines that consist of IFN-λ1, 2, 3 (also referred to as interleukin-29, 28A, and 28B, respectively), that are genetically encoded by three different genes located on chromosome 19 (Kotenko et al., 2003, Nat Immunol 4:69-77; Sheppard et al., 2003, Nat Immunol 4:63-8). At the protein level, IFN-λ2 and -λ3 are is highly homologous, with 96% amino acid identity, while IFN-λ1 shares approximately 81% homology with IFN-λ2 and -λ3 (Sheppard et al., 2003, Nat Immunol 4:63-8). IFN-λs activate signal transduction via the JAK/STAT pathway similar to that induced by type I IFN, including the activation of JAK1 and TYK2 kinases, the phosphorylation of STAT proteins, and the activation of the transcription complex of IFN-stimulated gene factor 3 (ISGF3) (Witte et al., 2010, Cytokine Growth Factor Rev 21:237-51; Zhou et al., 2007, J Virol 81:7749-58).

A major difference between type III and type I IFN systems is the distribution of their respective receptor complexes. IFN-α/β signals through two extensively expressed type I interferon receptors, and the resulting systemic toxicity associated with IFN-α/β administration has limited their use as therapeutic agents (Pestka et al., 2007, J Biol Chem 282:20047-51). In contrast, IFN-λs signal through a heterodimeric receptor complex consisting of unique IFN-λ receptor 1 (IFN-λR1) and IL-10 receptor 2 (IL-10R2). As previously reported (Witte et al., 2009, Genes Immun 10:702-14), IFN-λR1 has a very restricted expression pattern with the highest levels in epithelial cells, melanocytes, and hepatocytes, and the lowest level in primary central nervous system (CNS) cells. Blood immune system cells express high levels of a short IFN-λ receptor splice variant (sIFN-λR1) that inhibits IFN-λ action. The limited responsiveness of neuronal cells and immune cells implies that the severe toxicity frequently associated with IFN-α therapy may be absent or significantly reduced with IFN-λs (Witte et al., 2009, Genes Immun 10:702-14; Witte et al., 2010, Cytokine Growth Factor Rev 21:237-51). A recent publication reported that while IFN-α and IFN-λ induce expression of a common set of ISGs (interferon-stimulated genes) in hepatocytes, unlike IFN-α, administration of IFN-λ did not induce STAT activation or ISG expression in purified lymphocytes or monocytes (Dickensheets et al., 2013, J Leukoc Biol. 93, published online 12/20/12). It was suggested that IFN-λ may be superior to IFN-α for treatment of chronic HCV infection, as it is less likely to induce leukopenias that are often associated with IFN-α therapy (Dickensheets et al., 2013).

IFN-λs display structural features similar to IL-10-related cytokines, but functionally possess type I IFN-like anti-viral and anti-proliferative activity (Witte et al., 2009, Genes Immun 10:702-14; Ank et al., 2006, J Virol 80:4501-9; Robek et al., 2005, J Virol 79:3851-4). IFN-λ1 and -λ2 have been demonstrated to reduce viral replication or the cytopathic effect of various viruses, including DNA viruses (hepatitis B virus (Robek et al., 2005, J Virol 79:3851-4, Doyle et al., 2006, Hepatology 44:896-906) and herpes simplex virus 2 (Ank et al., 2008, J Immunol 180:2474-85)), ss (+) RNA viruses (EMCV; Sheppard et al., 2003, Nat Immunol 4:63-8) and hepatitis C virus (Robek et al., 2005, J Virol 79:3851-4, Doyle et al., 2006, Hepatology 44:896-906; Marcello et al., 2006, Gastroenterol 131:1887-98; Pagliaccetti et al., 2008, J Biol Chem 283:30079-89), ss (-) RNA viruses (vesicular stomatitis virus; Pagliaccetti et al., 2008, J Biol Chem 283:30079-89) and influenza-A virus (Jewell et al., 2010, J Virol 84:11515-22) and double-stranded RNA viruses, such as rotavirus (Pott et al., 2011, PNAS USA 108:7944049). IFN-λ3 has been identified from genetic studies as a key cytokine in HCV infection (Ge et al., 2009, Nature 461:399-401), and has also shown potent activity against EMCV (Dellgren et al., 2009, Genes Immun 10:125-31). A deficiency of rhinovirus-induced IFN-λ production was reported to be highly correlated with the severity of rhinovirus-induced asthma exacerbation (Contoli et al., 2006, Nature Med 12:1023-26) and IFN-λ therapy has been suggested as a new approach for treatment of allergic asthma (Edwards and Johnston, 2011, EMBO Mol Med 3 :306-8; Koltsida et al., 2011, EMBO Mol Med 3:348-61).

The anti-proliferative activity of IFN-λs has been established in several human cancer cell lines, including neuroendocrine carcinoma BON1 (Zitzmann et al., 2006, Biochem Biophys Res Commun 344:1334-41), glioblastoma LN319 (Meager et al., 2005, Cytokine 31:109-18), immortalized keratinocyte HaCaT (Maher et al., 2008, Cancer Biol Ther 7:1109-15), melanoma F01 (Guenterberg et al., 2010, Mol Cancer Ther 9:510-20), and esophageal carcinoma TE-11 (Li et al., 2010, Eur J Cancer 46:180-90). In animal models, IFN-λs induce both tumor apoptosis and destruction through innate and adaptive immune responses, suggesting that local delivery of IFN-λ might be a useful adjunctive strategy in the treatment of human malignancies (Numasaki et al., 2007, J Immunol 178:5086-98). A Fab-linked interferon-λ was demonstrated to have potent anti-tumor and anti-viral activity in targeted cells (Liu et al., 2013, PLoS One 8:e63940).

In clinical settings, PEGylated IFN-λ1 (PEG-IFN-λ1) has been provisionally used for patients with chronic hepatitis C virus infection. In a phase Ib study (n=56), antiviral activity was observed at all dose levels (0.5-3.0 µg/kg), and viral load reduced 2.3 to 4.0 logs when PEG-IFN-λ1 was administrated to genotype 1 HCV patients who relapsed after IFN-α therapy (Muir et al., 2010, Hepatology 52:822-32). A phase IIb study (n=526) showed that patients with HCV genotypes 1 and 4 had significantly higher response rates to treatment with PEG-IFN-λ1 compared to PEG-IFN-α. At the same time, rates of adverse events commonly associated with type I interferon treatment were lower with PEG-IFN-λ1 than with PEG-IFN-α. Neutropenia and thrombocytopenia were infrequently observed and the rates of flu-like symptoms, anemia, and musculoskeletal symptoms decreased to about 1/3 of that seen with PEG-IFN-α treatment. However, rates of serious adverse events, depression and other common adverse events (≥ 10%) were similar between PEG-IFN-λ1 and PEG-IFN-α. Higher rates of hepatotoxicity were seen in the highest-dose PEG-IFN-λ1 compared with PEG-IFN-α ("Investigational Compound PEG-Interferon Lambda Achieved Higher Response Rates with Fewer Flu-like and Musculoskeletal Symptoms and Cytopenias Than PEG-Interferon Alfa in Phase IIb Study of 526 Treatment-Naive Hepatitis C Patients," April 2, 2011, Press Release from Bristol-Myers Squibb).

In various embodiments of the invention as defined in the claims, the subject leukocyte redirecting bispecific antibodies, ADCs and/or checkpoint inhibitor mAbs may be used in combination with one or more interferons, such as interferon-α, interferon-β, interferon-λ1, interferon-λ2, or interferon-λ3. When used with other agents, the interferon may be administered prior to, concurrently with, or after the other agent. When administered concurrently, the interferon may be either conjugated to or separate from the other agent.

### Checkpoint Inhibitor Antibodies

Studies with checkpoint inhibitor antibodies for cancer therapy have generated unprecedented response rates in cancers previously thought to be resistant to cancer treatment (see, e.g., Ott & Bhardwaj, 2013, Frontiers in Immunology 4:346; Menzies & Long, 2013, Ther Adv Med Oncol 5:278-85; Pardoll, 2012, Nature Reviews Cancer 12:252-64; Mavilio & Lugli, ). Therapy with antagonistic checkpoint blocking antibodies against immune system checkpoints such as CTLA4, PD1 and PD-L1 are one of the most promising new avenues of immunotherapy for cancer and other diseases. In contrast to the majority of anti-cancer agents, checkpoint inhibitors do not target tumor cells directly, but rather target lymphocyte receptors or their ligands in order to enhance the endogenous antitumor activity of the immune system. (Pardoll, 2012, Nature Reviews Cancer 12:252-264) Because such antibodies act primarily by regulating the immune response to diseased cells, tissues or pathogens, they may be used in combination with other therapeutic modalities, such as the subject leukocyte redirecting bispecific antibodies, ADCs and/or interferons to enhance the anti-tumor effect of such agents.

It is now clear that tumors can escape immune surveillance by co-opting certain immune-checkpoint pathways, particularly in T cells that are specific for tumor antigens (Pardoll, 2012, Nature Reviews Cancer 12:252-264). Because many such immune checkpoints are initiated by ligand-receptor interactions, they can be readily blocked by antibodies against the ligands and/or their receptors (Pardoll, 2012, Nature Reviews Cancer 12:252-264). Although checkpoint inhibitor antibodies against CTLA4, PD1 and PD-L1 are the most clinically advanced, other potential checkpoint antigens are known and may be used as the target of therapeutic antibodies, such as LAG3, B7-H3, B7-H4 and TIM3 (Pardoll, 2012, Nature Reviews Cancer 12:252-264).

Programmed cell death protein 1 (PD1, also known as CD279) encodes a cell surface membrane protein of the immunoglobulin superfamily, which is expressed in B cells and NK cells (Shinohara et al., 1995, Genomics 23:704-6; Blank et al., 2007, Cancer Immunol Immunother 56:739-45; Finger et al., 1997, Gene 197:177-87; Pardoll, 2012, Nature Reviews Cancer 12:252-264). The major role of PD1 is to limit the activity of T cells in peripheral tissues during inflammation in response to infection, as well as to limit autoimmunity (Pardoll, 2012, Nature Reviews Cancer 12:252-264). PD1 expression is induced in activated T cells and binding of PD1 to one of its endogenous ligants acts to inhibit T-cell activation by inhibiting stimulatory kinases (Pardoll, 2012, Nature Reviews Cancer 12:252-264). PD1 also acts to inhibit the TCR "stop signal" (Pardoll, 2012, Nature Reviews Cancer 12:252-264). PD1 is highly expressed on T_{reg} cells and may increase their proliferation in the presence of ligand (Pardoll, 2012, Nature Reviews Cancer 12:252-264).

Anti-PD1 antibodies have been used for treatment of melanoma, non-small-cell lung cancer, bladder cancer, prostate cancer, colorectal cancer, head and neck cancer, triple-negative breast cancer, leukemia, lymphoma and renal cell cancer (Topalian et al., 2012, N Engl J Med 366:2443-54; Lipson et al., 2013, Clin Cancer Res 19:462-8; Berger et al., 2008, Clin Cancer Res 14:3044-51; Gildener-Leapman et al., 2013, Oral Oncol 49:1089-96; Menzies & Long, 2013, Ther Adv Med Oncol 5:278-85). Because PD1/PD-L1 and CTLA4 act by different pathways, it is possible that combination therapy with checkpoint inhibitor antibodies against each may provide an enhanced immune response.

Exemplary anti-PD1 antibodies include lambrolizumab (MK-3475, MERCK), nivolumab (BMS-936558, BRISTOL-MYERS SQUIBB), AMP-224 (MERCK), and pidilizumab (CT-011, CURETECH LTD.). Anti-PD1 antibodies are commercially available, for example from ABCAM^{®} (AB137132), BIOLEGEND^{®} (EH12.2H7, RMP1-14) and AFFYMETRIX EBIOSCIENCE (J105, J116, MIH4).

Programmed cell death 1 ligand 1 (PD-L1, also known as CD274 and B7-H1) is a ligand for PD1, found on activated T cells, B cells, myeloid cells and macrophages. Although there are two endogenous ligands for PD1 - PD-L1 and PD-L2, anti-tumor therapies have focused on anti-PD-L1 antibodies. The complex of PD1 and PD-L1 inhibits proliferation of CD8+ T cells and reduces the immune response (Topalian et al., 2012, N Engl J Med 366:2443-54; Brahmer et al., 2012, N Eng J Med 366:2455-65). Anti-PD-L1 antibodies have been used for treatment of non-small cell lung cancer, melanoma, colorectal cancer, renal-cell cancer, pancreatic cancer, gastric cancer, ovarian cancer, breast cancer, and hematologic malignancies (Brahmer et al., N Eng J Med 366:2455-65; Ott et al., 2013, Clin Cancer Res 19:5300-9; Radvanyi et al., 2013, Clin Cancer Res 19:5541; Menzies & Long, 2013, Ther Adv Med Oncol 5:278-85; Berger et al., 2008, Clin Cancer Res 14:13044-51).

Exemplary anti-PD-L1 antibodies include MDX-1105 (MEDAREX), MEDI4736 (MEDIMMUNE) MPDL3280A (GENENTECH) and BMS-936559 (BRISTOL-MYERS SQUIBB). Anti-PD-L1 antibodies are also commercially available, for example from AFFYMETRIX EBIOSCIENCE (MIH1).

Cytotoxic T-lymphocyte antigen 4 (CTLA4, also known as CD152) is also a member of the immunoglobulin superfamily that is expressed exclusively on T-cells. CTLA4 acts to inhibit T-cell activation and is reported to inhibit helper T-cell activity and enhance regulatory T-cell immunosuppressive activity (Pardoll, 2012, Nature Reviews Cancer 12:252-264). Although the precise mechanism of action of CTL4-A remains under investigation, it has been suggested that it inhibits T cell activation by outcompeting CD28 in binding to CD80 and CD86, as well as actively delivering inhibitor signals to the T cell (Pardoll, 2012, Nature Reviews Cancer 12:252-264). Anti-CTL4A antibodies have been used in clinical trials for treatment of melanoma, prostate cancer, small cell lung cancer, non-small cell lung cancer (Robert & Ghiringhelli, 2009, Oncologist 14:848-61; Ott et al., 2013, Clin Cancer Res 19:5300; Weber, 2007, Oncologist 12:864-72; Wada et al., 2013, J Transl Med 11:89). A significant feature of anti-CTL4A is the kinetics of anti-tumor effect, with a lag period of up to 6 months after initial treatment required for physiologic response (Pardoll, 2012, Nature Reviews Cancer 12:252-264). In some cases, tumors may actually increase in size after treatment initiation, before a reduction is seen (Pardoll, 2012, Nature Reviews Cancer 12:252-264).

Exemplary anti-CTLA4 antibodies include ipilimumab (Bristol-Myers Squibb) and tremelimumab (PFIZER). Anti-PD1 antibodies are commercially available, for example from ABCAM^{®} (AB134090), SINO BIOLOGICAL INC. (11159-H03H, 11159-H08H), and THERMO SCIENTIFIC PIERCE (PA5-29572, PA5-23967, PA5-26465, MA1-12205, MA1-35914). Ipilimumab has recently received FDA approval for treatment of metastatic melanoma (Wada et al., 2013, J Transl Med 11:89).

The person of ordinary skill will realize that methods of determining optimal dosages of checkpoint inhibitor antibodies to administer to a patient in need thereof, either alone or in combination with one or more other agents, may be determined by standard dose-response and toxicity studies that are well known in the art. In an exemplary embodiment, a checkpoint inhibitor antibody may preferably be administered at about 0.3-10 mg/kg, or the maximum tolerated dose, administered about every three weeks or about every six weeks. Alternatively, the checkpoint inhibitor antibody may be administered by an escalating dosage regimen including administering a first dosage at about 3 mg/kg, a second dosage at about 5 mg/kg, and a third dosage at about 9 mg/kg. Alternatively, the escalating dosage regimen includes administering a first dosage of checkpoint inhibitor antibody at about 5 mg/kg and a second dosage at about 9 mg/kg. Another stepwise escalating dosage regimen may include administering a first dosage of checkpoint inhibitor antibody about 3 mg/kg, a second dosage of about 3 mg/kg, a third dosage of about 5 mg/kg, a fourth dosage of about 5 mg/kg, and a fifth dosage of about 9 mg/kg. In another aspect, a stepwise escalating dosage regimen may include administering a first dosage of 5 mg/kg, a second dosage of 5 mg/kg, and a third dosage of 9 mg/kg. Exemplary reported dosages of checkpoint inhibitor mAbs include 3 mg/kg ipilimumab administered every three weeks for four doses; 10 mg/kg ipilimumab every three weeks for eight cycles; 10 mg/kg every three weeks for four cycles then every 12 weeks for a total of three years; 10 mg/kg MK-3475 every two or every three weeks; 2 mg/kg MK-3475 every three weeks; 15 mg/kg tremilimumab every three months; 0.1, 0.3, 1, 3 or 10 mg/kg nivolumab every two weeks for up to 96 weeks; 0.3, 1, 3, or 10 mg/kg BMS-936559 every two weeks for up to 96 weeks (Kyi & Postow, October 23, 2013, FEBS Lett [Epub ahead of print]; Callahan & Wolchok, 2013, J Leukoc Biol 94:41-53).

These and other known agents that stimulate immune response to tumors and/or pathogens may be used in combination with leukocyte redirecting bispecific antibodies alone or in further combination with an interferon, such as interferon-α, and/or an antibody-drug conjugate for improved cancer therapy. Other known co-stimulatory pathway modulators that may be used in combination include, but are not limited to, agatolimod, belatacept, blinatumomab, CD40 ligand, anti-B7-1 antibody, anti-B7-2 antibody, anti-B7-H4 antibody, AG4263, eritoran, anti-OX40 antibody, ISF-154, and SGN-70; B7-1, B7-2, ICAM-1, ICAM-2, ICAM-3, CD48, LFA-3, CD30 ligand, CD40 ligand, heat stable antigen, B7h, OX40 ligand, LIGHT, CD70 and CD24.

In certain embodiments of the invention as defined in the claims, anti-KIR antibodies may also be used in combination with leukocyte-redirecting bsAbs, interferons, ADCs and/or checkpoint inhibitor antibodies. NK cells mediate anti-tumor and anti-infectious agent activity by spontaneous cytotoxicity and by ADCC when activated by antibodies (Kohrt et al., 2013, Blood, [Epub ahead of print 12/10/13]). The degree of cytotoxic response is determined by a balance of inhibitory and activating signals received by the NK cells (Kohrt et al., 2013). The killer cell immunoglobulin-like receptor (KIR) mediates an inhibitory signal that decreases NK cell response. Anti-KIR antibodies, such as lirlumab (Innate Pharma) and IPH2101 (Innate Pharma) have demonstrated anti-tumor activity in multiple myeloma (Benson et al., 2012, Blood 120:4324-33). *In vitro,* anti-KIR antibodies prevent the tolerogenic interaction of NK cells with target cells and augments the NK cell cytotoxic response to tumor cells (Kohrt et al., 2013). *In vivo,* in combination with rituximab (anti-CD20), anti-KIR antibodies at a dose of 0.5 mg/kg induced enhanced NK cell-mediated, rituximab-dependent cytotoxicity against lymphoma tumors (Kohrt et al., 2013). Anti-KIR mAbs may be combined with ADCs, leukocyte-redirecting bsAbs, interferons and/or checkpoint inhibitor antibodies to potentiate cytotoxicity to tumor cells or pathogenic organisms.

### General Antibody Techniques

Techniques for preparing monoclonal antibodies against virtually any target antigen are well known in the art. *See,* for example, Kohler and Milstein, Nature 256: 495 (1975), and Coligan et al. (eds.), CURRENT PROTOCOLS IN IMMUNOLOGY, VOL. 1, pages 2.5.1-2.6.7 (John Wiley & Sons 1991). Briefly, monoclonal antibodies can be obtained by injecting mice with a composition comprising an antigen, removing the spleen to obtain B-lymphocytes, fusing the B-lymphocytes with myeloma cells to produce hybridomas, cloning the hybridomas, selecting positive clones which produce antibodies to the antigen, culturing the clones that produce antibodies to the antigen, and isolating the antibodies from the hybridoma cultures.

MAbs can be isolated and purified from hybridoma cultures by a variety of well-established techniques. Such isolation techniques include affinity chromatography with Protein-A Sepharose, size-exclusion chromatography, and ion-exchange chromatography. See, for example, Coligan at pages 2.7.1-2.7.12 and pages 2.9.1-2.9.3. Also, see Baines et al., "Purification of Immunoglobulin G (IgG)," in METHODS IN MOLECULAR BIOLOGY, VOL. 10, pages 79-104 (The Humana Press, Inc. 1992).

After the initial raising of antibodies to the immunogen, the antibodies can be sequenced and subsequently prepared by recombinant techniques. Humanization and chimerization of murine antibodies and antibody fragments are well known to those skilled in the art. The use of antibody components derived from humanized, chimeric or human antibodies obviates potential problems associated with the immunogenicity of murine constant regions.

### Chimeric Antibodies

A chimeric antibody is a recombinant protein in which the variable regions of a human antibody have been replaced by the variable regions of, for example, a mouse antibody, including the complementarity-determining regions (CDRs) of the mouse antibody. Chimeric antibodies exhibit decreased immunogenicity and increased stability when administered to a subject. General techniques for cloning murine immunoglobulin variable domains are disclosed, for example, in Orlandi et al., Proc. Nat'l Acad. Sci. USA 86: 3833 (1989). Techniques for constructing chimeric antibodies are well known to those of skill in the art. As an example, Leung et al., Hybridoma 13:469 (1994), produced an LL2 chimera by combining DNA sequences encoding the V_{κ} and V_{H} domains of murine LL2, an anti-CD22 monoclonal antibody, with respective human κ and IgG₁ constant region domains.

### Humanized Antibodies

Techniques for producing humanized MAbs are well known in the art (see, e.g., Jones et al., Nature 321: 522 (1986), Riechmann et al., Nature 332: 323 (1988), Verhoeyen et al., Science 239: 1534 (1988), Carter et al., Proc. Nat'l Acad. Sci. USA 89: 4285 (1992), Sandhu, Crit. Rev. Biotech. 12: 437 (1992), and Singer et al., J. Immun. 150: 2844 (1993)). A chimeric or murine monoclonal antibody may be humanized by transferring the mouse CDRs from the heavy and light variable chains of the mouse immunoglobulin into the corresponding variable domains of a human antibody. The mouse framework regions (FR) in the chimeric monoclonal antibody are also replaced with human FR sequences. As simply transferring mouse CDRs into human FRs often results in a reduction or even loss of antibody affinity, additional modification might be required in order to restore the original affinity of the murine antibody. This can be accomplished by the replacement of one or more human residues in the FR regions with their murine counterparts to obtain an antibody that possesses good binding affinity to its epitope. See, for example, Tempest et al., Biotechnology 9:266 (1991) and Verhoeyen et al., Science 239: 1534 (1988). Generally, those human FR amino acid residues that differ from their murine counterparts and are located close to or touching one or more CDR amino acid residues would be candidates for substitution.

### Human Antibodies

Methods for producing fully human antibodies using either combinatorial approaches or transgenic animals transformed with human immunoglobulin loci are known in the art (*e.g.,* Mancini et al., 2004, New Microbiol. 27:315-28; Conrad and Scheller, 2005, Comb. Chem. High Throughput Screen. 8:117-26; Brekke and Loset, 2003, Curr. Opin. Phamacol. 3:544-50). A fully human antibody also can be constructed by genetic or chromosomal transfection methods, as well as phage display technology, all of which are known in the art. See for example, McCafferty et al., Nature 348:552-553 (1990). Such fully human antibodies are expected to exhibit even fewer side effects than chimeric or humanized antibodies and to function *in vivo* as essentially endogenous human antibodies. In certain embodiments, the claimed methods and procedures may utilize human antibodies produced by such techniques.

In one alternative, the phage display technique may be used to generate human antibodies (*e.g.,* Dantas-Barbosa et al., 2005, Genet. Mol. Res. 4:126-40). Human antibodies may be generated from normal humans or from humans that exhibit a particular disease state, such as cancer (Dantas-Barbosa et al., 2005). The advantage to constructing human antibodies from a diseased individual is that the circulating antibody repertoire may be biased towards antibodies against disease-associated antigens.

In one non-limiting example of this methodology, Dantas-Barbosa et al. (2005) constructed a phage display library of human Fab antibody fragments from osteosarcoma patients. Generally, total RNA was obtained from circulating blood lymphocytes (*Id*.). Recombinant Fab were cloned from the µ, γ and κ chain antibody repertoires and inserted into a phage display library (*Id*.). RNAs were converted to cDNAs and used to make Fab cDNA libraries using specific primers against the heavy and light chain immunoglobulin sequences (Marks et al., 1991, J. Mol. Biol. 222:581-97). Library construction was performed according to Andris-Widhopf et al. (2000, In: PHAGE DISPLAY LABORATORY MANUAL, Barbas et al. (eds), 1st edition, Cold Spring Harbor Laboratory Press, Cold Spring Harbor, NY pp. 9.1 to 9.22). The final Fab fragments were digested with restriction endonucleases and inserted into the bacteriophage genome to make the phage display library. Such libraries may be screened by standard phage display methods, as known in the art (see, e.g., Pasqualini and Ruoslahti, 1996, Nature 380:364-366; Pasqualini, 1999, The Quart. J. Nucl. Med. 43:159-162).

Phage display can be performed in a variety of formats, for their review, see e.g. Johnson and Chiswell, Current Opinion in Structural Biology 3:5564-571 (1993). Human antibodies may also be generated by *in vitro* activated B cells. See U.S. Patent Nos. 5,567,610 and 5,229,275. The skilled artisan will realize that these techniques are exemplary and any known method for making and screening human antibodies or antibody fragments may be utilized.

In another alternative, transgenic animals that have been genetically engineered to produce human antibodies may be used to generate antibodies against essentially any immunogenic target, using standard immunization protocols. Methods for obtaining human antibodies from transgenic mice are disclosed by Green et al., Nature Genet. 7:13 (1994), Lonberg et al., Nature 368:856 (1994), and Taylor et al., Int. Immun. 6:579 (1994). A non-limiting example of such a system is the XENOMOUSE^{®} (e.g., Green et al., 1999, J. Immunol. Methods 231:11-23) from Abgenix (Fremont, CA). In the XENOMOUSE^{®} and similar animals, the mouse antibody genes have been inactivated and replaced by functional human antibody genes, while the remainder of the mouse immune system remains intact.

The XENOMOUSE^{®} was transformed with germline-configured YACs (yeast artificial chromosomes) that contained portions of the human IgH and Igkappa loci, including the majority of the variable region sequences, along accessory genes and regulatory sequences. The human variable region repertoire may be used to generate antibody producing B cells, which may be processed into hybridomas by known techniques. A XENOMOUSE^{®} immunized with a target antigen will produce human antibodies by the normal immune response, which may be harvested and/or produced by standard techniques discussed above. A variety of strains of XENOMOUSE^{®} are available, each of which is capable of producing a different class of antibody. Transgenically produced human antibodies have been shown to have therapeutic potential, while retaining the pharmacokinetic properties of normal human antibodies (Green et al., 1999). The skilled artisan will realize that the claimed compositions and methods are not limited to use of the XENOMOUSE^{®} system but may utilize any transgenic animal that has been genetically engineered to produce human antibodies.

### Antibody Cloning and Production

Various techniques, such as production of chimeric or humanized antibodies, may involve procedures of antibody cloning and construction. The antigen-binding Vκ (variable light chain) and V_{H} (variable heavy chain) sequences for an antibody of interest may be obtained by a variety of molecular cloning procedures, such as RT-PCR, 5'-RACE, and cDNA library screening. The V genes of an antibody from a cell that expresses a murine antibody can be cloned by PCR amplification and sequenced. To confirm their authenticity, the cloned V_{L} and V_{H} genes can be expressed in cell culture as a chimeric Ab as described by Orlandi et al., (Proc. Natl. Acad. Sci. USA, 86: 3833 (1989)). Based on the V gene sequences, a humanized antibody can then be designed and constructed as described by Leung et al. (Mol. Immunol., 32: 1413 (1995)).

cDNA can be prepared from any known hybridoma line or transfected cell line producing a murine antibody by general molecular cloning techniques (Sambrook et al., Molecular Cloning, A laboratory manual, 2nd Ed (1989)). The Vκ sequence for the antibody may be amplified using the primers VK1BACK and VK1FOR (Orlandi *et al.,* 1989) or the extended primer set described by Leung et al. (BioTechniques, 15: 286 (1993)). The V_{H} sequences can be amplified using the primer pair VH1BACK/VH1FOR (Orlandi *et al.,* 1989) or the primers annealing to the constant region of murine IgG described by Leung et al. (*Hybridoma,* 13:469 (1994)). Humanized V genes can be constructed by a combination of long oligonucleotide template syntheses and PCR amplification as described by Leung et al. (*Mol. Immunol.,* 32: 1413 (1995)).

PCR products for Vκ can be subcloned into a staging vector, such as a pBR327-based staging vector, VKpBR, that contains an Ig promoter, a signal peptide sequence and convenient restriction sites. PCR products for V_{H} can be subcloned into a similar staging vector, such as the pBluescript-based VHpBS. Expression cassettes containing the Vκ and V_{H} sequences together with the promoter and signal peptide sequences can be excised from VKpBR and VHpBS and ligated into appropriate expression vectors, such as pKh and pG1g, respectively (Leung et al., Hybridoma, 13:469 (1994)). The expression vectors can be co-transfected into an appropriate cell and supernatant fluids monitored for production of a chimeric, humanized or human antibody. Alternatively, the Vκ and V_{H} expression cassettes can be excised and subcloned into a single expression vector, such as pdHL2, as described by Gillies et al. (J. Immunol. Methods 125:191 (1989) and also shown in Losman et al., Cancer, 80:2660 (1997)).

Alternatively, expression vectors may be transfected into host cells that have been pre-adapted for transfection, growth and expression in serum-free medium. Exemplary cell lines that may be used include the Sp/EEE, Sp/ESF and Sp/ESF-X cell lines (see, e.g., U.S.

Patent Nos. 7,531,327; 7,537,930 and 7,608,425). These exemplary cell lines are based on the Sp2/0 myeloma cell line, transfected with a mutant Bcl-EEE gene, exposed to methotrexate to amplify transfected gene sequences and pre-adapted to serum-free cell line for protein expression.

### Antibody Fragments

Antibody fragments which recognize specific epitopes can be generated by known techniques. Antibody fragments are antigen binding portions of an antibody, such as F(ab')₂, Fab', F(ab)₂, Fab, Fv, scFv and the like. F(ab')₂ fragments can be produced by pepsin digestion of the antibody molecule and Fab' fragments can be generated by reducing disulfide bridges of the F(ab')₂ fragments. Alternatively, Fab' expression libraries can be constructed (Huse et al., 1989, Science, 246:1274-1281) to allow rapid and easy identification of monoclonal Fab' fragments with the desired specificity. F(ab)₂ fragments may be generated by papain digestion of an antibody.

A single chain Fv molecule (scFv) comprises a VL domain and a VH domain. The VL and VH domains associate to form a target binding site. These two domains are further covalently linked by a peptide linker (L). Methods for making scFv molecules and designing suitable peptide linkers are described in US Patent No. 4,704,692; US Patent No. 4,946,778; Raag and Whitlow, FASEB 9:73-80 (1995) and Bird and Walker, TIBTECH, 9: 132-137 (1991).

Techniques for producing single domain antibodies (DABs or VHH) are also known in the art, as disclosed for example in Cossins et al. (2006, Prot Express Purif 51 :253-259). Single domain antibodies may be obtained, for example, from camels, alpacas or llamas by standard immunization techniques. (See, e.g., Muyldermans et al., TIBS 26:230-235, 2001; Yau et al., J Immunol Methods 281:161-75, 2003; Maass et al., J Immunol Methods 324:13-25, 2007). The VHH may have potent antigen-binding capacity and can interact with novel epitopes that are inacessible to conventional VH-VL pairs. (Muyldermans et al., 2001). Alpaca serum IgG contains about 50% camelid heavy chain only IgG antibodies (HCAbs) (Maass et al., 2007). Alpacas may be immunized with known antigens, such as TNF-α, and VHHs can be isolated that bind to and neutralize the target antigen (Maass et al., 2007). PCR primers that amplify virtually all alpaca VHH coding sequences have been identified and may be used to construct alpaca VHH phage display libraries, which can be used for antibody fragment isolation by standard biopanning techniques well known in the art (Maass et al., 2007). In certain embodiments, anti-pancreatic cancer VHH antibody fragments may be utilized in the claimed compositions and methods.

An antibody fragment can be prepared by proteolytic hydrolysis of the full-length antibody or by expression in *E. coli* or another host of the DNA coding for the fragment. An antibody fragment can be obtained by pepsin or papain digestion of full-length antibodies by conventional methods. These methods are described, for example, by Goldenberg, U.S. Patent Nos. 4,036,945 and 4,331,647 and references contained therein. Also, see Nisonoff et al., Arch Biochem. Biophys. 89: 230 (1960); Porter, Biochem. J. 73: 119 (1959), Edelman et al., in METHODS IN ENZYMOLOGY VOL. 1, page 422 (Academic Press 1967), and Coligan at pages 2.8.1-2.8.10 and 2.10.-2.10.4.

### Antibody Allotypes

Immunogenicity of therapeutic antibodies is associated with increased risk of infusion reactions and decreased duration of therapeutic response (Baert et al., 2003, N Engl J Med 348:602-08). The extent to which therapeutic antibodies induce an immune response in the host may be determined in part by the allotype of the antibody (Stickler et al., 2011, Genes and Immunity 12:213-21). Antibody allotype is related to amino acid sequence variations at specific locations in the constant region sequences of the antibody. The allotypes of IgG antibodies containing a heavy chain γ-type constant region are designated as Gm allotypes (1976, J Immunol 117:1056-59).

For the common IgG1 human antibodies, the most prevalent allotype is G1m1 (Stickler et al., 2011, Genes and Immunity 12:213-21). However, the G1m3 allotype also occurs frequently in Caucasians (Stickler et al., 2011). It has been reported that G1m1 antibodies contain allotypic sequences that tend to induce an immune response when administered to non-G1m1 (nG1m1) recipients, such as G1m3 patients (Stickler et al., 2011). Non-G1m1 allotype antibodies are not as immunogenic when administered to G1m1 patients (Stickler et al., 2011).

The human G 1m 1 allotype comprises the amino acids aspartic acid at Kabat position 356 and leucine at Kabat position 358 in the CH3 sequence of the heavy chain IgG1. The nG1m1 allotype comprises the amino acids glutamic acid at Kabat position 356 and methionine at Kabat position 358. Both G1ml and nG1ml allotypes comprise a glutamic acid residue at Kabat position 357 and the allotypes are sometimes referred to as DEL and EEM allotypes. A non-limiting example of the heavy chain constant region sequences for G 1m 1 and nG 1m 1 allotype antibodies is shown for the exemplary antibodies rituximab (SEQ ID NO:85) and veltuzumab (SEQ ID NO:86).

### Rituximab heavy chain variable region sequence (SEQ ID NO:85)

ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKAEPKSCDKTHTCPPC
PAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPG
K

### Veltuzumab heavy chain variable region (SEQ ID NO:86)

ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCP
APELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISK
AKGQPREPQVYTLPPSREEMTKNQVSLTCLVKGFYPSDIAVEWESNGQPENNYK
TTPPVLDSDGSFFLYSKLTVDKSRWQQGNVFSCSVMHEALHNHYTQKSLSLSPG
K

Jefferis and Lefranc (2009, mAbs 1:1-7) reviewed sequence variations characteristic of IgG allotypes and their effect on immunogenicity. They reported that the G1m3 allotype is characterized by an arginine residue at Kabat position 214, compared to a lysine residue at Kabat 214 in the G1m17 allotype. The nG1m1,2 allotype was characterized by glutamic acid at Kabat position 356, methionine at Kabat position 358 and alanine at Kabat position 431. The G1m1,2 allotype was characterized by aspartic acid at Kabat position 356, leucine at Kabat position 358 and glycine at Kabat position 431. In addition to heavy chain constant region sequence variants, Jefferis and Lefranc (2009) reported allotypic variants in the kappa light chain constant region, with the Km1 allotype characterized by valine at Kabat position 153 and leucine at Kabat position 191, the Km1,2 allotype by alanine at Kabat position 153 and leucine at Kabat position 191, and the Km3 allotype characterized by alanine at Kabat position 153 and valine at Kabat position 191.

With regard to therapeutic antibodies, veltuzumab and rituximab are, respectively, humanized and chimeric IgG1 antibodies against CD20, of use for therapy of a wide variety of hematological malignancies and/or autoimmune diseases. **Table 1** compares the allotype sequences of rituximab vs. veltuzumab. As shown in **Table 1,** rituximab (G1m17,1) is a DEL allotype IgG1, with an additional sequence variation at Kabat position 214 (heavy chain CH1) of lysine in rituximab vs. arginine in veltuzumab. It has been reported that veltuzumab is less immunogenic in subjects than rituximab (*see, e.g.,* Morchhauser et al., 2009, J Clin Oncol 27:3346-53; Goldenberg et al., 2009, Blood 113:1062-70; Robak & Robak, 2011, BioDrugs 25:13-25), an effect that has been attributed to the difference between humanized and chimeric antibodies. However, the difference in allotypes between the EEM and DEL allotypes likely also accounts for the lower immunogenicity of veltuzumab.

**Table 1. Allotypes of Rituximab vs. Veltuzumab**

| | | Heavy chain position and associated allotypes | | | | | |
|---|---|---|---|---|---|---|---|
| | Complete allotype | 214 (allotype) | | 356/358 (allotype) | | 431 (allotype) | |
| Rituximab | G1m17,1 | K | 17 | DAL | 1 | A | - |
| Veltuzumab | G1m3 | R | 3 | E/M | - | A | - |

In order to reduce the immunogenicity of therapeutic antibodies in individuals of nG1m1 genotype, it is desirable to select the allotype of the antibody to correspond to the G1m3 allotype, characterized by arginine at Kabat 214, and the nG1m1,2 null-allotype, characterized by glutamic acid at Kabat position 356, methionine at Kabat position 358 and alanine at Kabat position 431. Surprisingly, it was found that repeated subcutaneous administration of G1m3 antibodies over a long period of time did not result in a significant immune response. In alternative embodiments, the human IgG4 heavy chain in common with the G1m3 allotype has arginine at Kabat 214, glutamic acid at Kabat 356, methionine at Kabat 359 and alanine at Kabat 431. Since immunogenicity appears to relate at least in part to the residues at those locations, use of the human IgG4 heavy chain constant region sequence for therapeutic antibodies is also a preferred embodiment. Combinations of G1m3 IgG1 antibodies with IgG4 antibodies may also be of use for therapeutic administration.

### Known Antibodies

### Target Antigens and Exemplary Antibodies

According to the instant disclosure, antibodies may be used that recognize and/or bind to antigens that are expressed at high levels on target cells and that are expressed predominantly or exclusively on diseased cells versus normal tissues. Exemplary antibodies of use for therapy of, for example, cancer include but are not limited to LL1 (anti-CD74), LL2 or RFB4 (anti-CD22), veltuzumab (hA20, anti-CD20), rituxumab (anti-CD20), obinutuzumab (GA101, anti-CD20), lambrolizumab (anti-PD1), nivolumab (anti-PD1),MK-3475 (anti-PD1), AMP-224 (anti-PD1), pidilizumab (anti-PD1), MDX-1105 (anti-PD-L1), MEDI4736 (anti-PD-L1), MPDL3280A (anti-PD-L1), BMS-936559 (anti-PD-L1), ipilimumab (anti-CTLA4), trevilizumab (anti-CTL4A), RS7 (anti-epithelial glycoprotein-1 (EGP-1, also known as TROP-2)), PAM4 or KC4 (both anti-mucin), MN-14 (anti-carcinoembryonic antigen (CEA, also known as CD66e or CEACAM5), MN-15 or MN-3 (anti-CEACAM6), Mu-9 (anti-colon-specific antigen-p), Immu 31 (an anti-alpha-fetoprotein), R1 (anti-IGF-1R), A19 (anti-CD19), TAG-72 (e.g., CC49), Tn, J591 or HuJ591 (anti-PSMA (prostate-specific membrane antigen)), AB-PG1-XG1-026 (anti-PSMA dimer), D2/B (anti-PSMA), G250 (an anti-carbonic anhydrase IX MAb), L243 (anti-HLA-DR) alemtuzumab (anti-CD52), bevacizumab (anti-VEGF), cetuximab (anti-EGFR), gemtuzumab (anti-CD33), ibritumomab tiuxetan (anti-CD20); panitumumab (anti-EGFR); tositumomab (anti-CD20); PAM4 (aka clivatuzumab, anti-mucin), BWA-3 (anti-histone H2A/H4), LG2-1 (anti-histone H3), MRA12 (anti-histone H1), PR1-1 (anti-histone H2B), LG11-2 (anti-histone H2B), LG2-2 (anti-histone H2B), and trastuzumab (anti-ErbB2). Such antibodies are known in the art (e.g., U.S. Patent Nos. 5,686,072; 5,874,540; 6,107,090; 6,183,744; 6,306,393; 6,653,104; 6,730.300; 6,899,864; 6,926,893; 6,962,702; 7,074,403; 7,230,084; 7,238,785; 7,238,786; 7,256,004; 7,282,567; 7,300,655; 7,312,318; 7,585,491; 7,612,180; 7,642,239; and U.S. Patent Application Publ. No. 20050271671; 20060193865; 20060210475; 20070087001.) Specific known antibodies of use include hPAM4 (U.S. Patent No. 7,282,567), hA20 (U.S. Patent No. 7,251,164), hA19 (U.S. Patent No. 7,109,304), hIMMU-31 (U.S. Patent No. 7,300,655), hLL1 (U.S. Patent No. 7,312,318, ), hLL2 (U.S. Patent No. 7,074,403), hMu-9 (U.S. Patent No. 7,387,773), hL243 (U.S. Patent No. 7,612,180), hMN-14 (U.S. Patent No. 6,676,924), hMN-15 (U.S. Patent No. 7,541,440), hR1 (U.S. Patent Application 12/772,645), hRS7 (U.S. Patent No. 7,238,785), hMN-3 (U.S. Patent No. 7,541,440), AB-PG1-XG1-026 (U.S. Patent Application 11/983,372, deposited as ATCC PTA-4405 and PTA-4406) and D2/B (WO 2009/130575).

Other useful antigens that may be targeted using the described conjugates include carbonic anhydrase IX, B7, CCCL19, CCCL21, CSAp, HER-2/new, BrE3, CD1, CD1a, CD2, CD3, CD4, CD5, CD8, CD11A, CD14, CD15, CD16, CD18, CD19, CD20 (e.g., C2B8, hA20, 1F5 MAbs), CD21, CD22, CD23, CD25, CD29, CD30, CD32b, CD33, CD37, CD38, CD40, CD40L, CD44, CD45, CD46, CD52, CD54, CD55, CD59, CD64, CD67, CD70, CD74, CD79a, CD80, CD83, CD95, CD126, CD133, CD138, CD147, CD154, CEACAM5, CEACAM6, CTLA4, alpha-fetoprotein (AFP), VEGF (e.g., AVASTIN^{®}, fibronectin splice variant), ED-B fibronectin (e.g., L19), EGP-1 (TROP-2), EGP-2 (e.g., 17-1A), EGF receptor (ErbB1) (e.g., ERBITUX^{®}), ErbB2, ErbB3, Factor H, FHL-1, Flt-3, folate receptor, Ga 733,GRO-β, HMGB-1, hypoxia inducible factor (HIF), HM1.24, HER-2/neu, insulin-like growth factor (ILGF), IFN-γ, IFN-α, IFN-β, IFN-λ, IL-2R, IL-4R, IL-6R, IL-13R, IL-15R, IL-17R, IL-18R, IL-2, IL-6, IL-8, IL-12, IL-15, IL-17, IL-18, IL-25, IP-10, IGF-1R, Ia, HM1.24, gangliosides, HCG, the HLA-DR antigen to which L243 binds, CD66 antigens, i.e., CD66a-d or a combination thereof, MAGE, mCRP, MCP-1, MIP-1A, MIP-1B, macrophage migration-inhibitory factor (MIF), MUC1, MUC2, MUC3, MUC4, MUC5ac, placental growth factor (PIGF), PSA (prostate-specific antigen), PSMA, PAM4 antigen, PD1 receptor, NCA-95, NCA-90, A3, A33, Ep-CAM, KS-1, Le(y), mesothelin, S100, tenascin, TAC, Tn antigen, Thomas-Friedenreich antigens, tumor necrosis antigens, tumor angiogenesis antigens, TNF-α, TRAIL receptor (R1 and R2), TROP-2, VEGFR, RANTES, T101, as well as cancer stem cell antigens, complement factors C3, C3a, C3b, C5a, C5, and an oncogene product.

A comprehensive analysis of suitable antigen (**C**luster **D**esignation, or CD) targets on hematopoietic malignant cells, as shown by flow cytometry and which can be a guide to selecting suitable antibodies for immunotherapy, is Craig and Foon, Blood prepublished online January 15, 2008; DOL 10.1182/blood-2007-11-120535.

The CD66 antigens consist of five different glycoproteins with similar structures, CD66a-e, encoded by the carcinoembryonic antigen (CEA) gene family members, BCG, CGM6, NCA, CGM1 and CEA, respectively. These CD66 antigens (e.g., CEACAM6) are expressed mainly in granulocytes, normal epithelial cells of the digestive tract and tumor cells of various tissues. Also included as suitable targets for cancers are cancer testis antigens, such as NY-ESO-1 (Theurillat et al., Int. J. Cancer 2007; 120(11):2411-7), as well as CD79a in myeloid leukemia (Kozlov et al., Cancer Genet. Cytogenet. 2005; 163(1):62-7) and also B-cell diseases, and CD79b for non-Hodgkin's lymphoma (Poison et al., Blood 110(2):616-623). A number of the aforementioned antigens are disclosed in U.S. Provisional Application Serial No. 60/426,379, entitled "Use of Multi-specific, Non-covalent Complexes for Targeted Delivery of Therapeutics," filed November 15, 2002. Cancer stem cells, which are ascribed to be more therapy-resistant precursor malignant cell populations (Hill and Perris, J. Natl. Cancer Inst. 2007; 99:1435-40), have antigens that can be targeted in certain cancer types, such as CD 133 in prostate cancer (Maitland et al., Ernst Schering Found. Sympos. Proc. 2006; 5:155-79), non-small-cell lung cancer (Donnenberg et al., J. Control Release 2007; 122(3):385-91), and glioblastoma (Beier et al., Cancer Res. 2007; 67(9):4010-5), and CD44 in colorectal cancer (Dalerba er al., Proc. Natl. Acad. Sci. USA 2007; 104(24)10158-63), pancreatic cancer (Li et al., Cancer Res. 2007; 67(3):1030-7), and in head and neck squamous cell carcinoma (Prince et al., Proc. Natl. Acad. Sci. USA 2007; 104(3)973-8).

Anti-cancer antibodies have been demonstrated to bind to histones in some case. Kato et al. (1991, Hum Antibodies Hybridomas 2:94-101) reported tha the lung cancer-specific human monoclonal antibody HB4C5 binds to histone H2B. Garzelli et al. (1994, Immunol Lett 39:277-82) observed that Epstein-Barr virus-transformed human B lymphocytes produce natural antibodies to histones. In certain embodiments, antibodies against histones may be of use in the subject combinations. Known anti-histone antibodies include, but are not limited to, BWA-3 (anti-histone H2A/H4), LG2-1 (anti-histone H3), MRA12 (anti-histone H1), PR1-1 (anti-histone H2B), LG11-2 (anti-histone H2B), and LG2-2 (anti-histone H2B) (see, e.g., Monestier et al., 1991, Eur J Immunol 21:1725-31; Monestier et al., 1993, Molec Immunol 30:1069-75).

For multiple myeloma therapy, suitable targeting antibodies have been described against, for example, CD38 and CD138 (Stevenson, Mol Med 2006; 12(11-12):345-346; Tassone et al., Blood 2004; 104(12):3688-96), CD74 (Stein et al., *ibid*.), CS1 (Tai et al., Blood 2008; 112(4):1329-37, and CD40 (Tai et al., 2005; Cancer Res. 65(13):5898-5906).

Macrophage migration inhibitory factor (MIF) is an important regulator of innate and adaptive immunity and apoptosis. It has been reported that CD74 is the endogenous receptor for MIF (Leng et al., 2003, J Exp Med 197:1467-76). The therapeutic effect of antagonistic anti-CD74 antibodies on MIF-mediated intracellular pathways may be of use for treatment of a broad range of disease states, such as cancers of the bladder, prostate, breast, lung, colon and chronic lymphocytic leukemia (e.g., Meyer-Siegler et al., 2004, BMC Cancer 12:34; Shachar & Haran, 2011, Leuk Lymphoma 52:1446-54). Milatuzumab (hLL1) is an exemplary anti-CD74 antibody of therapeutic use for treatment of MIF-mediated diseases.

An example of a most-preferred antibody/antigen pair is LL1, an anti-CD74 MAb (invariant chain, class II-specific chaperone, Ii) (see, e.g., U.S. Patent Nos. 6,653,104; 7,312,318). The CD74 antigen is highly expressed on B-cell lymphomas (including multiple myeloma) and leukemias, certain T-cell lymphomas, melanomas, colonic, lung, and renal cancers, glioblastomas, and certain other cancers (Ong et al., Immunology 98:296-302 (1999)). A review of the use of CD74 antibodies in cancer is contained in Stein et al., Clin Cancer Res. 2007 Sep 15;13(18 Pt 2):5556s-5563s. The diseases that are preferably treated with anti-CD74 antibodies include, but are not limited to, non-Hodgkin's lymphoma, Hodgkin's disease, melanoma, lung, renal, colonic cancers, glioblastome multiforme, histiocytomas, myeloid leukemias, and multiple myeloma.

The methods and compositions disclosed herein may utilize any of a variety of antibodies known in the art. Antibodies of use may be commercially obtained from a number of known sources. For example, a variety of antibody secreting hybridoma lines are available from the American Type Culture Collection (ATCC, Manassas, VA). A large number of antibodies against various disease targets, including but not limited to tumor-associated antigens, have been deposited at the ATCC and/or have published variable region sequences and are available for use in the disclosed methods and compositions. See, e.g., U.S. Patent Nos. 7,312,318; 7,282,567; 7,151,164; 7,074,403; 7,060,802; 7,056,509; 7,049,060; 7,045,132; 7,041,803; 7,041,802; 7,041,293; 7,038,018; 7,037,498; 7,012,133; 7,001,598; 6,998,468; 6,994,976; 6,994,852; 6,989,241; 6,974,863; 6,965,018; 6,964,854; 6,962,981; 6,962,813; 6,956,107; 6,951,924; 6,949,244; 6,946,129; 6,943,020; 6,939,547; 6,921,645; 6,921,645; 6,921,533; 6,919,433; 6,919,078; 6,916,475; 6,905,681; 6,899,879; 6,893,625; 6,887,468; 6,887,466; 6,884,594; 6,881,405; 6,878,812; 6,875,580; 6,872,568; 6,867,006; 6,864,062; 6,861,511; 6,861,227; 6,861,226; 6,838,282; 6,835,549; 6,835,370; 6,824,780; 6,824,778; 6,812,206; 6,793,924; 6,783,758; 6,770,450; 6,767,711; 6,764,688; 6,764,681; 6,764,679; 6,743,898; 6,733,981; 6,730,307; 6,720,155; 6,716,966; 6,709,653; 6,693,176; 6,692,908; 6,689,607; 6,689,362; 6,689,355; 6,682,737; 6,682,736; 6,682,734; 6,673,344; 6,653,104; 6,652,852; 6,635,482; 6,630,144; 6,610,833; 6,610,294; 6,605,441; 6,605,279; 6,596,852; 6,592,868; 6,576,745; 6,572;856; 6,566,076; 6,562,618; 6,545,130; 6,544,749; 6,534,058; 6,528,625; 6,528,269; 6,521,227; 6,518,404; 6,511,665; 6,491,915; 6,488,930; 6,482,598; 6,482,408; 6,479,247; 6,468,531; 6,468,529; 6,465,173; 6,461,823; 6,458,356; 6,455,044; 6,455,040, 6,451,310; 6,444,206' 6,441,143; 6,432,404; 6,432,402; 6,419,928; 6,413,726; 6,406,694; 6,403,770; 6,403,091; 6,395,276; 6,395,274; 6,387,350; 6,383,759; 6,383,484; 6,376,654; 6,372,215; 6,359,126; 6,355,481; 6,355,444; 6,355,245; 6,355,244; 6,346,246; 6,344,198; 6,340,571; 6,340,459; 6,331,175; 6,306,393; 6,254,868; 6,187,287; 6,183,744; 6,129,914; 6,120,767; 6,096,289; 6,077,499; 5,922,302; 5,874,540; 5,814,440; 5,798,229; 5,789,554; 5,776,456; 5,736,119; 5,716,595; 5,677,136; 5,587,459; 5,443,953, 5,525,338. These are exemplary only and a wide variety of other antibodies and their hybridomas are known in the art. The skilled artisan will realize that antibody sequences or antibody-secreting hybridomas against almost any disease-associated antigen may be obtained by a simple search of the ATCC, NCBI and/or USPTO databases for antibodies against a selected disease-associated target of interest. The antigen binding domains of the cloned antibodies may be amplified, excised, ligated into an expression vector, transfected into an adapted host cell and used for protein production, using standard techniques well known in the art (see, e.g., U.S. Patent Nos. 7,531,327; 7,537,930; 7,608,425 and 7,785,880).

In other examples of the instant disclosure, the antibody complexes bind to a MHC class I, MHC class II or accessory molecule, such as CD40, CD54, CD80 or CD86. The antibody complex also may bind to a leukocyte activation cytokine, or to a cytokine mediator, such as NF-xB.

In certain examples, one of the two different targets may be a cancer cell receptor or cancer-associated antigen, particularly one that is selected from the group consisting of B-cell lineage antigens (CD19, CD20, CD21, CD22, CD23, etc.), VEGF, VEGFR, EGFR, carcinoembryonic antigen (CEA), placental growth factor (PIGF), tenascin, HER-2/neu, EGP-1, EGP-2, CD25, CD30, CD33, CD38, CD40, CD45, CD52, CD74, CD80, CD138, NCA66, CEACAM1, CEACAM6 (carcinoembryonic antigen-related cellular adhesion molecule 6), MUC1, MUC2, MUC3, MUC4, MUC16, IL-6, α-fetoprotein (AFP), A3, CA125, colon-specific antigen-p (CSAp), folate receptor, HLA-DR, human chorionic gonadotropin (HCG), Ia, EL-2, insulin-like growth factor (IGF) and IGF receptor, KS-1, Le(y), MAGE, necrosis antigens, PAM-4, prostatic acid phosphatase (PAP), Pr1, prostate specific antigen (PSA), prostate specific membrane antigen (PSMA), S100, T101, TAC, TAG72, TRAIL receptors, and carbonic anhydrase IX.

### Immunoconjugates

In certain embodiments of the invention as defined in the claims, the antibodies or fragments thereof may be conjugated to one or more therapeutic or diagnostic agents. The therapeutic agents do not need to be the same but can be different, e.g. a drug and a radioisotope. For example, ¹³¹I can be incorporated into a tyrosine of an antibody or fusion protein and a drug attached to an epsilon amino group of a lysine residue. Therapeutic and diagnostic agents also can be attached, for example to reduced SH groups and/or to carbohydrate side chains. Many methods for making covalent or non-covalent conjugates of therapeutic or diagnostic agents with antibodies or fusion proteins are known in the art and any such known method may be utilized.

A therapeutic or diagnostic agent can be attached at the hinge region of a reduced antibody component via disulfide bond formation. Alternatively, such agents can be attached using a heterobifunctional cross-linker, such as N-succinyl 3-(2-pyridyldithio)propionate (SPDP). Yu et al., Int. J. Cancer 56: 244 (1994). General techniques for such conjugation are well-known in the art. See, for example, Wong, CHEMISTRY OF PROTEIN CONJUGATION AND CROSS-LINKING (CRC Press 1991); Upeslacis et al., "Modification of Antibodies by Chemical Methods," in MONOCLONAL ANTIBODIES: PRINCIPLES AND APPLICATIONS, Birch et al. (eds.), pages 187-230 (Wiley-Liss, Inc. 1995); Price, "Production and Characterization of Synthetic Peptide-Derived Antibodies," in MONOCLONAL ANTIBODIES: PRODUCTION, ENGINEERING AND CLINICAL APPLICATION, Ritter et al. (eds.), pages 60-84 (Cambridge University Press 1995). Alternatively, the therapeutic or diagnostic agent can be conjugated via a carbohydrate moiety in the Fc region of the antibody. The carbohydrate group can be used to increase the loading of the same agent that is bound to a thiol group, or the carbohydrate moiety can be used to bind a different therapeutic or diagnostic agent.

Methods for conjugating peptides to antibody components via an antibody carbohydrate moiety are well-known to those of skill in the art. See, for example, Shih et al., Int. J. Cancer 41: 832 (1988); Shih et al., Int. J. Cancer 46: 1101 (1990); and Shih et al., U.S. Patent No. 5,057,313. The general method involves reacting an antibody component having an oxidized carbohydrate portion with a carrier polymer that has at least one free amine function. This reaction results in an initial Schiff base (imine) linkage, which can be stabilized by reduction to a secondary amine to form the final conjugate.

The Fc region may be absent if the antibody used as the antibody component of the immunoconjugate is an antibody fragment. However, it is possible to introduce a carbohydrate moiety into the light chain variable region of a full-length antibody or antibody fragment. See, for example, Leung et al., J. Immunol. 154: 5919 (1995); Hansen et al., U.S. Patent No. 5,443,953 (1995), Leung et al., U.S. patent No. 6,254,868. The engineered carbohydrate moiety is used to attach the therapeutic or diagnostic agent.

In some embodiments of the invention as defined in the claims, a chelating agent may be attached to an antibody, antibody fragment or fusion protein and used to chelate a therapeutic or diagnostic agent, such as a radionuclide. Exemplary chelators include but are not limited to DTPA (such as Mx-DTPA), DOTA, TETA, NETA or NOTA. Methods of conjugation and use of chelating agents to attach metals or other ligands to proteins are well known in the art (see, e.g., U.S. Patent No. 7,563,433).

In certain embodiments of the invention as defined in the claims, radioactive metals or paramagnetic ions may be attached to proteins or peptides by reaction with a reagent having a long tail, to which may be attached a multiplicity of chelating groups for binding ions. Such a tail can be a polymer such as a polylysine, polysaccharide, or other derivatized or derivatizable chains having pendant groups to which can be bound chelating groups such as, *e.g.,* ethylenediaminetetraacetic acid (EDTA), diethylenetriaminepentaacetic acid (DTPA), porphyrins, polyamines, crown ethers, bis-thiosemicarbazones, polyoximes, and like groups known to be useful for this purpose.

Chelates may be directly linked to antibodies or peptides, for example as disclosed in U.S. Patent 4,824,659. Particularly useful metal-chelate combinations include 2-benzyl-DTPA and its monomethyl and cyclohexyl analogs, used with diagnostic isotopes in the general energy range of 60 to 4,000 keV, such as ¹²⁵I, ¹³¹I, ¹²³I, ¹²⁴I, ⁶²Cu, ⁶⁴Cu, ¹⁸F, ¹¹¹In, ⁶⁷Ga, ⁶⁸Ga, ^{99m}Tc, ^{94m}Tc, ¹¹C, ¹³N, ¹⁵O, ⁷⁶Br, for radioimaging. The same chelates, when complexed with non-radioactive metals, such as manganese, iron and gadolinium are useful for MRI. Macrocyclic chelates such as NOTA, DOTA, and TETA are of use with a variety of metals and radiometals, most particularly with radionuclides of gallium, yttrium and copper, respectively. Such metal-chelate complexes can be made very stable by tailoring the ring size to the metal of interest. Other ring-type chelates such as macrocyclic polyethers, which are of interest for stably binding nuclides, such as ²²³Ra for RAIT are encompassed.

More recently, methods of ¹⁸F-labeling of use in PET scanning techniques have been disclosed, for example by reaction of F-18 with a metal or other atom, such as aluminum. The ¹⁸F-Al conjugate may be complexed with chelating groups, such as DOTA, NOTA or NETA that are attached directly to antibodies or used to label targetable constructs in pre-targeting methods. Such F-18 labeling techniques are disclosed in U.S. Patent No. 7,563,433.

In specific preferred embodiments of the invention as defined in the claims, an immunoconjugate may be an antibody-drug conjugate (ADC). Two exemplary drugs of use in ADC production are SN-38 and a prodrug form of 2-pyrrolinodoxorubicine (P2PDox). Compositions and methods of production of SN-38-conjugated ADCs are disclosed, for example, in U.S. Patent Nos. 7,999,083; 8,080,250; 8,741,300; 8,759,496. Compositions and methods of production of P2PDox ADCs are disclosed, for example, in U.S. Patent No. 8,877,101.

### Methods of Producing Bispecific Antibodies

According to the instant disclosure, a combination therapy may utilize one or more bispecific antibodies (bsAbs), such as a leukocyte redirecting bsAb. A bispecific antibody as used herein is an antibody that contains binding sites for two different antigens, or two different epitopes on the same antigen. An antibody that can only bind to a single epitope on a single antigen is monospecific, regardless of the number of antigen-binding sites on the antibody molecule.

Early attempts at bispecific antibody construction either utilized chemical cross-linking or hybrid hybridomas or quadromas to join the two halves of two different antibodies together (e.g., Staerz et al., 1985, Nature 314:628-31; Milstein and Cuello, Nature 1983; 305:537-540; Karpovsky et al., 1984, J Exp Med 160:1686-701). Although the techniques work to make bsAbs, various production problems made use of such complexes difficult, such as the production of mixed populations containing different combinations of antigen-binding sites, difficulty in protein expression, the need to purify the bsAb of interest, low yields, expense of production, etc.

More recent approaches have utilized genetically engineered constructs that are capable of producing homogeneous products of single bsAbs, without the need for extensive purification to remove unwanted byproducts. Such constructs have included tandem scFv, diabodies, tandem diabodies, dual variable domain antibodies and heterodimerization using a motif such as Chl/Ck domain or DNL^{™} (Chames & Baty, 2009, Curr Opin Drug Discov Devel 12:276-83; Chames & Baty, mAbs 1:539-47).

Triomabs is a variation on the quadroma approach that use a combination of mouse IgG2a and rat IgG2b antibodies to preferentially produce the recombinant antibody, compared to the random pairing typically seen in rat/rat or mouse/mouse quadromas (Chames & Baty, mAbs 1:539-47). An anti-CD3 × anti-EpCAM bsAb (catumaxomab) created by this technique was able to efficiently recruit macrophages and NK cells and to activate T cells (Chames & Baty, mAbs 1:539-47). As discussed above, catumaxomab has been approved in Europe for treatment of malignant ascites in patients with EpCAM positive carcinomas (Chames & Baty, mAbs 1:539-47). Surprisingly, the recombinant bsAb was reported to induce only moderate anti-mouse and anti-rat responses in humans (Chames & Baty, mAbs 1:539-47), probably due at least in part to the i.p. route of administration for ascites. Ertumaxomab is another triomab targeting HER2, which may be of use for metastatic breast cancer. Bi20 is another triomab that targets CD20. In vitro, Bi20 exibited efficient lyis of B cells from CLL patients (Chames & Baty, mAbs 1:539-47).

BITE^{®} refers to tandem scFvs that are joined by a short peptide linker (Chames & Baty, mAbs 1:539-47). Blinatumomab is an anti-CD19 × anti-CD3 BITE^{®} with reported efficacy in hematologic cancers, such as non-Hodgkin's lymphoma and ALL, at very low concentrations (Nagorsen et al., 2009, Leukemia & Lymphoma 50:886-91; Chames & Baty, mAbs 1:539-47; Topp et al., 2012, Blood 120:5185-87; Bargou et al., 2008, Science 321:974-77). Another BITE^{®} with specificity for EpCAM has been used in gastrointestinal, ovarian, colorectal and lung cancer (Amann et al., 2009, J Immunother 32:452-64; Chames & Baty, mAbs 1:539-47). Another BITE^{®} (MEDI-565) targeted to CEACAM5 has been proposed for use in melanoma, colorectal, lung, pancreatic, stomach, ovarian, uterine, and breast cancers (Sanders et al., 1994, J Pathol 172:343-8). BITE^{®} has been reported to exhibit anti-tumor activity at picomolar or even femtomolar concentrations (Chames & Baty, mAbs 1:539-47).

Another method of bsAb formation, involving assembly of two heavy and two light chains derived from two different pre-existing antibodies, is based on a knobs-into-holes approach that facilitates heterodimer formation and prevents homodimer formation (Schaefer et al., 2011, Proc Natl. Acad Sci USA 108:11187-92). The "CrossMab" technique further involves the exchange of heavy and light chain domains within the Fab of one half of the bispecific antibody, making the two arms so different that light-heavy chain mispairing can not occur (Schaefer et al., 2011). The knobs-into-holes approach introduces amino acids with bulky side chains into the CH3 domain of one heavy chain that fit into appropriately designed cavities in the CH3 domain of the other heavy chain. The combination of approaches prevents mis-match of both heavy chain to heavy chain and heavy chain to light chain interactions, resulting in primarily a single product. The initial CrossMab, generated against angiopoietin-2 (Ang-2) and VEGF-A, exhibited binding characteristics comparable to the parent mAbs, with potent anti-angiogenic and anti-tumoral activity (Schaefer et al., 2011, Proc Natl. Acad Sci USA 108:11187-92; Kienast et al., Clin Cancer Res, Oct. 25, 2013, Epub ahead of print).

In addition to the DART^{™} technology discussed above, other approaches to bsAb production have included tetravalent IgG-scFv fusions (Dong e tal., 2011, MAbs 3:273-88); dual-acting Fab (DAF) antibodies (Bostrom et al., 2009, Science 323:1610-14); Igg-like dual-variable domain antibodies (DVD-Ig) (Wu et al., 2007, Nat Biotechnol 25:1290-97); and use of dynamic exchange between IgG4 molecules (van der Neut Kolfschoten et al., 2007, Science 317:1554-57). Although the DNL^{™} technology discussed below is preferred for formation of leukocyte redirecting bsAbs, the person of ordinary skill will realize that other types of bsAbs may be used in the claimed methods and compositions.

### DOCK-AND-LOCK^{™} (DNL^{™})

In some examples of the instant disclosure, a bispecific antibody, either alone or else complexed to one or more effectors such as cytokines, is formed as a DOCK-AND-LOCK^{™} (DNL^{™}) complex (see, e.g., U.S. Patent Nos. 7,521,056; 7,527,787; 7,534,866; 7,550,143; 7,666,400; 7,901,680; 7,906,118; 7,981,398; 8,003,111.) Generally, the technique takes advantage of the specific and high-affinity binding interactions that occur between a dimerization and docking domain (DDD) sequence of the regulatory (R) subunits of cAMP-dependent protein kinase (PKA) and an anchor domain (AD) sequence derived from any of a variety of AKAP proteins (Baillie et al., FEBS Letters. 2005; 579: 3264. Wong and Scott, Nat. Rev. Mol. Cell Biol. 2004; 5: 959). The DDD and AD peptides may be attached to any protein, peptide or other molecule. Because the DDD sequences spontaneously dimerize and bind to the AD sequence, the technique allows the formation of complexes between any selected molecules that may be attached to DDD or AD sequences.

Although the standard DNL^{™} complex comprises a trimer with two DDD-linked molecules attached to one AD-linked molecule, variations in complex structure allow the formation of dimers, trimers, tetramers, pentamers, hexamers and other multimers. In some embodiments, the DNL^{™} complex may comprise two or more antibodies, antibody fragments or fusion proteins which bind to the same antigenic determinant or to two or more different antigens. The DNL^{™} complex may also comprise one or more other effectors, such as proteins, peptides, immunomodulators, cytokines, interleukins, interferons, binding proteins, peptide ligands, carrier proteins, toxins, ribonucleases such as onconase, inhibitory oligonucleotides such as siRNA, antigens or xenoantigens, polymers such as PEG, enzymes, therapeutic agents, hormones, cytotoxic agents, anti-angiogenic agents, pro-apoptotic agents or any other molecule or aggregate.

PKA, which plays a central role in one of the best studied signal transduction pathways triggered by the binding of the second messenger cAMP to the R subunits, was first isolated from rabbit skeletal muscle in 1968 (Walsh et al., J. Biol. Chem. 1968;243:3763). The structure of the holoenzyme consists of two catalytic subunits held in an inactive form by the R subunits (Taylor, J. Biol. Chem. 1989;264:8443). Isozymes of PKA are found with two types of R subunits (RI and RII), and each type has a and β isoforms (Scott, Pharmacol. Ther. 1991;50:123). Thus, the four isoforms of PKA regulatory subunits are RIα, RIβ, RIIα and RIIβ, each of which comprises a DDD moiety amino acid sequence. The R subunits have been isolated only as stable dimers and the dimerization domain has been shown to consist of the first 44 amino-terminal residues of RIIα (Newlon et al., Nat. Struct. Biol. 1999; 6:222). As discussed below, similar portions of the amino acid sequences of other regulatory subunits are involved in dimerization and docking, each located near the N-terminal end of the regulatory subunit. Binding of cAMP to the R subunits leads to the release of active catalytic subunits for a broad spectrum of serine/threonine kinase activities, which are oriented toward selected substrates through the compartmentalization of PKA via its docking with AKAPs (Scott et al., J. Biol. Chem. 1990;265;21561)

Since the first AKAP, microtubule-associated protein-2, was characterized in 1984 (Lohmann et al., Proc. Natl. Acad. Sci USA 1984; 81:6723), more than 50 AKAPs that localize to various sub-cellular sites, including plasma membrane, actin cytoskeleton, nucleus, mitochondria, and endoplasmic reticulum, have been identified with diverse structures in species ranging from yeast to humans (Wong and Scott, Nat. Rev. Mol. Cell Biol. 2004;5:959). The AD of AKAPs for PKA is an amphipathic helix of 14-18 residues (Carr et al., J. Biol. Chem. 1991;266: 14188). The amino acid sequences of the AD are varied among individual AKAPs, with the binding affinities reported for RII dimers ranging from 2 to 90 nM (Alto et al., Proc. Natl. Acad. Sci. USA 2003;100:4445). AKAPs will only bind to dimeric R subunits. For human RIIα, the AD binds to a hydrophobic surface formed by the 23 amino-terminal residues (Colledge and Scott, Trends Cell Biol. 1999; 6:216). Thus, the dimerization domain and AKAP binding domain of human RIIα are both located within the same N-terminal 44 amino acid sequence (Newlon et al., Nat. Struct. Biol. 1999;6:222; Newlon et al., EMBO J. 2001;20:1651), which is termed the DDD herein.

We have developed a platform technology to utilize the DDD of human PKA regulatory subunits and the AD of AKAP as an excellent pair of linker modules for docking any two entities, referred to hereafter as **A** and **B**, into a noncovalent complex, which could be further locked into a DNL^{™} complex through the introduction of cysteine residues into both the DDD and AD at strategic positions to facilitate the formation of disulfide bonds. The general methodology of the approach is as follows. Entity **A** is constructed by linking a DDD sequence to a precursor of **A,** resulting in a first component hereafter referred to as **a**. Because the DDD sequence would effect the spontaneous formation of a dimer, **A** would thus be composed of **a₂**. Entity **B** is constructed by linking an AD sequence to a precursor of **B,** resulting in a second component hereafter referred to as **b**. The dimeric motif of DDD contained in **a₂** will create a docking site for binding to the AD sequence contained in b, thus facilitating a ready association of **a₂** and **b** to form a binary, trimeric complex composed of **a₂b**. This binding event is stabilized with a subsequent reaction to covalently secure the two entities via disulfide bridges, which occurs very efficiently based on the principle of effective local concentration because the initial binding interactions should bring the reactive thiol groups placed onto both the DDD and AD into proximity (Chmura et al., Proc. Natl. Acad. Sci. USA 2001;98:8480) to ligate site-specifically. Using various combinations of linkers, adaptor modules and precursors, a wide variety of DNL^{™} constructs of different stoichiometry may be produced and used (see, e.g., U.S. Nos. 7,550,143; 7,521,056; 7,534,866; 7,527,787 and 7,666,400.)

By attaching the DDD and AD away from the functional groups of the two precursors, such site-specific ligations are also expected to preserve the original activities of the two precursors. This approach is modular in nature and potentially can be applied to link, site-specifically and covalently, a wide range of substances, including peptides, proteins, antibodies, antibody fragments, and other effector moieties with a wide range of activities. Utilizing the fusion protein method of constructing AD and DDD conjugated effectors described in the Examples below, virtually any protein or peptide may be incorporated into a DNL^{™} construct. However, the technique is not limiting and other methods of conjugation may be utilized.

A variety of methods are known for making fusion proteins, including nucleic acid synthesis, hybridization and/or amplification to produce a synthetic double-stranded nucleic acid encoding a fusion protein of interest. Such double-stranded nucleic acids may be inserted into expression vectors for fusion protein production by standard molecular biology techniques (see, e.g. Sambrook et al., MOLECULAR CLONING, A LABORATORY MANUAL, 2nd Ed, 1989). In such preferred embodiments, the AD and/or DDD moiety may be attached to either the N-terminal or C-terminal end of an effector protein or peptide. However, the skilled artisan will realize that the site of attachment of an AD or DDD moiety to an effector moiety may vary, depending on the chemical nature of the effector moiety and the part(s) of the effector moiety involved in its physiological activity. Site-specific attachment of a variety of effector moieties may be performed using techniques known in the art, such as the use of bivalent cross-linking reagents and/or other chemical conjugation techniques.

Dock-and-Lock^{™} (DNL^{™}) technology has been used to produce a variety of complexes in assorted formats (Rossi et al., 2012, Bioconjug Chem 23:309-23). Bispecific hexavalent antibodies (bsHexAbs) based on veltuzumab (anti-CD20) and epratuzumab (anti-CD22) were constructed by combining a stabilized (Fab)₂ fused to a dimerization and docking domain (DDD) with an IgG containing an anchor domain (AD) appended at the C-terminus of each heavy chain (C_{H}3-AD2-IgG) (Rossi et al., 2009, Blood 113, 6161-71). Compared to mixtures of their parental mAbs, these Fc-based bsHexAbs, referred to henceforth as "Fc-bsHexAbs", induced unique signaling events (Gupta et al., 2010, Blood 116:3258-67), and exhibited potent cytotoxicity *in vitro.* However, the Fc-bsHexAbs were cleared from circulation of mice approximately twice as fast as the parental mAbs (Rossi et al., 2009, Blood 113, 6161-71). Although the Fc-bsHexAbs are highly stable *ex vivo,* it is possible that some dissociation occurs *in vivo,* for example by intracellular processing. Further, the Fc-bsHexAbs lack CDC activity.

Fc-based immunocytokines have also been assembled as DNL^{™} complexes, comprising two or four molecules of interferon-alpha 2b (IFNα2b) fused to the C-terminal end of the C_{H}3-AD2-IgG Fc (Rossi et al., 2009, Blood 114:3864-71; Rossi et al., 2010, Cancer Res 70:7600-09; Rossi et al., 2011, Blood 118:1877-84). The Fc-IgG-IFNα maintained high specific activity, approaching that of recombinant IFNα, and were remarkably potent *in vitro* and *in vivo* against non-Hodgkin lymphoma (NHL) xenografts. The T_{1/2} of the Fc-IgG-IFNα in mice was longer than PEGylated IFNα, but half as long as the parental mAbs. Similar to the Fc-bsHexAbs, the Fc-IgG-IFNα dissociated *in vivo* over time and exhibited diminished CDC, but ADCC was enhanced.

### Structure-Function Relationships in AD and DDD Moieties

For different types of DNL^{™} constructs, different AD or DDD sequences may be utilized. Exemplary DDD and AD sequences are provided below.
DDD1
   SHIQIPPGLTELLQGYTVEVLRQQPPDLVEFAVEYFTRLREARA (SEQ ID NO: 1)
DDD2
   CGHIQIPPGLTELLQGYTVEVLRQQPPDLVEFAVEYFTRLREARA (SEQ ID NO:2)
AD1
   QIEYLAKQIVDNAIQQA (SEQ ID NO: 3)
AD2
   CGQIEYLAKQIVDNAIQQAGC (SEQ ID NO:4)

The skilled artisan will realize that DDD1 and DDD2 are based on the DDD sequence of the human RIIα isoform of protein kinase A. However, in alternative embodiments, the DDD and AD moieties may be based on the DDD sequence of the human RIα form of protein kinase A and a corresponding AKAP sequence, as exemplified in DDD3, DDD3C and AD3 below.
DDD3
   SLRECELYVQKHNIQALLKDSIVQLCTARPERPMAFLREYFERLEKEEAK (SEQ ID NO:5)
DDD3C
   MSCGGSLRECELYVQKHNIQALLKDSIVQLCTARPERPMAFLREYFERLEKEEAK (SEQ ID NO:6)
AD3
   CGFEELAWKIAKMIWSDVFQQGC (SEQ ID NO:7)

Alternatively, other sequence variants of AD and/or DDD moieties may be utilized in construction of the DNL^{™} complexes. For example, there are only four variants of human PKA DDD sequences, corresponding to the DDD moieties of PKA RIα, RIIα, RIβ and RIIβ. The RIIα DDD sequence is the basis of DDD1 and DDD2 disclosed above. The four human PKA DDD sequences are shown below. The DDD sequence represents residues 1-44 of RIIα, 1-44 of RIIβ, 12-61 of RIα and 13-66 of RIβ. (Note that the sequence of DDD1 is modified slightly from the human PKA RIIα DDD moiety.)
PKA RIα
   SLRECELYVQKHNIQALLKDVSIVQLCTARPERPMAFLREYFEKLEKEEAK (SEQ ID NO:8)
PKA RIβ
   SLKGCELYVQLHGIQQVLKDCIVHLCISKPERPMKFLREHFEKLEKEENRQILA (SEQ ID NO:9)
PKA RIIα
   SHIQIPPGLTELLQGYTVEVGQQPPDLVDFAVEYFTRLREARRQ (SEQ ID NO:10)
PKA RIIβ
   SIEIPAGLTELLQGFTVEVLRHQPADLLEFALQHFTRLQQENER (SEQ ID NO:11)

The structure-function relationships of the AD and DDD domains have been the subject of investigation. (See, e.g., Burns-Hamuro et al., 2005, Protein Sci 14:2982-92; Carr et al., 2001, J Biol Chem 276:17332-38; Alto et al., 2003, Proc Natl Acad Sci USA 100:4445-50; Hundsrucker et al., 2006, Biochem J 396:297-306; Stokka et al., 2006, Biochem J 400:493-99; Gold et al., 2006, Mol Cell 24:383-95; Kinderman et al., 2006, Mol Cell 24:397-408.)

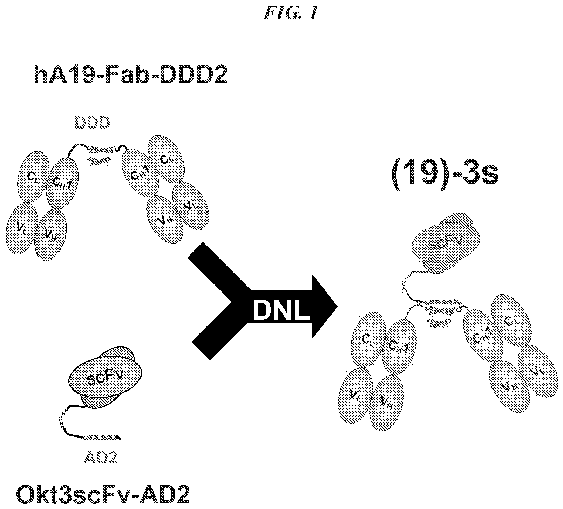

For example, Kinderman et al. (2006, Mol Cell 24:397-408) examined the crystal structure of the AD-DDD binding interaction and concluded that the human DDD sequence contained a number of conserved amino acid residues that were important in either dimer formation or AKAP binding, underlined in SEQ ID NO:1 below. (See Figure 1 of Kinderman et al., 2006.) The skilled artisan will realize that in designing sequence variants of the DDD sequence, one would desirably avoid changing any of the underlined residues, while conservative amino acid substitutions might be made for residues that are less critical for dimerization and AKAP binding.
SHIQIPPGLTELLQGYTVEVLRQQPPDLVEFAVEYFTRLREARA (SEQ ID NO:1)

As discussed in more detail below, conservative amino acid substitutions have been characterized for each of the twenty common L-amino acids. Thus, based on the data of Kinderman (2006) and conservative amino acid substitutions, potential alternative DDD sequences based on SEQ ID NO:1 are shown in **Table 2**. In devising **Table 2**, only highly conservative amino acid substitutions were considered. For example, charged residues were only substituted for residues of the same charge, residues with small side chains were substituted with residues of similar size, hydroxyl side chains were only substituted with other hydroxyls, etc. Because of the unique effect of proline on amino acid secondary structure, no other residues were substituted for proline. A limited number of such potential alternative DDD moiety sequences are shown in SEQ ID NO:12 to SEQ ID NO:31 below. The skilled artisan will realize that alternative species within the genus of DDD moieties can be constructed by standard techniques, for example using a commercial peptide synthesizer or well-known site-directed mutagenesis techniques. The effect of the amino acid substitutions on AD moiety binding may also be readily determined by standard binding assays, for example as disclosed in Alto et al. (2003, Proc Natl Acad Set USA 100:4445-50).

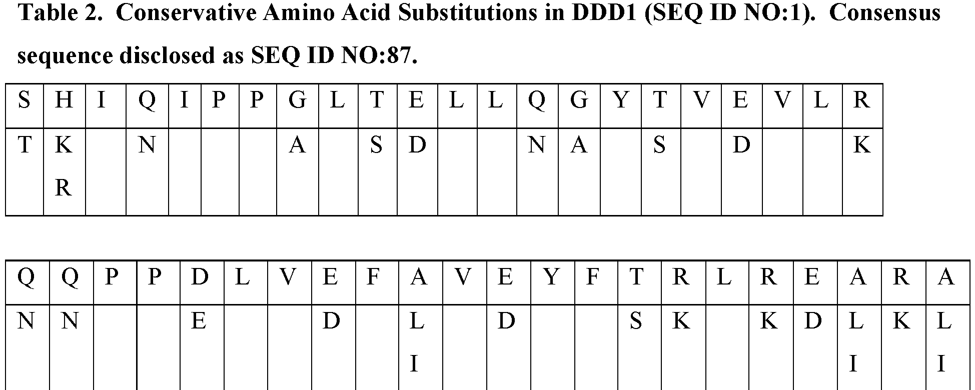

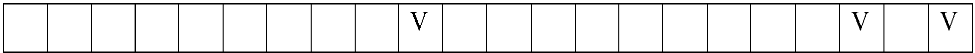

THIQIPPGLTELLQGYTVEVLRQQPPDLVEFAVEYFTRLREARA (SEQ ID NO:12)
SKIQIPPGLTELLQGYTVEVLRQQPPDLVEFAVEYFTRLREARA (SEQ ID NO:13)
SRIQIPPGLTELLQGYTVEVLRQQPPDLVEFAVEYFTRLREARA (SEQ ID NO:14)
SHINIPPGLTELLQGYTVEVLRQQPPDLVEFAVEYFTRLREARA (SEQ ID NO:15)
SHIQIPPALTELLQGYTVEVLRQQPPDLVEFAVEYFTRLREARA (SEQ ID NO:16)
SHIQIPPGLSELLQGYTVEVLRQQPPDLVEFAVEYFTRLREARA (SEQ ID NO:17)
SHIQIPPGLTDLLQGYTVEVLRQQPPDLVEFAVEYFTRLREARA (SEQ ID NO:18)
SHIQIPPGLTELLNGYTVEVLRQQPPDLVEFAVEYFTRLREARA (SEQ ID NO:19)
SHIQIPPGLTELLQAYTVEVLRQQPPDLVEFAVEYFTRLREARA (SEQ ID NO:20)
SHIQIPPGLTELLQGYSVEVLRQQPPDLVEFAVEYFTRLREARA (SEQ ID NO:21)
SHIQIPPGLTELLQGYTVDVLRQQPPDLVEFAVEYFTRLREARA (SEQ ID NO:22)
SHIQIPPGLTELLQGYTVEVLKQQPPDLVEFAVEYFTRLREARA (SEQ ID NO:23)
SHIQIPPGLTELLQGYTVEVLRNQPPDLVEFAVEYFTRLREARA (SEQ ID NO:24)
SHIQIPPGLTELLQGYTVEVLRQNPPDLVEFAVEYFTRLREARA (SEQ ID NO:25)
SHIQIPPGLTELLQGYTVEVLRQQPPELVEFAVEYFTRLREARA (SEQ ID NO:26)
SHIQIPPGLTELLQGYTVEVLRQQPPDLVDFAVEYFTRLREARA (SEQ ID NO:27)
SHIQIPPGLTELLQGYTVEVLRQQPPDLVEFLVEYFTRLREARA (SEQ ID NO:28)
SHIQIPPGLTELLQGYTVEVLRQQPPDLVEFIVEYFTRLREARA (SEQ ID NO:29)
SHIQIPPGLTELLQGYTVEVLRQQPPDLVEFVVEYFTRLREARA (SEQ ID NO:30)
SHIQIPPGLTELLQGYTVEVLRQQPPDLVEFAVDYFTRLREARA (SEQ ID NO:31)

Alto et al. (2003, Proc Natl Acad Sci USA 100:4445-50) performed a bioinformatic analysis of the AD sequence of various AKAP proteins to design an RII selective AD sequence called AKAP-IS (SEQ ID NO:3), with a binding constant for DDD of 0.4 nM. The AKAP-IS sequence was designed as a peptide antagonist of AKAP binding to PKA. Residues in the AKAP-IS sequence where substitutions tended to decrease binding to DDD are underlined in SEQ ID NO:3 below. The skilled artisan will realize that in designing sequence variants of the AD sequence, one would desirably avoid changing any of the underlined residues, while conservative amino acid substitutions might be made for residues that are less critical for DDD binding. **Table 3** shows potential conservative amino acid substitutions in the sequence of AKAP-IS (AD1, SEQ ID NO:3), similar to that shown for DDD1 (SEQ ID NO: 1) in **Table 2** above.

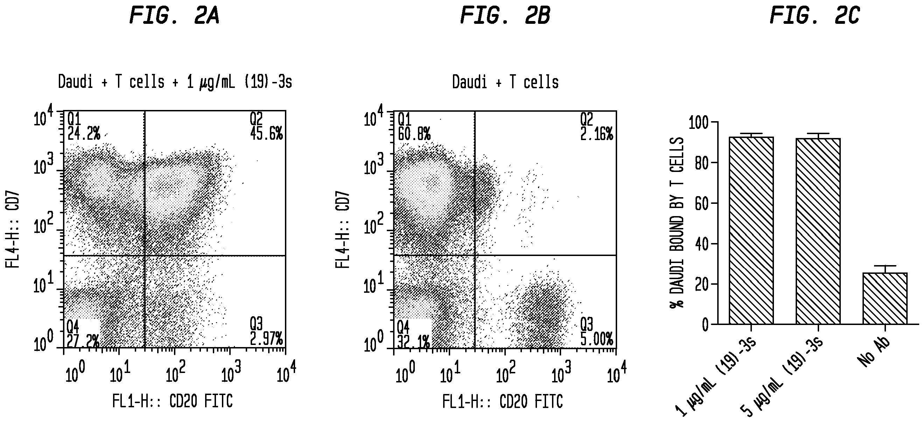

A limited number of such potential alternative AD moiety sequences are shown in SEQ ID NO:32 to SEQ ID NO:49 below. Other species within the genus of possible AD moiety sequences could be made, tested and used by the skilled artisan, based on the data of Alto et al. (2003). It is noted that Figure 2 of Alto (2003) shows a number of amino acid substitutions that may be made, while retaining binding activity to DDD moieties, based on actual binding experiments.
AKAP-IS
QIEYLAKQIVDNAIQQA (SEQ ID NO:3)

**Table 3. Conservative Amino Acid Substitutions in AD1 (SEQ ID NO:3). Consensus sequence disclosed as SEQ ID NO:88.**

| Q | I | E | Y | L | A | K | Q | I | V | D | N | A | I | Q | Q | A |
|---|---|---|---|---|---|---|---|---|---|---|---|---|---|---|---|---|
| N | L V | D | F T S | I V | | R | N | | | E | Q | | | N | N | L I V |

NIEYLAKQIVDNAIQQA (SEQ ID NO:32)
QLEYLAKQIVDNAIQQA (SEQ ID NO:33)
QVEYLAKQIVDNAIQQA (SEQ ID NO:34)
QIDYLAKQIVDNAIQQA (SEQ ID NO:35)
QIEFLAKQIVDNAIQQA (SEQ ID NO:36)
QIETLAKQIVDNAIQQA (SEQ ID NO:37)
QIESLAKQIVDNAIQQA (SEQ ID NO:38)
QIEYIAKQIVDNAIQQA (SEQ ID NO:39)
QIEYVAKQIVDNAIQQA (SEQ ID NO:40)
QIEYLARQIVDNAIQQA (SEQ ID NO:41)
QIEYLAKNIVDNAIQQA (SEQ ID NO:42)
QIEYLAKQIVENAIQQA (SEQ ID NO:43)
QIEYLAKQIVDQAIQQA (SEQ ID NO:44)
QIEYLAKQIVDNAINQA (SEQ ID NO:45)
QIEYLAKQIVDNAIQNA (SEQ ID NO:46)
QIEYLAKQIVDNAIQQL (SEQ ID NO:47)
QIEYLAKQIVDNAIQQI (SEQ ID NO:48)
QIEYLAKQIVDNAIQQV (SEQ ID NO:49)

Gold et al. (2006, Mol Cell 24:383-95) utilized crystallography and peptide screening to develop a SuperAKAP-IS sequence (SEQ ID NO:50), exhibiting a five order of magnitude higher selectivity for the RII isoform of PKA compared with the RI isoform. Underlined residues indicate the positions of amino acid substitutions, relative to the AKAP-IS sequence, which increased binding to the DDD moiety of RIIα. In this sequence, the N-terminal Q residue is numbered as residue number 4 and the C-terminal A residue is residue number 20. Residues where substitutions could be made to affect the affinity for RIIα were residues 8, 11, 15, 16, 18, 19 and 20 (Gold et al., 2006). It is contemplated that in certain alternative embodiments, the SuperAKAP-IS sequence may be substituted for the AKAP-IS AD moiety sequence to prepare DNL^{™} constructs. Other alternative sequences that might be substituted for the AKAP-IS AD sequence are shown in SEQ ID NO:51-53. Substitutions relative to the AKAP-IS sequence are underlined. It is anticipated that, as with the AD2 sequence shown in SEQ ID NO:4, the AD moiety may also include the additional N-terminal residues cysteine and glycine and C-terminal residues glycine and cysteine.
SuperAKAP-IS
   QIEYVAKQIVDYAIHQA (SEQ ID NO:50)
Alternative AKAP sequences
   QIEYKAKQIVDHAIHQA (SEQ ID NO:51)
   QIEYHAKQIVDHAIHQA (SEQ ID NO:52)
   QIEYVAKQIVDHAIHQA (SEQ ID NO:53)

Figure 2 of Gold et al. disclosed additional DDD-binding sequences from a variety of AKAP proteins, shown below.

### RII-Specific AKAPs

AKAP-KL
   PLEYQAGLLVQNAIQQAI (SEQ ID NO:54)
AKAP79
   LLIETASSLVKNAIQLSI (SEQ ID NO:55)
AKAP-Lbc
   LIEEAASRIVDAVIEQVK (SEQ ID NO:56)

### RI-Specific AKAPs

AKAPce
   alyqfadrfselviseal (SEQ ID NO:57)
riad
   leqvanqladqiikeat (SEQ ID NO:58)
pv38
   feelawkiakmiwsdvf (SEQ ID NO:59)

### Dual-Specificity AKAPs

akap7
   elvrlskrlvenavlkav (SEQ ID NO:60)
map2d
   taeevsarivqvvtaeav (SEQ ID NO:61)
dakap1
   qikqaafqlisqvileat (SEQ ID NO:62)
Dakap2
   lawkiakmivsdvmqq (SEQ ID NO:63)

Stokka et al. (2006, Biochem J 400:493-99) also developed peptide competitors of AKAP binding to PKA, shown in SEQ ID NO:64-66. The peptide antagonists were designated as Ht31 (SEQ ID NO:64), RIAD (SEQ ID NO:65) and PV-38 (SEQ ID NO:66). The Ht-31 peptide exhibited a greater affinity for the RII isoform of PKA, while the RIAD and PV-38 showed higher affinity for RI.
Ht31
   DLIEEAASRIVDAVIEQVKAAGAY (SEQ ID NO:64)
RIAD
   LEQYANQLADQIIKEATE (SEQ ID NO:65)
PV-38
   FEELAWKIAKMIWSDVFQQC (SEQ ID NO:66)

Hundsrucker et al. (2006, Biochem J 396:297-306) developed still other peptide competitors for AKAP binding to PKA, with a binding constant as low as 0.4 nM to the DDD of the RII form of PKA. The sequences of various AKAP antagonistic peptides are provided in Table 1 of Hundsrucker et al., reproduced in **Table 4** below. AKAPIS represents a synthetic RII subunit-binding peptide. All other peptides are derived from the RII-binding domains of the indicated AKAPs.

**Table 4. AKAP Peptide sequences**

| | Peptide Sequence |
|---|---|
| AKAPIS | QIEYLAKQIVDNAIQQA (SEQ ID NO:3) |
| AKAPIS-P | QIEYLAKQIPDNAIQQA (SEQ ID NO:67) |
| Ht31 | KGADLIEEAASRIVDAVIEQVKAAG (SEQ ID NO:68) |
| Ht31-P | KGADLIEEAASRIPDAPIEQVKAAG (SEQ ID NO:69) |
| AKAP7*δ*-wt-pep | PEDAELVRLSKRLVENAVLKAVQQY (SEQ ID NO:70) |
| AKAP7*δ*-L304T-pep | PEDAELVRTSKRLVENAVLKAVQQY (SEQ ID NO:71) |
| AKAP7*δ*-L308D-pep | PEDAELVRLSKRDVENAVLKAVQQY (SEQ ID NO:72) |
| AKAP7*δ*-P-pep | PEDAELVRLSKRLPENAVLKAVQQY (SEQ ID NO:73) |
| AKAP7*δ*-PP-pep | PEDAELVRLSKRLPENAPLKAVQQY (SEQ ID NO:74) |
| AKAP7*δ*-L314E-pep | PEDAELVRLSKRLVENAVEKAVQQY (SEQ ID NO:75) |
| AKAP1-pep | EEGLDRNEEIKRAAFQIISQVISEA (SEQ ID NO:76) |
| AKAP2-pep | LVDDPLEYQAGLLVQNAIQQAIAEQ (SEQ ID NO:77) |
| AKAP5-pep | QYETLLIETASSLVKNAIQLSIEQL (SEQ ID NO:78) |
| AKAP9-pep | LEKQYQEQLEEEVAKVIVSMSIAFA (SEQ ID NO:79) |
| AKAP10-pep | NTDEAQEELAWKIAKMIVSDIMQQA (SEQ ID NO:80) |
| AKAP11-pep | VNLDKKAVLAEKIVAEAIEKAEREL (SEQ ID NO:81) |
| AKAP12-pep | NGILELETKSSKLVQNIIQTAVDQF (SEQ ID NO:82) |
| AKAP14-pep | TQDKNYEDELTQVALALVEDVINYA (SEQ ID NO:83) |
| Rab32-pep | ETSAKDNINIEEAARFLVEKILVNH (SEQ ID NO:84) |

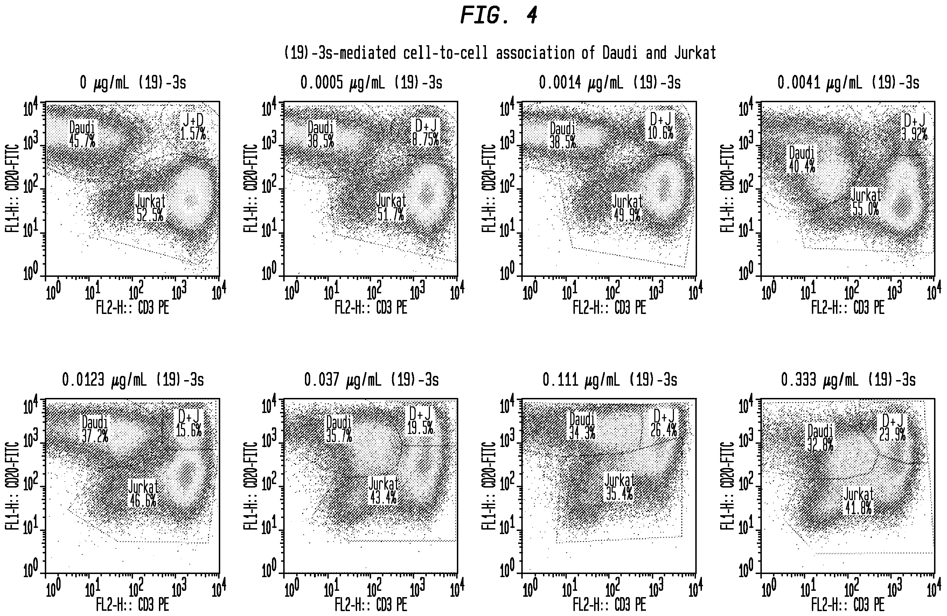

Residues that were highly conserved among the AD domains of different AKAP proteins are indicated below by underlining with reference to the AKAP IS sequence (SEQ ID NO:3). The residues are the same as observed by Alto et al. (2003), with the addition of the C-terminal alanine residue. (See FIG. 4 of Hundsrucker et al. (2006).) The sequences of peptide antagonists with particularly high affinities for the RII DDD sequence were those of AKAP-IS, AKAP76-wt-pep, AKAP7δ-L304T-pep and AKAP7δ-L308D-pep.
AKAP-IS
QIEYLAKQIVDNAIQQA (SEQ ID NO:3)

Carr et al. (2001, J Biol Chem 276:17332-38) examined the degree of sequence homology between different AKAP-binding DDD sequences from human and non-human proteins and identified residues in the DDD sequences that appeared to be the most highly conserved among different DDD moieties. These are indicated below by underlining with reference to the human PKA RIIα DDD sequence of SEQ ID NO:1. Residues that were particularly conserved are further indicated by italics. The residues overlap with, but are not identical to those suggested by Kinderman et al. (2006) to be important for binding to AKAP proteins. The skilled artisan will realize that in designing sequence variants of DDD, it would be most preferred to avoid changing the most conserved residues (italicized), and it would be preferred to also avoid changing the conserved residues (underlined), while conservative amino acid substitutions may be considered for residues that are neither underlined nor italicized..
SHIQ*IP*P*GL*TE*LL*Q*G*Y*T*V*EVLR*Q*QP*P*DL*VE*FA*VE*YF*TR*L*REA*R*A (SEQ ID NO: 1)

A modified set of conservative amino acid substitutions for the DDD1 (SEQ ID NO: 1) sequence, based on the data of Carr et al. (2001) is shown in **Table 5**. Even with this reduced set of substituted sequences, there are over 65,000 possible alternative DDD moiety sequences that may be produced, tested and used by the skilled artisan without undue experimentation. The skilled artisan could readily derive such alternative DDD amino acid sequences as disclosed above for **Table 2** and **Table 3.**

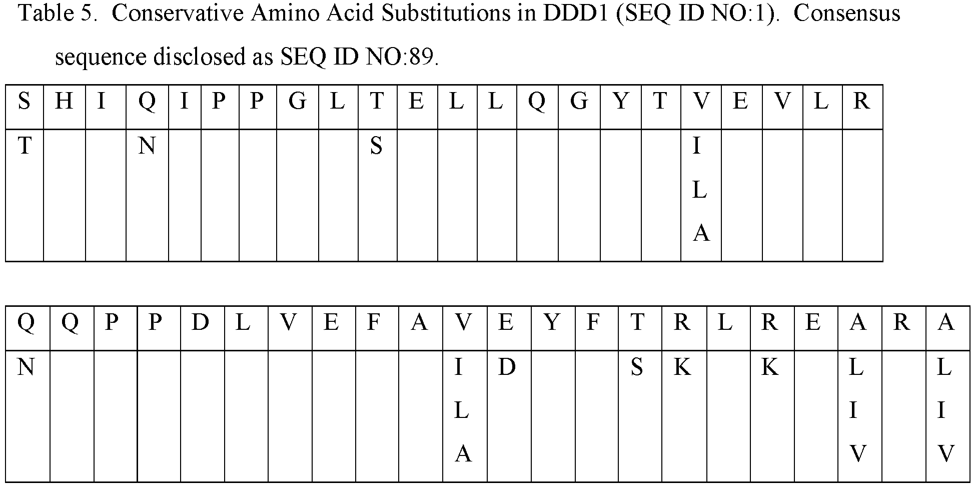

The skilled artisan will realize that these and other amino acid substitutions in the DDD or AD amino acid sequences may be utilized to produce alternative species within the genus of AD or DDD moieties, using techniques that are standard in the field and only routine experimentation.

### Amino Acid Substitutions

Alternatively, the disclosed methods and compositions may involve production and use of proteins or peptides with one or more substituted amino acid residues. For example, the DDD and/or AD sequences used to make DNL^{™} constructs may be modified as discussed above.

The skilled artisan will be aware that, in general, amino acid substitutions typically involve the replacement of an amino acid with another amino acid of relatively similar properties (i.e., conservative amino acid substitutions). The properties of the various amino acids and effect of amino acid substitution on protein structure and function have been the subject of extensive study and knowledge in the art.

For example, the hydropathic index of amino acids may be considered (Kyte & Doolittle, 1982, J. Mol. Biol., 157:105-132). The relative hydropathic character of the amino acid contributes to the secondary structure of the resultant protein, which in turn defines the interaction of the protein with other molecules. Each amino acid has been assigned a hydropathic index on the basis of its hydrophobicity and charge characteristics (Kyte & Doolittle, 1982), these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). In making conservative substitutions, the use of amino acids whose hydropathic indices are within ± 2 is preferred, within ± 1 are more preferred, and within ± 0.5 are even more preferred.

Amino acid substitution may also take into account the hydrophilicity of the amino acid residue (e.g., U.S. Pat. No. 4,554,101). Hydrophilicity values have been assigned to amino acid residues: arginine (+3.0); lysine (+3.0); aspartate (+3.0); glutamate (+3.0); serine (+0.3); asparagine (+0.2); glutamine (+0.2); glycine (0); threonine (-0.4); proline (-0.5 .+-.1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). Replacement of amino acids with others of similar hydrophilicity is preferred.

Other considerations include the size of the amino acid side chain. For example, it would generally not be preferred to replace an amino acid with a compact side chain, such as glycine or serine, with an amino acid with a bulky side chain, e.g., tryptophan or tyrosine. The effect of various amino acid residues on protein secondary structure is also a consideration. Through empirical study, the effect of different amino acid residues on the tendency of protein domains to adopt an alpha-helical, beta-sheet or reverse turn secondary structure has been determined and is known in the art (see, e.g., Chou & Fasman, 1974, Biochemistry, 13:222-245; 1978, Ann. Rev. Biochem., 47: 251-276; 1979, Biophys. J., 26:367-384).

Based on such considerations and extensive empirical study, tables of conservative amino acid substitutions have been constructed and are known in the art. For example: arginine and lysine; glutamate and aspartate; serine and threonine; glutamine and asparagine; and valine, leucine and isoleucine. Alternatively: Ala (A) leu, ile, val; Arg (R) gln, asn, lys; Asn (N) his, asp, lys, arg, gln; Asp (D) asn, glu; Cys (C) ala, ser; Gln (Q) glu, asn; Glu (E) gln, asp; Gly (G) ala; His (H) asn, gln, lys, arg; Ile (I) val, met, ala, phe, leu; Leu (L) val, met, ala, phe, ile; Lys (K) gln, asn, arg; Met (M) phe, ile, leu; Phe (F) leu, val, ile, ala, tyr; Pro (P) ala; Ser (S), thr; Thr (T) ser; Trp (W) phe, tyr; Tyr (Y) trp, phe, thr, ser; Val (V) ile, leu, met, phe, ala.

Other considerations for amino acid substitutions include whether or not the residue is located in the interior of a protein or is solvent exposed. For interior residues, conservative substitutions would include: Asp and Asn; Ser and Thr; Ser and Ala; Thr and Ala; Ala and Gly; Ile and Val; Val and Leu; Leu and Ile; Leu and Met; Phe and Tyr; Tyr and Trp. (See, e.g., PROWL website at rockefeller.edu) For solvent exposed residues, conservative substitutions would include: Asp and Asn; Asp and Glu; Glu and Gln; Glu and Ala; Gly and Asn; Ala and Pro; Ala and Gly; Ala and Ser; Ala and Lys; Ser and Thr; Lys and Arg; Val and Leu; Leu and Ile; Ile and Val; Phe and Tyr. (Id.) Various matrices have been constructed to assist in selection of amino acid substitutions, such as the PAM250 scoring matrix, Dayhoff matrix, Grantham matrix, McLachlan matrix, Doolittle matrix, Henikoff matrix, Miyata matrix, Fitch matrix, Jones matrix, Rao matrix, Levin matrix and Risler matrix (*Idem*.)

In determining amino acid substitutions, one may also consider the existence of intermolecular or intramolecular bonds, such as formation of ionic bonds (salt bridges) between positively charged residues (e.g., His, Arg, Lys) and negatively charged residues (e.g., Asp, Glu) or disulfide bonds between nearby cysteine residues.

Methods of substituting any amino acid for any other amino acid in an encoded protein sequence are well known and a matter of routine experimentation for the skilled artisan, for example by the technique of site-directed mutagenesis or by synthesis and assembly of oligonucleotides encoding an amino acid substitution and splicing into an expression vector construct.

### Therapeutic Agents

In embodiments of the invention as defined in the claims, therapeutic agents such as cytotoxic agents, anti-angiogenic agents, pro-apoptotic agents, antibiotics, hormones, hormone antagonists, chemokines, drugs, prodrugs, toxins, enzymes or other agents may be used, either conjugated to the subject bsAbs, ADCs and/or antibodies or separately administered before, simultaneously with, or after the bsAbs, ADCs and/or antibodies. Drugs of use may possess a pharmaceutical property selected from the group consisting of antimitotic, antikinase, alkylating, antimetabolite, antibiotic, alkaloid, anti-angiogenic, pro-apoptotic agents and combinations thereof.

Exemplary drugs of use may include, but are not limited to, 5-fluorouracil, afatinib, aplidin, azaribine, anastrozole, anthracyclines, axitinib, AVL-101, AVL-291, bendamustine, bleomycin, bortezomib, bosutinib, bryostatin-1, busulfan, calicheamycin, camptothecin, carboplatin, 10-hydroxycamptothecin, carmustine, celebrex, chlorambucil, cisplatin (CDDP), Cox-2 inhibitors, irinotecan (CPT-11), SN-38, carboplatin, cladribine, camptothecans, crizotinib, cyclophosphamide, cytarabine, dacarbazine, dasatinib, dinaciclib, docetaxel, dactinomycin, daunorubicin, doxorubicin, 2-pyrrolinodoxorubicine (2P-DOX), cyano-morpholino doxorubicin, doxorubicin glucuronide, epirubicin glucuronide, erlotinib, estramustine, epidophyllotoxin, erlotinib, entinostat, estrogen receptor binding agents, etoposide (VP16), etoposide glucuronide, etoposide phosphate, exemestane, fingolimod, floxuridine (FUdR), 3',5'-O-dioleoyl-FudR (FUdR-dO), fludarabine, flutamide, farnesyl-protein transferase inhibitors, flavopiridol, fostamatinib, ganetespib, GDC-0834, GS-1101, gefitinib, gemcitabine, hydroxyurea, ibrutinib, idarubicin, idelalisib, ifosfamide, imatinib, L-asparaginase, lapatinib, lenolidamide, leucovorin, LFM-A13, lomustine, mechlorethamine, melphalan, mercaptopurine, 6-mercaptopurine, methotrexate, mitoxantrone, mithramycin, mitomycin, mitotane, navelbine, neratinib, nilotinib, nitrosurea, olaparib, plicomycin, procarbazine, paclitaxel, PCI-32765, pentostatin, PSI-341, raloxifene, semustine, sorafenib, streptozocin, SU11248, sunitinib, tamoxifen, temazolomide (an aqueous form of DTIC), transplatinum, thalidomide, thioguanine, thiotepa, teniposide, topotecan, uracil mustard, vatalanib, vinorelbine, vinblastine, vincristine, vinca alkaloids and ZD1839.

Toxins of use may include ricin, abrin, alpha toxin, saporin, ribonuclease (RNase), e.g., onconase, DNase I, *Staphylococcal* enterotoxin-A, pokeweed antiviral protein, gelonin, diphtheria toxin, *Pseudomonas* exotoxin, and *Pseudomonas* endotoxin.

Chemokines of use may include RANTES, MCAF, MIP1-alpha, MIP1-Beta and IP-10.

In certain embodiments, anti-angiogenic agents, such as angiostatin, baculostatin, canstatin, maspin, anti-VEGF antibodies, anti-PlGF peptides and antibodies, anti-vascular growth factor antibodies, anti-Flk-1 antibodies, anti-Flt-1 antibodies and peptides, anti-Kras antibodies, anti-cMET antibodies, anti-MIF (macrophage migration-inhibitory factor) antibodies, laminin peptides, fibronectin peptides, plasminogen activator inhibitors, tissue metalloproteinase inhibitors, interferons, interleukin-12, IP-10, Gro-β, thrombospondin, 2-methoxyoestradiol, proliferin-related protein, carboxiamidotriazole, CM101, Marimastat, pentosan polysulphate, angiopoietin-2, interferon-alpha, herbimycin A, PNU145156E, 16K prolactin fragment, Linomide (roquinimex), thalidomide, pentoxifylline, genistein, TNP-470, endostatin, paclitaxel, accutin, angiostatin, cidofovir, vincristine, bleomycin, AGM-1470, platelet factor 4 or minocycline may be of use.

Immunomodulators of use may be selected from a cytokine, a stem cell growth factor, a lymphotoxin, a hematopoietic factor, a colony stimulating factor (CSF), an interferon (IFN), erythropoietin, thrombopoietin and a combination thereof. Specifically useful are lymphotoxins such as tumor necrosis factor (TNF), hematopoietic factors, such as interleukin (IL), colony stimulating factor, such as granulocyte-colony stimulating factor (G-CSF) or granulocyte macrophage-colony stimulating factor (GM-CSF), interferon, such as interferons-α, -β or -λ, and stem cell growth factor, such as that designated "S1 factor". Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; prostaglandin, fibroblast growth factor; prolactin; placental lactogen, OB protein; tumor necrosis factor-α and - β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF- α and TGF- β; insulin-like growth factor-I and -II; erythropoietin (EPO); osteoinductive factors; interferons such as interferon-α, -β, and -γ; colony stimulating factors (CSFs) such as macrophage-CSF (M-CSF); interleukins (ILs) such as IL-1, IL-1α, IL-2, IL-3, IL-4, IL-5, IL-6, IL-7, IL-8, IL-9, IL-10, IL-11, IL-12; IL-13, IL-14, IL-15, IL-16, IL-17, IL-18, IL-21, IL-25, LIF, kit-ligand or FLT-3, angiostatin, thrombospondin, endostatin, tumor necrosis factor and LT.

Radionuclides of use include, but are not limited to- ¹¹¹In, ¹⁷⁷Lu, ²¹²Bi, ²¹³Bi, ²¹¹At, ⁶²Cu, ⁶⁷Cu, ⁹⁰Y, ¹²⁵I, ¹³¹I, ³²P, ³³P, ⁴⁷Sc, ¹¹¹Ag ⁶⁷Ga, ¹⁴²Pr, ¹⁵³Sm, ¹⁶¹Tb, ¹⁶⁶Dy, ¹⁶⁶Ho, ¹⁸⁶Re, ¹⁸⁸Re, ¹⁸⁹Re, ²¹²Pb, ²²³Ra, ²²⁵Ac, ⁵⁹Fe, ⁷⁵Se, ⁷⁷As, ⁸⁹Sr, ⁹⁹Mo, ¹⁰⁵Rh, ¹⁰⁹Pd, ¹⁴³Pr, ¹⁴⁹Pm, ¹⁶⁹Er, ¹⁹⁴Ir, ¹⁹⁸Au, ¹⁹⁹Au, ²¹¹Pb, and ²²⁷Th. The therapeutic radionuclide preferably has a decay-energy in the range of 20 to 6,000 keV, preferably in the ranges 60 to 200 keV for an Auger emitter, 100-2,500 keV for a beta emitter, and 4,000-6,000 keV for an alpha emitter. Maximum decay energies of useful beta-particle-emitting nuclides are preferably 20-5,000 keV, more preferably 100-4,000 keV, and most preferably 500-2,500 keV. Also preferred are radionuclides that substantially decay with Auger-emitting particles. For example, Co-58, Ga-67, Br-80m, Tc-99m, Rh-103m, Pt-109, In-111, Sb-119, 1-125, Ho-161, Os-189m and Ir-192. Decay energies of useful beta-particle-emitting nuclides are preferably <1,000 keV, more preferably <100 keV, and most preferably <70 keV. Also preferred are radionuclides that substantially decay with generation of alpha-particles. Such radionuclides include, but are not limited to: Dy-152, At-211, Bi-212, Ra-223, Rn-219, Po-215, Bi-211, Ac-225, Fr-221, At-217, Bi-213, Th-227 and Fm-255. Decay energies of useful alpha-particle-emitting radionuclides are preferably 2,000-10,000 keV, more preferably 3,000-8,000 keV, and most preferably 4,000-7,000 keV. Additional potential radioisotopes of use include ¹¹C, ¹³N, ¹⁵O, ⁷⁵Br, ¹⁹⁸Au, ²²⁴Ac, ¹²⁶I, ¹³³I, ⁷⁷Br, ^{113m}In, ⁹⁵Ru, ⁹⁷Ru, ¹⁰³Ru, ¹⁰⁵Ru, ¹⁰⁷Hg, ²⁰³Hg, ^{121m}Te, ^{122m}Te, ^{125m}Te, ¹⁶⁵Tm, ¹⁶⁷Tm, ¹⁶⁸Tm, ¹⁹⁷Pt, ¹⁰⁹Pd, ¹⁰⁵Rh, ¹⁴²Pr, ¹⁴³Pr, ¹⁶¹Tb, ¹⁶⁶Ho, ¹⁹⁹Au, ⁵⁷Co, ⁵⁸Co, ⁵¹Cr, ⁵⁹Fe, ⁷⁵Se, ²⁰¹Tl, ²²⁵Ac, ⁷⁶Br, ¹⁶⁹Yb, and the like. Some useful diagnostic nuclides may include ¹⁸F, ⁵²Fe, ⁶²Cu, ⁶⁴Cu, ⁶⁷Cu, ⁶⁷Ga, ⁶⁸Ga, ⁸⁶Y, ⁸⁹Zr, ⁹⁴Tc, ^{94m}Tc, ^{99m}Tc, or ¹¹¹In.

Therapeutic agents may include a photoactive agent or dye. Fluorescent compositions, such as fluorochrome, and other chromogens, or dyes, such as porphyrins sensitive to visible light, have been used to detect and to treat lesions by directing the suitable light to the lesion. In therapy, this has been termed photoradiation, phototherapy, or photodynamic therapy. See Jori et al. (eds.), PHOTODYNAMIC THERAPY OF TUMORS AND OTHER DISEASES (Libreria Progetto 1985); van den Bergh, Chem. Britain (1986), 22:430. Moreover, monoclonal antibodies have been coupled with photoactivated dyes for achieving phototherapy. See Mew et al., J. Immunol. (1983),130:1473; idem., Cancer Res. (1985), 45:4380; Oseroff et al., Proc. Natl. Acad. Sci. USA (1986), 83:8744; idem., Photochem. Photobiol. (1987), 46:83; Hasan et al., Prog. Clin. Biol. Res. (1989), 288:471; Tatsuta et al., Lasers Surg. Med. (1989), 9:422; Pelegrin et al., Cancer (1991), 67:2529.

Other useful therapeutic agents may comprise oligonucleotides, especially antisense oligonucleotides that preferably are directed against oncogenes and oncogene products, such as bcl-2 or p53. A preferred form of therapeutic oligonucleotide is siRNA. The skilled artisan will realize that any siRNA or interference RNA species may be attached to an antibody or fragment thereof for delivery to a targeted tissue. Many siRNA species against a wide variety of targets are known in the art, and any such known siRNA may be utilized in the claimed methods and compositions.

Known siRNA species of potential use include those specific for IKK-gamma (U.S. Patent 7,022,828); VEGF, Flt-1 and Flk-1/KDR (U.S. Patent 7,148,342); Bcl2 and EGFR (U.S. Patent 7,541,453); CDC20 (U.S. Patent 7,550,572); transducin (beta)-like 3 (U.S. Patent 7,576,196); KRAS (U.S. Patent 7,576,197); carbonic anhydrase II (U.S. Patent 7,579,457); complement component 3 (U.S. Patent 7,582,746); interleukin-1 receptor-associated kinase 4 (IRAK4) (U.S. Patent 7,592,443); survivin (U.S. Patent 7,608,7070); superoxide dismutase 1 (U.S. Patent 7,632,938); MET proto-oncogene (U.S. Patent 7,632,939); amyloid beta precursor protein (APP) (U.S. Patent 7,635,771); IGF-1R (U.S. Patent 7,638,621); ICAM1 (U.S. Patent 7,642,349); complement factor B (U.S. Patent 7,696,344); p53 (7,781,575), and apolipoprotein B (7,795,421).

Additional siRNA species are available from known commercial sources, such as Sigma-Aldrich (St Louis, MO), Invitrogen (Carlsbad, CA), Santa Cruz Biotechnology (Santa Cruz, CA), Ambion (Austin, TX), Dharmacon (Thermo Scientific, Lafayette, CO), Promega (Madison, WI), Mirus Bio (Madison, WI) and Qiagen (Valencia, CA), among many others. Other publicly available sources of siRNA species include the siRNAdb database at the Stockholm Bioinformatics Centre, the MIT/ICBP siRNA Database, the RNAi Consortium shRNA Library at the Broad Institute, and the Probe database at NCBI. For example, there are 30,852 siRNA species in the NCBI Probe database. The skilled artisan will realize that for any gene of interest, either a siRNA species has already been designed, or one may readily be designed using publicly available software tools. Any such siRNA species may be delivered using the subject DNL^{™} complexes.

### Methods of Therapeutic Treatment

In an embodiment of the invention as defined in the claims, the subject is a mammal, including humans, domestic or companion pets, such as dogs and cats, comprising administering to the subject a therapeutically effective amount of a combination of cytotoxic and/or immunomodulatory agents.

The administration of the cytotoxic bsAbs, ADCs and/or checkpoint inhibitor antibodies can be supplemented by administering concurrently or sequentially a therapeutically effective amount of another antibody that binds to or is reactive with another antigen on the surface of the target cell. Preferred additional MAbs comprise at least one humanized, chimeric or human MAb selected from the group consisting of a MAb reactive with CD4, CD5, CD8, CD14, CD15, CD16, CD19, IGF-1R, CD20, CD21, CD22, CD23, CD25, CD30, CD32b, CD33, CD37, CD38, CD40, CD40L, CD45, CD46, CD52, CD54, CD70, CD74, CD79a, CD79b, CD80, CD95, CD126, CD133, CD138, CD154, CEACAM5, CEACAM6, B7, AFP, PSMA, EGP-1, EGP-2, carbonic anhydrase IX, PAM4 antigen, MUC1, MUC2, MUC3, MUC4, MUC5, Ia, MIF, HM1.24, HLA-DR, tenascin, Flt-3, VEGFR, PIGF, ILGF, IL-6, IL-25, tenascin, TRAIL-R1, TRAII,-R2, complement factor C5, oncogene product, or a combination thereof. Various antibodies of use, such as anti-CD19, anti-CD20, and anti-CD22 antibodies, are known to those of skill in the art. See, for example, Ghetie et al., Cancer Res. 48:2610 (1988); Hekman et al., Cancer Immunol. Immunother. 32:364 (1991); Longo, Curr. Opin. Oncol. 8:353 (1996), U.S. Patent Nos. 5,798,554; 6,187,287; 6,306,393; 6,676,924; 7,109,304; 7,151,164; 7,230,084; 7,230,085; 7,238,785; 7,238,786; 7,282,567; 7,300,655; 7,312,318; 7,501,498; 7,612,180; 7,670,804; and U.S. Patent Application Publ. Nos. 20080131363; 20070172920; 20060193865; and 20080138333.

The combination therapy can be further supplemented with the administration, either concurrently or sequentially, of at least one therapeutic agent. For example, "CVB" (1.5 g/m² cyclophosphamide, 200-400 mg/m² etoposide, and 150-200 mg/m² carmustine) is a regimen used to treat non-Hodgkin's lymphoma. Patti et al., Eur. J. Haematol. 51: 18 (1993). Other suitable combination chemotherapeutic regimens are well-known to those of skill in the art. See, for example, Freedman et al., "Non-Hodgkin's Lymphomas," in CANCER MEDICINE, VOLUME 2, 3rd Edition, Holland et al. (eds.), pages 2028-2068 (Lea & Febiger 1993). As an illustration, first generation chemotherapeutic regimens for treatment of intermediate-grade non-Hodgkin's lymphoma (NHL) include C-MOPP (cyclophosphamide, vincristine, procarbazine and prednisone) and CHOP (cyclophosphamide, doxorubicin, vincristine, and prednisone). A useful second generation chemotherapeutic regimen is m-BACOD (methotrexate, bleomycin, doxorubicin, cyclophosphamide, vincristine, dexamethasone and leucovorin), while a suitable third generation regimen is MACOP-B (methotrexate, doxorubicin, cyclophosphamide, vincristine, prednisone, bleomycin and leucovorin). Additional useful drugs include phenyl butyrate, bendamustine, and bryostatin-1.

The combinations of therapeutic agents can be formulated according to known methods to prepare pharmaceutically useful compositions, whereby the bsAb, ADC, interferon and/or checkpoint inhibitor antibody is combined in a mixture with a pharmaceutically suitable excipient. Sterile phosphate-buffered saline is one example of a pharmaceutically suitable excipient. Other suitable excipients are well-known to those in the art. See, for example, Ansel et al., PHARMACEUTICAL DOSAGE FORMS AND DRUG DELIVERY SYSTEMS, 5th Edition (Lea & Febiger 1990), and Gennaro (ed.), REMINGTON'S PHARMACEUTICAL SCIENCES, 18th Edition (Mack Publishing Company 1990), and revised editions thereof.

The subject bsAbs, ADCs, interferons and/or antibodies can be formulated for intravenous administration via, for example, bolus injection or continuous infusion. Preferably, the bsAb, ADC and/or antibody is infused over a period of less than about 4 hours, and more preferably, over a period of less than about 3 hours. For example, the first bolus could be infused within 30 minutes, preferably even 15 min, and the remainder infused over the next 2-3 hrs. Formulations for injection can be presented in unit dosage form, e.g., in ampoules or in multi-dose containers, with an added preservative. The compositions can take such forms as suspensions, solutions or emulsions in oily or aqueous vehicles, and can contain formulatory agents such as suspending, stabilizing and/or dispersing agents. Alternatively, the active ingredient can be in powder form for constitution with a suitable vehicle, e.g., sterile pyrogen-free water, before use.

Additional pharmaceutical methods may be employed to control the duration of action of the therapeutic combinations. Control release preparations can be prepared through the use of polymers to complex or adsorb the agents to be administered. For example, biocompatible polymers include matrices of poly(ethylene-co-vinyl acetate) and matrices of a polyanhydride copolymer of a stearic acid dimer and sebacic acid. Sherwood et al., Bio/Technology 10: 1446 (1992). The rate of release from such a matrix depends upon the molecular weight of the therapeutic agent, the amount of agent within the matrix, and the size of dispersed particles. Saltzman et al., Biophys. J. 55: 163 (1989); Sherwood *et al., supra.* Other solid dosage forms are described in Ansel et al., PHARMACEUTICAL DOSAGE FORMS AND DRUG DELIVERY SYSTEMS, 5th Edition (Lea & Febiger 1990), and Gennaro (ed.), REMINGTON'S PHARMACEUTICAL SCIENCES, 18th Edition (Mack Publishing Company 1990), and revised editions thereof.

The bsAbs, interferons and/or checkpoint inhibitor antibodies may be administered to a mammal subcutaneously or even by other parenteral routes, such as intravenously, intramuscularly, intraperitoneally or intravascularly. ADCs may be administered intravenously, intraperitoneally or intravascularly. Moreover, the administration may be by continuous infusion or by single or multiple boluses. Preferably, the bsAb, ADC, interferon and/or checkpoint inhibitor antibody is infused over a period of less than about 4 hours, and more preferably, over a period of less than about 3 hours.

More generally, the dosage of an administered bsAb, ADC, interferon and/or checkpoint inhibitor antibody for humans will vary depending upon such factors as the patient's age, weight, height, sex, general medical condition and previous medical history. It may be desirable to provide the recipient with a dosage of bsAb, ADC and/or antibody that is in the range of from about 1 mg/kg to 25 mg/kg as a single intravenous infusion, although a lower or higher dosage also may be administered as circumstances dictate. A dosage of 1-20 mg/kg for a 70 kg patient, for example, is 70-1,400 mg, or 41-824 mg/m² for a 1.7-m patient. The dosage may be repeated as needed, for example, once per week for 4-10 weeks, once per week for 8 weeks, or once per week for 4 weeks. It may also be given less frequently, such as every other week for several months, or monthly or quarterly for many months, as needed in a maintenance therapy.

Alternatively, a bsAb, ADC, and/or checkpoint inhibitor antibody may be administered as one dosage every 2 or 3 weeks, repeated for a total of at least 3 dosages. Or, the combination may be administered twice per week for 4-6 weeks. If the dosage is lowered to approximately 200-300 mg/m² (340 mg per dosage for a 1.7-m patient, or 4.9 mg/kg for a 70 kg patient), it may be administered once or even twice weekly for 4 to 10 weeks. Alternatively, the dosage schedule may be decreased, namely every 2 or 3 weeks for 2-3 months. It has been determined, however, that even higher doses, such as 20 mg/kg once weekly or once every 2-3 weeks can be administered by slow i.v. infusion, for repeated dosing cycles. The dosing schedule can optionally be repeated at other intervals and dosage may be given through various parenteral routes, with appropriate adjustment of the dose and schedule.

The person of ordinary skill will realize that while the dosage schedules discussed above are relevant for ADCs, bsAbs and/or mAbs, the interferon agents should be administered at substantially lower dosages to avoid systemic toxicity. Dosages of interferons (such as PEGINTERFERON) for humans are more typically in the microgram range, for example 180 µg s.c. once per week, or 100 to 180 µg, or 135 µg, or 135 µg/1.73 m², or 90 µg/1.73 m², or 250 µg s.c. every other day may be of use, depending on the type of interferon.

While the bsAbs, interferons, ADCs and/or checkpoint inhibitor antibodies may be administered as a periodic bolus injection, in alternative embodiments the bsAbs, ADCs, interferons and/or checkpoint inhibitor antibodies may be administered by continuous infusion. In order to increase the Cmax and extend the PK of the therapeutic agents in the blood, a continuous infusion may be administered for example by indwelling catheter. Such devices are known in the art, such as HICKMAN^{®}, BROVIAC^{®} or PORT-A-CATH^{®} catheters (see, e.g., Skolnik et al., Ther Drug Monit 32:741-48, 2010) and any such known indwelling catheter may be used. A variety of continuous infusion pumps are also known in the art and any such known infusion pump may be used. The dosage range for continuous infusion may be between 0.1 and 3.0 mg/kg per day. More preferably, the bsAbs, ADCs, interferons and/or checkpoint inhibitor antibodies can be administered by intravenous infusions over relatively short periods of 2 to 5 hours, more preferably 2-3 hours.

Examples of such cancers are noted below and include: squamous cell cancer (e.g., epithelial squamous cell cancer), Ewing sarcoma, Wilms tumor, astrocytomas, lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma multiforme, cervical cancer, ovarian cancer, liver cancer, bladder cancer, hepatoma, hepatocellular carcinoma, neuroendocrine tumors, medullary thyroid cancer, differentiated thyroid carcinoma, breast cancer, ovarian cancer, colon cancer, rectal cancer, endometrial cancer or uterine carcinoma, salivary gland carcinoma, kidney or renal cancer, prostate cancer, vulvar cancer, anal carcinoma, penile carcinoma, as well as head-and-neck cancer. The term "cancer" includes primary malignant cells or tumors (e.g., those whose cells have not migrated to sites in the subject's body other than the site of the original malignancy or tumor) and secondary malignant cells or tumors (e.g., those arising from metastasis, the migration of malignant cells or tumor cells to secondary sites that are different from the site of the original tumor). Cancers conducive to treatment methods of the present invention involves cells which express, over-express, or abnormally express IGF-1R.

Other examples of cancers or malignancies include, but are not limited to: Acute Childhood Lymphoblastic Leukemia, Acute Lymphoblastic Leukemia, Acute Lymphocytic Leukemia, Acute Myeloid Leukemia, Adrenocortical Carcinoma, Adult (Primary) Hepatocellular Cancer, Adult (Primary) Liver Cancer, Adult Acute Lymphocytic Leukemia, Adult Acute Myeloid Leukemia, Adult Hodgkin's Lymphoma, Adult Lymphocytic Leukemia, Adult Non-Hodgkin's Lymphoma, Adult Primary Liver Cancer, Adult Soft Tissue Sarcoma, AIDS-Related Lymphoma, AIDS-Related Malignancies, Anal Cancer, Astrocytoma, Bile Duct Cancer, Bladder Cancer, Bone Cancer, Brain Stem Glioma, Brain Tumors, Breast Cancer, Cancer of the Renal Pelvis and Ureter, Central Nervous System (Primary) Lymphoma, Central Nervous System Lymphoma, Cerebellar Astrocytoma, Cerebral Astrocytoma, Cervical Cancer, Childhood (Primary) Hepatocellular Cancer, Childhood (Primary) Liver Cancer, Childhood Acute Lymphoblastic Leukemia, Childhood Acute Myeloid Leukemia, Childhood Brain Stem Glioma, Childhood Cerebellar Astrocytoma, Childhood Cerebral Astrocytoma, Childhood Extracranial Germ Cell Tumors, Childhood Hodgkin's Disease, Childhood Hodgkin's Lymphoma, Childhood Hypothalamic and Visual Pathway Glioma, Childhood Lymphoblastic Leukemia, Childhood Medulloblastoma, Childhood Non-Hodgkin's Lymphoma, Childhood Pineal and Supratentorial Primitive Neuroectodermal Tumors, Childhood Primary Liver Cancer, Childhood Rhabdomyosarcoma, Childhood Soft Tissue Sarcoma, Childhood Visual Pathway and Hypothalamic Glioma, Chronic Lymphocytic Leukemia, Chronic Myelogenous Leukemia, Colon Cancer, Cutaneous T-Cell Lymphoma, Endocrine Pancreas Islet Cell Carcinoma, Endometrial Cancer, Ependymoma, Epithelial Cancer, Esophageal Cancer, Ewing's Sarcoma and Related Tumors, Exocrine Pancreatic Cancer, Extracranial Germ Cell Tumor, Extragonadal Germ Cell Tumor, Extrahepatic Bile Duct Cancer, Eye Cancer, Female Breast Cancer, Gaucher's Disease, Gallbladder Cancer, Gastric Cancer, Gastrointestinal Carcinoid Tumor, Gastrointestinal Tumors, Germ Cell Tumors, Gestational TROPhoblastic Tumor, Hairy Cell Leukemia, Head and Neck Cancer, Hepatocellular Cancer, Hodgkin's Lymphoma, Hypergammaglobulinemia, Hypopharyngeal Cancer, Intestinal Cancers, Intraocular Melanoma, Islet Cell Carcinoma, Islet Cell Pancreatic Cancer, Kaposi's Sarcoma, Kidney Cancer, Laryngeal Cancer, Lip and Oral Cavity Cancer, Liver Cancer, Lung Cancer, Lymphoproliferative Disorders, Macroglobulinemia, Male Breast Cancer, Malignant Mesothelioma, Malignant Thymoma, Medulloblastoma, Melanoma, Mesothelioma, Metastatic Occult Primary Squamous Neck Cancer, Metastatic Primary Squamous Neck Cancer, Metastatic Squamous Neck Cancer, Multiple Myeloma, Multiple Myeloma/Plasma Cell Neoplasm, Myelodysplastic Syndrome, Myelogenous Leukemia, Myeloid Leukemia, Myeloproliferative Disorders, Nasal Cavity and Paranasal Sinus Cancer, Nasopharyngeal Cancer, Neuroblastoma, Non-Hodgkin's Lymphoma, Nonmelanoma Skin Cancer, Non-Small Cell Lung Cancer, Occult Primary Metastatic Squamous Neck Cancer, Oropharyngeal Cancer, Osteo-/Malignant Fibrous Sarcoma, Osteosarcoma/Malignant Fibrous Histiocytoma, Osteosarcoma/Malignant Fibrous Histiocytoma of Bone, Ovarian Epithelial Cancer, Ovarian Germ Cell Tumor, Ovarian Low Malignant Potential Tumor, Pancreatic Cancer, Paraproteinemias, Polycythemia vera, Parathyroid Cancer, Penile Cancer, Pheochromocytoma, Pituitary Tumor, Primary Central Nervous System Lymphoma, Primary Liver Cancer, Prostate Cancer, Rectal Cancer, Renal Cell Cancer, Renal Pelvis and Ureter Cancer, Retinoblastoma, Rhabdomyosarcoma, Salivary Gland Cancer, Sarcoidosis Sarcomas, Sezary Syndrome, Skin Cancer, Small Cell Lung Cancer, Small Intestine Cancer, Soft Tissue Sarcoma, Squamous Neck Cancer, Stomach Cancer, Supratentorial Primitive Neuroectodermal and Pineal Tumors, T-Cell Lymphoma, Testicular Cancer, Thymoma, Thyroid Cancer, Transitional Cell Cancer of the Renal Pelvis and Ureter, Transitional Renal Pelvis and Ureter Cancer, TROPhoblastic Tumors, Ureter and Renal Pelvis Cell Cancer, Urethral Cancer, Uterine Cancer, Uterine Sarcoma, Vaginal Cancer, Visual Pathway and Hypothalamic Glioma, Vulvar Cancer, Waldenstrom's Macroglobulinemia, Wilms' Tumor, and any other hyperproliferative disease, besides neoplasia, located in an organ system listed above.

In an embodiment of the invention as defined in the claims, the bispecific antibody may be used to treat malignant or premalignant conditions and to prevent progression to a neoplastic or malignant state, including but not limited to those disorders described above. Such uses are indicated in conditions known or suspected of preceding progression to neoplasia or cancer, in particular, where non-neoplastic cell growth consisting of hyperplasia, metaplasia, or most particularly, dysplasia has occurred (for review of such abnormal growth conditions, see Robbins and Angell, BASIC PATHOLOGY, 2d Ed., W. B. Saunders Co., Philadelphia, pp. 68-79 (1976)).

Dysplasia is frequently a forerunner of cancer, and is found mainly in the epithelia. It is the most disorderly form of non-neoplastic cell growth, involving a loss in individual cell uniformity and in the architectural orientation of cells. Dysplasia characteristically occurs where there exists chronic irritation or inflammation. Dysplastic disorders which can be treated include, but are not limited to, anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis hemimelia, dysplasia epiphysialis multiplex, dysplasia epiphysialis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia, hidrotic ectodermal dysplasia, hypohidrotic ectodermal dysplasia, lymphopenic thymic dysplasia, mammary dysplasia, mandibulofacial dysplasia, metaphysial dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, opthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

Additional pre-neoplastic disorders which can be treated include, but are not limited to, benign dysproliferative disorders (e.g., benign tumors, fibrocystic conditions, tissue hypertrophy, intestinal polyps or adenomas, and esophageal dysplasia), leukoplakia, keratoses, Bowen's disease, Farmer's Skin, solar cheilitis, and solar keratosis.

In preferred embodiments of the invention as defined in the claims, the bispecific antibody is used to inhibit growth, progression, and/or metastasis of cancers, in particular those listed above.

Additional hyperproliferative diseases, disorders, and/or conditions include, but are not limited to, progression, and/or metastases of malignancies and related disorders such as leukemia (including acute leukemias (e.g., acute lymphocytic leukemia, acute myelocytic leukemia (including myeloblastic, promyelocytic, myelomonocytic, monocytic, and erythroleukemia)) and chronic leukemias (e.g., chronic myelocytic (granulocytic) leukemia and chronic lymphocytic leukemia)), polycythemia vera, lymphomas (e.g., Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, emangioblastoma, acoustic neuroma, oligodendroglioma, meningioma, melanoma, neuroblastoma, and retinoblastoma.

### Expression Vectors

The instant discosure also concerns DNA sequences comprising a nucleic acid encoding an antibody, antibody fragment, cytokine or constituent fusion protein of a bsAb, such as a DNL^{™} construct. Fusion proteins may comprise an antibody or fragment or cytokine attached to, for example, an AD or DDD moiety.

Various examples relate to expression vectors comprising the coding DNA sequences. The vectors may contain sequences encoding the light and heavy chain constant regions and the hinge region of a human immunoglobulin to which may be attached chimeric, humanized or human variable region sequences. The vectors may additionally contain promoters that express the encoded protein(s) in a selected host cell, enhancers and signal or leader sequences. Vectors that are particularly useful are pdHL2 or GS. More preferably, the light and heavy chain constant regions and hinge region may be from a human EU myeloma immunoglobulin, where optionally at least one of the amino acid in the allotype positions is changed to that found in a different IgG1 allotype, and wherein optionally amino acid 253 of the heavy chain of EU based on the EU number system may be replaced with alanine. See Edelman et al., Proc. Natl. Acad. Sci USA 63: 78-85 (1969). In other embodiments, an IgG1 sequence may be converted to an IgG4 sequence.

The skilled artisan will realize that methods of genetically engineering expression constructs and insertion into host cells to express engineered proteins are well known in the art and a matter of routine experimentation. Host cells and methods of expression of cloned antibodies or fragments have been described, for example, in U.S. Patent Nos. 7,531,327, 7,537,930, 7,785,880, 8,076,410, 8,153,433 and 8,372,603.

### Kits

The instant disclosure also concerns kits containing components suitable for treating or diagnosing diseased tissue in a patient. Exemplary kits may contain one or more bsAbs, ADCs, interferons, and/or checkpoint inhibitor antibodies as described herein. If the composition containing components for administration is not formulated for delivery via the alimentary canal, such as by oral delivery, a device capable of delivering the kit components through some other route may be included. One type of device, for applications such as parenteral delivery, is a syringe that is used to inject the composition into the body of a subject. Inhalation devices may also be used. In certain examples, a therapeutic agent may be provided in the form of a prefilled syringe or autoinjection pen containing a sterile, liquid formulation or lyophilized preparation.

The kit components may be packaged together or separated into two or more containers. In some embodiments, the containers may be vials that contain sterile, lyophilized formulations of a composition that are suitable for reconstitution. A kit may also contain one or more buffers suitable for reconstitution and/or dilution of other reagents. Other containers that may be used include, but are not limited to, a pouch, tray, box, tube, or the like. Kit components may be packaged and maintained sterilely within the containers. Another component that can be included is instructions to a person using a kit for its use.

### EXAMPLES

### Example 1. T-Cell Redirecting Bispecific Antibody DOCK-AND-LOCK^{™} (DNL^{™}) Complexes

Several species of exemplary leukocyte redirecting bispecific antibodies were made as DNL^{™} complexes, as described below. The complexes were effective to induce an immune response against appropriate target cells including, but not limited to, Trop-2⁺ cancer cells.

### Materials and Methods

General techniques for making and using DOCK-AND-LOCK^{™} (DNL^{™}) complexes are described in the Examples below. An exemplary leukocyte redirecting bispecific antibody with binding sites for CD3 and CD19 was made as a DNL^{™} complex, referred to as (19)-3s (**FIG. 1**). An anti-CD19 F(ab)₂ DNL module was constructed by recombinant fusion of a dimerization and docking domain (DDD2) at the carboxyl terminal end of the Fd chain. An anti-CD3-scFv module was designed from Okt3 mAb with addition of an anchor domain (AD2) and assembled in the format V_{H}-L1-V_{K}-L2-6H-L3-AD2 ("6H" disclosed as SEQ ID NO: 105), where the V domains were fused via a flexible peptide linker and the AD2 peptide was preceded by a 6-His linker (SEQ ID NO: 105). The sequences of the anti-CD3 variable regions, linkers and AD2 were as shown below.

### V_{H} sequence of anti-CD3 scFv

QVQLQQSGAELARPGASVKMSCKASGYTFTRYTMHWVKQRPGQGLEWIGYINPSR
GYTNYNQKFKDKATLTTDKSSSTAYMQLSSLTSEDSAVYYCARYYDDHYSLDYWG
QGTTLTVSS (SEQ ID NO:96)

### L1 Linker

GGGGSGGGGSGGGGS (SEQ ID NO:97)

### V_{K} sequence of anti-CD3 scFv

DIVLTQSPAIMSASPGEKVTMTCSASSSVSYMNWYQQKSGTSPKRWIYDTSKLASGV

PAHFRGSGSGTSYSLTISGMEAEDAATYYCQQWSSNPFTFGSGTKLEIKR (SEQ
ID NO:98)

### L2 Linker

GGGGS (SEQ ID NO:99)

### Poly-His-L3 Linker

HHHHHHGGGSG (SEQ ID NO:100)

### AD2

### CGQIEYLAKQIVDNAIQQAGC (SEQ ID NO:101)

Expression vectors and DNL^{™} modules - DNL^{™} complexes were constructed comprising antibody moieties against various disease-associated antigens, linked to an anti-CD3 antibody moiety, generally abbreviated as (X)-3s bsAbs. Independent production cell lines were developed in SpESFX-10 mouse myeloma cells (Rossi et al., 2011, Biotechnol Prog 27:766-75) for each of the DNL^{™} modules used to make the (X)-3s bsAbs. A cDNA sequence encoding the Okt3scFv-AD2 polypeptide (SEQ ID NOs:96-101) was synthesized and cloned into the pdHL2 expression vector via 5' Xba I and 3' Eag I restriction sites. The construct comprised the V_{H} domain fused to the V_{L} in an scFv with the structure V_{H}-L1-V_{K}-L2-6H-L3-AD2 ("6H" disclosed as SEQ ID NO:105). The expressed protein had two amino acid substitutions from the original Okt3 mAb. A cysteine residue in the CDR-H3 was changed to serine (Kipryanov, 1997, J Immunol Methods 200:69-77). The penultimate residue of the V_{L} was changed from aspartate to lysine.

The Okt3scFv-AD2 module was combined with various C_{H}1-DDD2-Fab modules to generate a panel of (X)-3s trivalent bsAbs (**Table 6**). The C_{H}1-DDD2-Fab-pdHL2 expression vectors were constructed as described previously for similar constructs (Rossi et al., 2008, Cancer Res 68:8384-92). Briefly, expression vectors encoding C_{H}1-DDD2-Fab were generated from the corresponding IgG-pdHL2 expression vectors by excising the coding sequence for the C_{H}1-Hinge-C_{H}2-C_{H}3 domains with Sac II and Eag I restriction enzymes and replacing it with a 507 bp sequence encoding C_{H}1-DDD2, which was excised from the C_{H}1-DDD2-Fab-hA20-pdHL2 expression vector (Rossi et al., 2008, Cancer Res 68:8384-92) with the same enzymes. C_{H}1-DDD2-Fab modules were derived from the humanized mAbs hA19 (anti-CD19), labetuzumab (hMN-14, anti-CEACAM5), clivatuzumab (hPAM4, anti-mucin), hMN-15 (anti-CEACAM6), hRS7 (anti-TROP-2), veltuzumab (hA20, anti-CD20), hL243 (anti-HLA-DR) and epratuzumab (hLL2, anti-CD22). The mAb designated hA19 was humanized from the mouse anti-CD19 mAb B43 (Uckun et al., 1988, Blood 71:13-29). Each expression vector was linearized by digestion with Sal I restriction enzyme and used to transfect SpESFX-10 cells by electroporation.

Clones were selected in media containing 0.2 µM methotrexate (MTX) and screened for protein expression by ELISA. Okt3scFv-AD2 was captured on Ni-NTA HisSorb plates (Qiagen) and detected with an anti-AD2 mAb. C_{H}1-DDD2-Fab modules were captured with goat-anti-human-kappa chain and detected with goat-anti-human-F(ab')₂-HRP. Productivity of protein-expression was amplified by stepwise increases in MTX concentration up to 3 µM. Okt3scFv-AD2 and C_{H}1-DDD2-Fab modules were purified to homogeneity from the broth of roller bottle cultures by affinity chromatography using Ni-SEPHAROSE^{®} and Kappa-Select resins, respectively. The DNL^{™} method was used to assemble (X)-3s bsAbs via the site-specific conjugation of mole equivalents of Okt3scFv-AD2 and C_{H}1-DDD2-Fab modules. For example, approximately 100 mg of (19)-3s were produced by combining 22 mg of Okt3scFv-AD2 with 80 mg of C_{H}1-DDD2-Fab-hA19. The mixture was reduced overnight at room temperature with 1 mM reduced glutathione prior to the addition of 2 mM oxidized glutathione. The (19)-3s was purified from the reaction mixture by sequential affinity chromatography with Kappa-Select and Ni- SEPHAROSE^{®}. Additional (X)-3s constructs were assembled at various scales following a similar process.

**Table 6. (X)-3s DNL^{™} Constructs**

| Code | Target | C_{H}1-DDD2-Fab | AD2-anti-CD3 |
|---|---|---|---|
| (19)-3s | CD19 | C_{H}1-DDD2-Fab-hA19 | scFv-AD2- Okt3 |
| (20)-3s | CD20 | C_{H}1-DDD2-Fab-hA20 | scFv-AD2- Okt3 |
| (22)-3s | CD22 | C_{H}1-DDD2-Fab-hLL2 | scFv-AD2- Okt3 |
| (C2)-3s | HLA-DR | C_{H}1-DDD2-Fab-hL243 | scFv-AD2- Okt3 |
| (M1)-3s | MUC5AC | C_{H}1-DDD2-Fab-hPAM4 | scFv-AD2- Okt3 |
| (14)-3s | CEACAM5 | C_{H}1-DDD2-Fab-hMN-14 | scFv-AD2- Okt3 |
| (15)-3s | CEACEAM6 | C_{H}1-DDD2-Fab-hMN-15 | scFv-AD2- Okt3 |
| (E1)-3s | TROP-2 | C_{H}1-DDD2-Fab-hRS7 | scFv-AD2- Okt3 |

Analytical Methods - Size-exclusion high-performance liquid chromatography (SE-HPLC) was performed with an Alliance HPLC System with a BIOSUITE^{™} 250, 4-µm UHR SEC column (Waters Corp). Electrospray ionization time of flight (ESI-TOF) liquid chromatography/mass spectrometry (LC-MS) was performed with a 1200-series HPLC coupled with a 6210 TOF MS (Agilent Technologies, Santa Clara, CA). The (19)-3s was resolved by reversed phase HPLC (RP-HPLC) at 60°C, using a 14-min gradient of 30 - 80% acetonitrile in 0.1% aqueous formic acid with an Aeris widepore 3.6 µm C4 column (Phenomenex). For the TOF MS, the capillary and fragmentor voltages were set to 5500 and 300 V, respectively.

Cell Lines and Reagents - Raji, Ramos, Daudi, LS174T and Capan-1 cell lines were purchased from the American Type Cell Culture Collection (ATCC, Manassas, MD) and Nalm-6 cells were purchased from Deutsche Sammlung von Mikroorganismen und Zellinien (DSMZ, Braunchweig, Germany). All cell lines, except Capan-1, were maintained in RPMI-1640 containing 10% FBS, 1% L-glutamine, 1% penicillin-streptomycin and 1% MEM nonessential amino acids. Capan-1 cells were maintained with 20% FBS. All cell culture media and supplements were purchased from Life Technologies (Carlsbad, CA).

PBMCs and T cell isolation - Human peripheral blood mononuclear cells (PBMC) were purified from whole donor blood (Blood Center of NJ, East Orange, NJ) using UNI-SEP_{MAXI} tubes (Novamed, Ltd, Jerusalem, Israel). CD3-positive T cells were isolated from PBMCs by negative selection using the Pan T Cell Isolation Kit (Miltenyi Biotec, Auburn, CA), according to the manufacturer's protocol. Efficiency of T cell isolation was assessed by FACS after staining the enriched T cells with anti-CD3-PE antibody. In some cases, further staining with CD-19 and CD-14 was performed as well to identify contaminating cells.

T cell activation - Isolated T cells were plated in 6-well tissue culture plates at a final density of 2.25 × 10⁶ cells/well. Daudi cells were added to some wells at a final density of 1.5 × 10⁶ cells/well, other wells were left to contain only T cells. Alternatively, PBMCs were added to 6-well tissue culture plates at a final cell density of 6 × 10⁶ cells/well. The volume of each well was brought up to 3 mL. To the appropriate wells, 3 ng/mL of (19)-3s, (M1)-3s or (19)-DDD2 was added. After incubation overnight at 37°C, 1 mL of each sample was removed. The cells were pelleted and labeled on ice with CD69-APC and CD3-PE for 20 minutes. Cells were washed 2 times with 1% BSA in PBS and analyzed using a FACSCALIBER^{™} flow cytometer (BD Biosciences, San Jose, CA).

T-cell proliferation - PBMCs were seeded in T25 flasks at a concentration of 1 × 10⁶ cells/mL containing the specified reagents. For B cell-depleted flasks, B cells were removed by negative selection using a B-cell isolation kit from Miltenyi according to manufacturer's protocol. On select days, 100 µL of media was removed from each flask, labeled with anti-CD7-APC for 20 minutes on ice, washed once and resuspended in 300 µL of 1% BSA/PBS containing 7-AAD. For each sample, the entire volume is analyzed using a FACSCALIBER^{™} flow cytometer. Each sample is counted in duplicate. Analysis is performed using FlowJo Software. For each sample, dead (7-AAD+) cells, and debris (based on forward vs. side scatter) was removed. Finally, live CD7+ cells were selected and plotted using Prism software.

Cell Binding Assays (Jurkat/Capan-1) - Jurkat cells were stained with PKH26 Red Fluorescent Cell Linker Kit (Sigma) according to manufacturer's protocol. Capan-1 cells were stained with 5 µM CFSE (carboxyfluorescein diacetate succinimidyl ester, Life Technologies) according to manufacturer's protocol. Labeled Capan-1 cells were added to 8-well chamber slides (ThermoWaltham, MA) and allowed to attach overnight. The following day, media was removed and PKH26-labeled Jurkat cells were added in media containing 0.1 µg/mL of (E1)-3s, (M1)-3s or (19)-3s. Following a 1-hour incubation at 37°C, slides were washed with PBS to remove any unbound cells and observed by fluorescence microscopy.

Cell Binding Assays (Jurkat/Daudi) - Jurkat and Daudi cells were labeled with anti-CD3-PE and anti-CD20-FITC, respectively. Labeled cells were then coincubated at a 2.5:1 ratio with 0.1 µg/mL (19)-3s for 30 minutes at room temperature. Aliquots of cells were then observed by fluorescence microscopy.

Cytotoxicity Assay (Hematologic Tumor Cell Lines) - Target cells were labeled with PKH67 Green Fluorescent Cell Linker Kit (Sigma) according to the manufacturer's protocol. Briefly, 5 × 10⁶ target cells were resuspended in 250 µL of diluent C. In a second tube 1 µL of PKH26 dye is added to 250 µL of diluent C. The cell suspension is then added to the dye solution, mixed thoroughly and incubated at RT for 2 minutes. The reaction was quenched by adding an equal volume of FBS. The labeled cells were then washed 3 times with complete RPMI. Unstimulated, isolated T cells were used as effector cells. Effector cells and PKH67-labeled target cells were combined at a 10:1 ratio and plated in 48-well plates containing serial dilutions of (19)-3s or (14)-3s. Each well contained 5 × 10⁴ target cells and 5 × 10⁵ effector cells. Jeko-1 assays were performed in 20% RPMI. Plates were incubated for 18 -24 hours in a 37°C incubator containing 5% CO₂. Following incubation, all cells were removed from 48-well plates into flow cytometer tubes and resuspended in 1% BSA/PBS containing 1 ug/mL of 7AAD, to distinguish live from dead cells, and 30,000 COUNTBRIGHT^{™} Absolute Counting Beads (Life Technologies). Cells were analyzed on a FACSCALIBER^{™} flow cytometer. For each sample, 8,000 COUNTBRIGHT^{™} beads were counted as a normalized reference. Data were analyzed using FlowJo software (Treestar, Inc., Ashland, OR). For each sample, dead cells and debris were excluded and total live target cells were counted.

Cytotoxicity Assay (Solid Tumor Cell Lines) - Target cells were labeled with PKH67 Green Fluorescent Cell Linker Kit (Sigma) following the same procedure as for staining with PKH23. Effector cells used were as follows: For Capan-1 assays, CD8+ enriched T cells were used, following purification from a CD8+ enrichment column (R&D Systems, Minneapolis, MN). For LS174T cells: Stimulated T cells were used after incubation of PBMC for 5 days in media containing 25 U/mL IL-2 and 50 ng/mL Okt3 Mab, followed by 2 days incubation in media containing 25 U/mL IL-2 alone. Effector cells and PKH67-labeled target cells were combined at a 3:1 ratio (5×10⁴ target cells and 1.5×10⁵ effector cells/well) and plated over 48-well plates containing serial dilutions of (E1)-3s, (14)-3s or (19)-3s. Capan-1 assays were performed in 20% RPMI. Plates were incubated for 42 - 48 hours in a 37°C incubator containing 5% CO₂. Following incubation, suspension cells were combined with trypsinized attached cells from all wells and transferred into flow cytometer tubes. Cells were washed one time and resuspended in 1% BSA/PBS containing 1 ug/mL of 7AAD, to distinguish live from dead cells, and 30,000 COUNTBRIGHT^{™} Absolute Counting Beads. Cells were analyzed on a FACSCALIBER^{™} flow cytometer. For each sample, 8,000 COUNTBRIGHT^{™} beads were counted as a normalized reference. Data were analyzed using FlowJo software (Treestar, Inc., Ashland, OR). For each sample, dead cells and debris were excluded and total live target cells were counted.

*In Vivo* Efficacy - Female NOD/SCID mice, 8 weeks old, were purchased from Charles River (Wilmington, MA). Mice were injected s.c. with a mixture of Raji (1×10⁶) and human PBMCs (5×10⁶ cells) mixed 1:1 with matrigel. Therapy began 1 hour later. Treatment regimens, dosages, and number of animals in each experiment are described in the **Results**. Animals were monitored daily for signs of tumor out-growth. Once tumors appeared, they were measured twice weekly. Tumor volume (TV) was determined by measurements in two dimensions using calipers, with volumes defined as: *L* × *w*²/2, where *L* is the longest dimension of the tumor and *w* the shortest. Efficacy was determined by a log-rank test using Prism GraphPad software (v5; LaJolla, CA) on Kaplan-Meier curves using survival surrogate endpoints as time for tumor progression (TTP) to 1.0 cm³. Significance was considered at *P* <0.05.

### Results

Construction and biochemical analysis of leukocyte redirecting bispecific antibodies. The DNL^{™} method was used to generate a panel of (X)-3s, leukocyte redirecting bsAbs for targeting of various tumor-associated antigens including CD19, CD20, HLA-DR, TROP-2, CEACAM5 and MUC5AC. The purity of these structures was demonstrated by SE-HPLC and SDS-PAGE analysis, where only bands representing the three constituent polypeptides (Okt3scFv-AD2, hA19-Fd-DDD2 and hA19 kappa) were evident (data not shown). LC-MS analysis identified a single RP-HPLC peak having a deconvoluted mass spectrum consistent (mass accuracy = 11 ppm) with the calculated mass (137432.37 Da) of (19)-3s from its deduced amino acid sequence, including the predicted amino-terminal pyroglutamates on the Okt3scFv-AD2 and each of the two C_{H}1-DDD2-hA19 Fd chains (data not shown). No additional post-translational modifications, including glycosylation were indicated.

Immune synapse formation between Daudi Burkitt lymphoma and T cells, mediated by (19)-3s. The effects of the leukocyte redirecting (19)-3s DNL^{™} complex on targeting effector T cells to CD19⁺ lymphoma cells was examined (**FIG. 2**). Freshly isolated T cells were combined with Daudi cells at an E:T ratio of 2.5:1. Cells were treated with 0, 1 or 5 µg/mL of (19)-3s DNL^{™} complex for 30 min at room temperature prior to analysis by flow cytometry. Anti-CD20-FITC and anti-CD7-APC were used to identify Daudi and T cells, respectively. Co-binding was indicated as the % of CD20⁺/CD7⁺ events. After treatment with (19)-3s, 45.5% of flow events were CD20/CD7 dual-positive, indicating synapsed Daudi and T cells (**FIG. 2A**), compared to 2% measured for the mixed cells without antibody (**FIG. 2B**). Addition of (19)-3s resulted in association of >90% of the Daudi with T cells (**FIG. 2C**). These results show that the (19)-3s DNL^{™} complex was effective to direct T cells to the targeted antigen-expressing lymphoma cells.

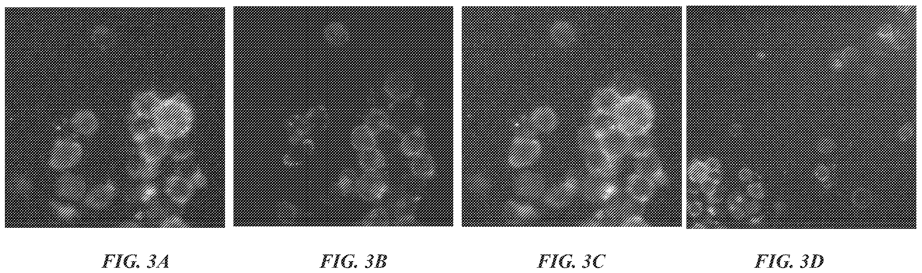

Synapse formation between T cells and target lymphoma cells was demonstrated by fluorescence microscopy (**FIG. 3**) Jurkat (T cells) and Daudi (B cells) were combined at a 1:1 ratio, treated with 0.1 µg/mL of the (19)-3s DNL^{™} complex for 30 minutes and stained with anti-CD20-FITC (**FIG. 3A**) and anti-CD3-PE (**FIG. 3B**), prior to analysis by fluorescence microscopy. The merged image (**FIG. 3C**) reveals synapse formation between green-stained Daudi and red-stained Jurkat cells. Synapse formation was not evident in the absence of (19)-3s (**FIG. 3D**). **FIG. 3C** demonstrates that the target lymphoma cells are in direct contact with the targeted T cells.

A dose-response series was performed for (19)-3s mediated association of T cells to an exemplary B-cell lymphoma line (**FIG. 4**). As shown in **FIG. 4**, under the conditions of this experiment, saturation of (19)-3s-mediated cell-to-cell association of T cells to target cells was reached at a concentration between 0.037 and 0.111 µg/ml of the DNL^{™} complex.

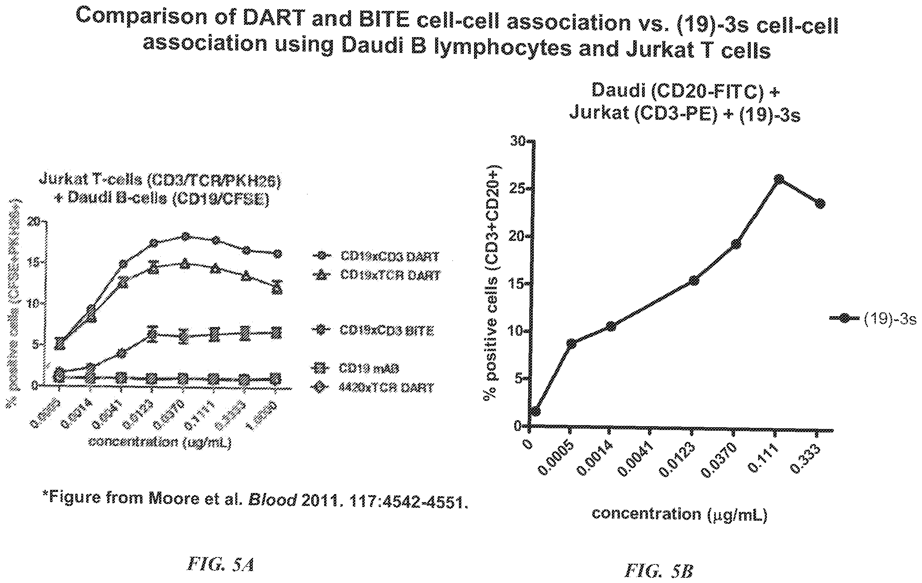

**FIG. 5** shows a comparision of the relative efficacies of BITE^{®} (**FIG. 5A**), DART^{™} (**FIG. 5A**) and DNL^{™} (**FIG. 5B**) anti-CD3 × anti-CD19 complexes for redirecting T cells to targeted CD19⁺ B cells. The data for BITE^{®} and DART^{™} was obtained from Moore et al. (2011, Blood 117:4542-51). At the lowest concentration tested of 0.0005 µg/ml, the (19)-3s DNL^{™} complex was more effective than BITE^{®} or DART^{™} at targeting T cells to B-cell lymphoma (**FIG. 5**). The (19)-3s DNL^{™} complex also induced a slightly higher maximum level of cell-to-cell association than the comparable BITE^{®} and DART^{™} complexes (**FIG. 5A**). Although difficult to extrapolate from the single data points generated for the (19)-3s DNL^{™} complex, the EC₅₀ levels appeared to be similar for BITE^{®}, DART^{™} and DNL^{™} (**FIG. 5**).

(19)-3s, (E1)-3s and (M1)-3s-mediated cell-cell association of T cells to target tumor cells. To evaluate the ability of the T-cell redirecting BsAbs to facilitate the association of T cells to their target tumor cells, Jurkat T cells were coincubated with target tumor cells containing (X)-3s and evaluated by flow cytometry and fluorescence microscopy. Jurkat T cells are a CD4+ T cell leukemia line, chosen for their ability to demonstrate T cell binding without depletion of the FITC labeled Daudi cells in the presence of various concentrations of (19)-3s and analyzed by flow cytometry for the detection of double positive (CD3+CD20+) populations indicating T cell-B cell associated complexes. An apparent cell-cell association was seen following treatment with 0.5 ng/mL of (19)-3s and after treatment with 0.1 µg/mL over 25% of the cell population existed in a cell-cell association (**FIG. 5**). Fluorescent microscopy supports this data, as immune synapses are evident following treatment with 0.1 µg/mL (19)-3s (**FIG. 4**). No synapse formation was seen in the absence of (19)-3s (data not shown).

This cell-cell association was observed in the pancreatic tumor line Capan-1 as well (**FIG. 6**). Capan-1 expresses high levels of TROP2 and moderate levels of MUC5AC. Therefore, both the TROP2-targeting bsAb, (E1)-3s (**FIG. 6C**), and MUC5AC -targeting bsAb, (M1)-3s (**FIG. 6B**) were compared to the non-targeting control bsAb, (19)-3s (**FIG. 6A**). CFSE-labeled Capan-1 cells were coincubated with PKH26-labeled Jurkat in the presence of these bsAbs. Fluorescent microscopy revealed, as expected, large T-cell/Capan complexes mediated by (E1)-3s, followed by smaller, yet substantial complexes mediated by (M1)-3s and relatively low complex formation following (19)-3s treatment (**FIG. 6**).

(19)-3s specifically induces T cell activation and proliferation. The ability of (19)-3s to activate T cells was evaluated either in PBMCs (**FIG. 7A**), or T cells coincubated with Daudi B cells (**FIG. 7B**), by measuring the expression levels of CD69, an early marker of T cell activation. Treatment with 3 ng/mL of (19)-3s induced T cell activation in T cells coincubated with Daudi B cells as indicated by a >50-fold increase in CD69 expression compared with non-targeting control antibodies, (19)-DDD2 and (M1)-3s, as well as T cells treated with (19)-3s without Daudi target cells (**FIG. 7B**). Similar results were observed when the antibodies were incubated with PBMCs, containing both T and B cells; (19)-3s stimulated CD69 expression levels >20-fold higher than non-targeting controls (**FIG. 7A**). In the absence of target cells, purified T cells treated with (19)-3s did not show activation (**FIG. 7C**).

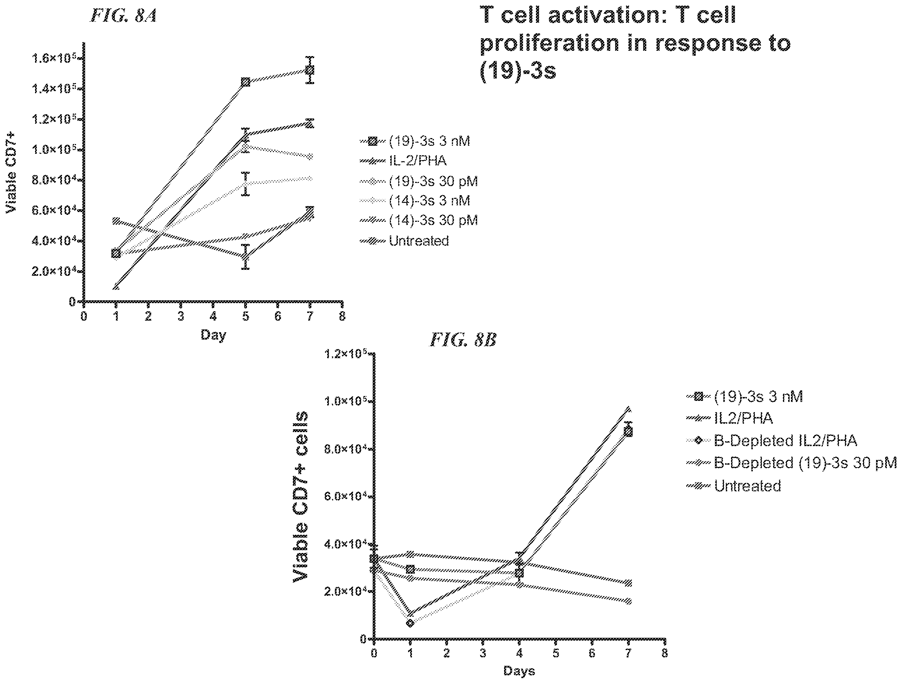

T cell proliferation, as another indication of T cell activation, was evaluated after treatment of PBMCs with various CD3-targeting antibodies. (19)-3s at 3 nM or 30 pM induced T cell proliferation similar to that of the positive control IL-2/PHA (**FIG. 8A**). Non-targeting control antibody, (14)-3s, shows some non-specific T cell proliferation at the highest (3 nM) concentration (**FIG. 8A**). However, T cell proliferation was not observed in PBMCs depleted of B cells (**FIG. 8B**), suggesting that target cells are necessary for specific (19)-3s induced T cell proliferation.

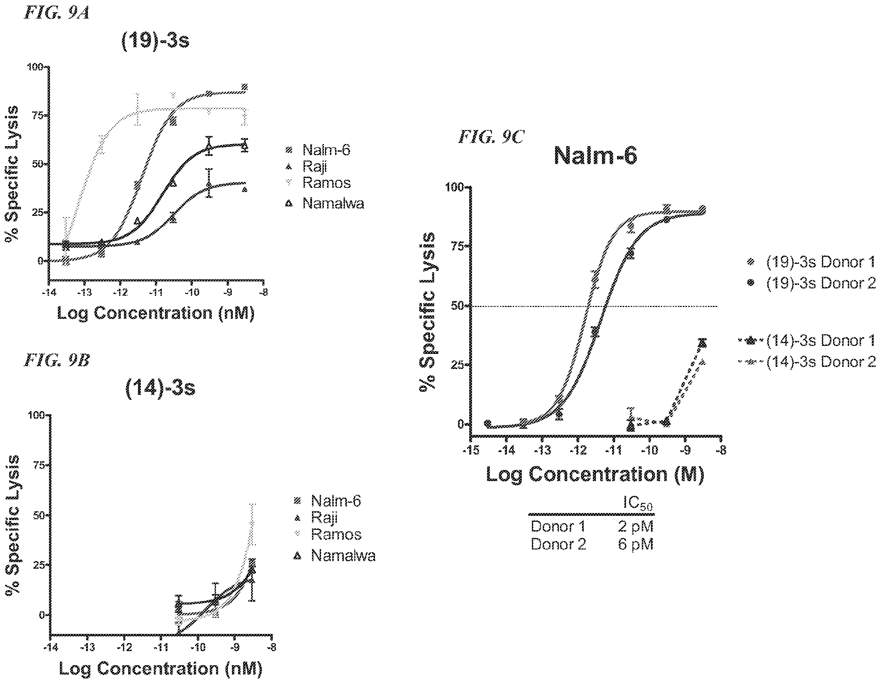

(X)-3s re-directed T-cell mediated killing of malignant cell lines. The cytotoxicity of each leukocyte targeting molecule was evaluated by its ability to mediate lysis of specific tumor target cells. For the hematologic tumor cell lines, a 10:1 E:T ratio using an unstimulated, enriched T cell population as the effector cells in an 18 - 24 hour assay demonstrated the optimal assay conditions. The CD19-targeting bsAb, (19)-3s induced the most potent specific killing of the relatively low CD19-expressing cell lines Ramos (IC₅₀ = 0.17 pM, Lysis_{Max} = 79%) Daudi (IC₅₀ = 1 pM, Lysis_{Max} = 60%), and Nalm6 (IC₅₀ = 6 pM, Lysis_{Max} = 93%) (**FIG. 9A**). Interestingly, the high CD19-expressing cell lines, Namalwa (IC₅₀ = 63 pM, Lysis_{Max} = 60%) and Raji (IC₅₀ = 3 nM, Lysis_{Max} = 41%) were the least sensitive to (19)-3s (**FIG. 9A**). The non-targeting (14)-3s DNL^{™} construct had little cytotoxic effect in any of the cell lines tested (**FIG. 9B**). Consistent cytotoxic effects of the (19)-3s construct on the Nalm-6 ALL cell line were obtained with PBMCs obtained from two different donors (**FIG. 9C**).

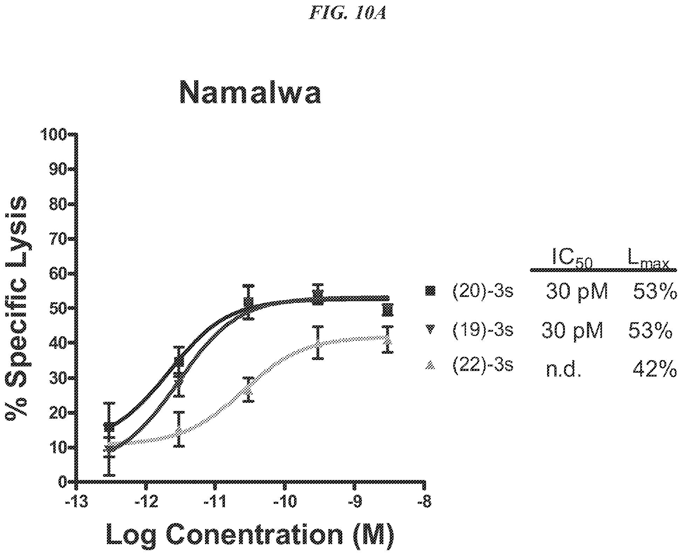

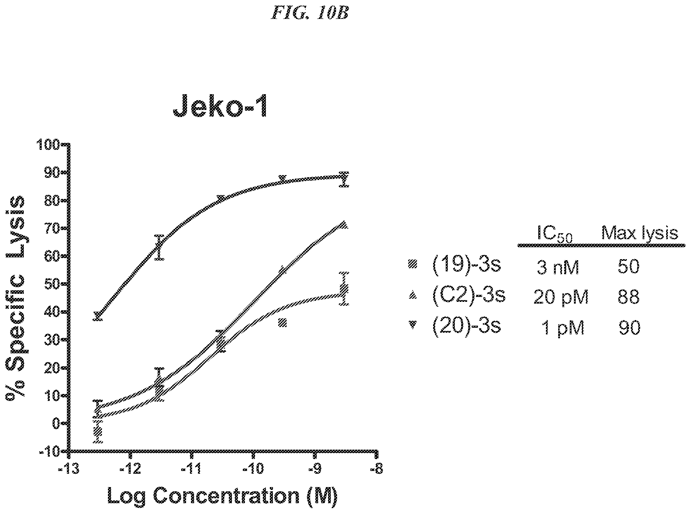

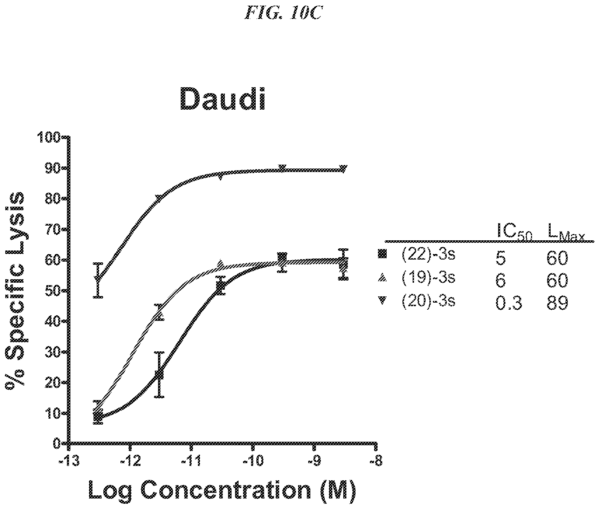

The *in vitro* cytotoxic effects of (20)-3 s, (22)-3s and (C2)-3s T-cell redirecting bsAbs were determined in several cell lines (**FIG. 10**). The CD22-targeting bsAb, (22)-3s, demonstrated potent (IC₅₀ = 5 pM, Lysis_{Max} = 60%) specific T-cell mediated lysis in the CD22-positive Daudi cell line (**FIG. 10C**), but not in the CD22-negative Namalwa cells (**FIG. 10A**).

The CD20-targeting bsAb, (20)-3s demonstrated the highest potency in the higher-expressing CD20 cell lines, Daudi (IC₅₀ = <0.3 pM, Lysis_{Max} = 90%) (**FIG. 10C**) and Jeko (IC₅₀ = 1 pM, Lysis_{Max} = 90%) (**FIG. 10B**), compared to the lower CD20-expressing Namalwa cell line (IC₅₀ = 30 pM, Lysis_{Max} = 53%) (**FIG. 10A**).

The HLA-DR-targeting bsAb, (C2)-3s was tested in the HLA-DR expressing Jeko-1 cell line (IC₅₀ = 20 pM, Lysis_{Max} = 88%) (**FIG. 10B**).

At an E:T ratio of 10:1, using isolated T cells as effector cells, the bsAbs induced potent T cell-mediated cytotoxicity in various B cell malignancies, including Burkitt lymphoma (Daudi, Ramos, Namalwa) mantle cell lymphoma (Jeko-1) and acute lymphoblastic leukemia (Nalm-6) (**Table 7**). A non-tumor binding control, (14)-3s, induced only moderate T-cell killing at >10 nM. The nature of the antigen/epitope, particularly its size and proximity to the cell surface, appears to be more important than antigen density for T-cell retargeting potency (**Table 7**). It is likely that (20)-3s is consistently more potent than (19)-3s and (C2)-3s, even when the expression of CD19 or HLA-DR is considerably higher than CD20, as seen with Namalwa and Jeko-1, respectively (**Table 7**). This is likely because the CD20 epitope comprises a small extracellular loop having close proximity to the cell surface. When compared directly using Daudi, (22)-3s was the least potent. Compared to CD19 and CD20, CD22 is expressed at the lowest density, is a rapidly internalizing antigen, and its epitope is further away from the cell surface. Each of these factors may contribute to its reduced potency. Finally, sensitivity to T-cell retargeted killing is cell-line-dependent, as observed using (19)-3s, where Raji (IC₅₀ >3 nM) is largely unresponsive yet Ramos (IC₅₀ = 2 pM) is highly sensitive, even though the former expresses higher CD19 antigen density (**Table 7**).

In conclusion, (19)-3s, (20)-3s, (22)-3s and (C2)-3s bind to T cells and target B cells simultaneously and induce T-cell-mediated killing *in vitro.* The modular nature of the DNL method allowed the rapid production of several related conjugates for redirected leukocyte killing of various B cell malignancies, without the need for additional recombinant engineering and protein production. The close proximity of the CD20 extracellular epitope to the cell surface resulted in the highest potency for (20)-3s.

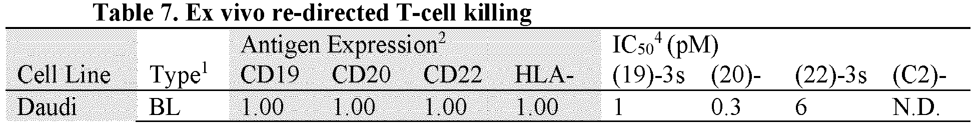

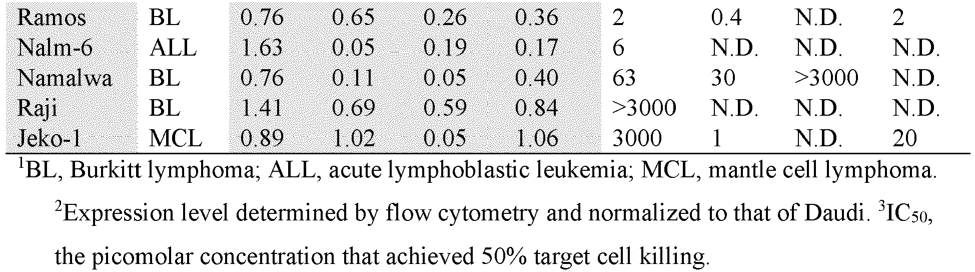

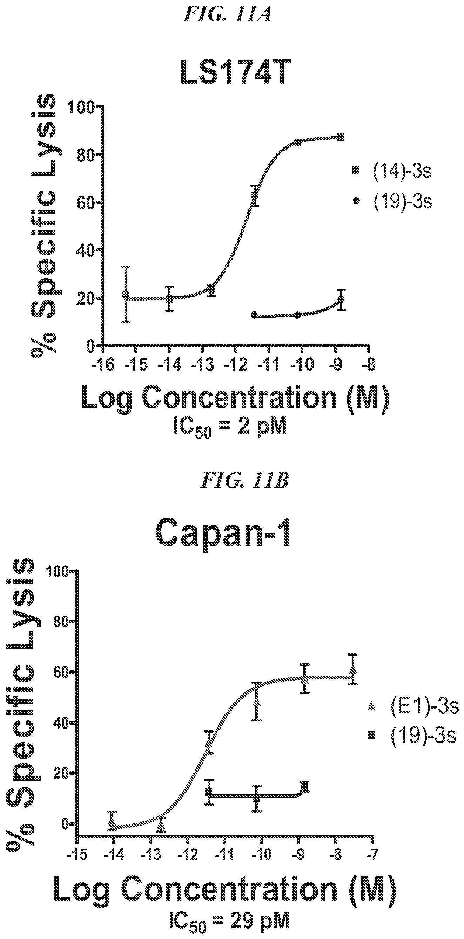

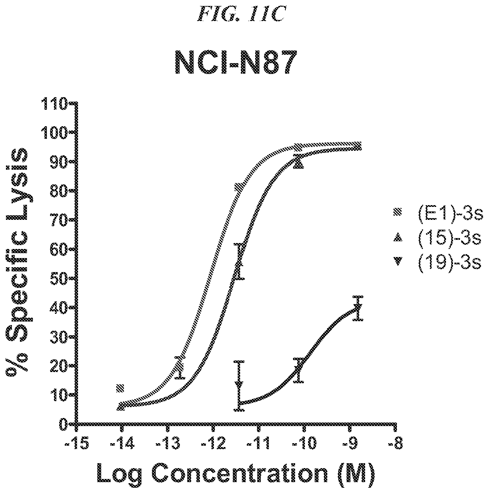

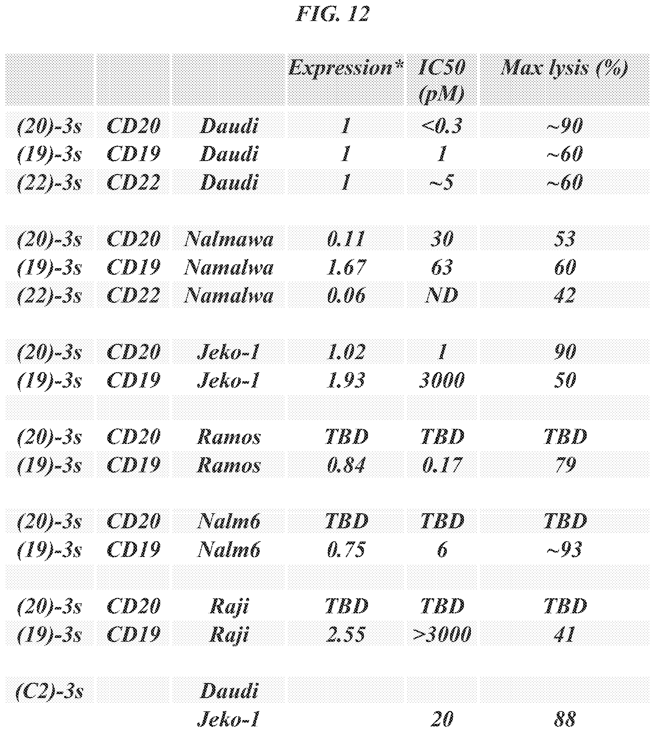

The *in vitro* cytotoxic effects of leukocyte redirecting bsAbs were also determined in solid tumor cells (**FIG. 11**). For the solid tumor cell lines, optimal assay conditions were determined to be a 3:1 E:T ratio using stimulated T cells in a 42 - 48 hour assay. Each bsAb induced specific T-cell mediated lysis of the tumor target cells. The CEACAM5-expressing human colon adenocarcinoma cell line, LS-174T, demonstrated potent specific lysis (IC₅₀ = 2 pM) following treatment with (14)-3s (**FIG. 11A**). (E1)-3s mediated potent specific lysis of the TROP2 expressing Capan-1 human pancreatic adenocarcinoma cell line (IC₅₀ = 29 pM) (**FIG. 11B**). The gastric carcinoma cell line NCI-N87, which expresses high levels of both CEACAM6 and TROP 2 demonstrated very potent specific lysis to both T-cell targeting molecules, (15)-3s and (E1)-3s (IC₅₀ = 3 pM and 0.85 pM respectively) (**FIG. 11C**). The non-targeting control antibody, (19)-3s, induced low (<20%) non-specific lysis at concentrations > 1 nM for Capan-1 and LS174T, and moderate (~40%) non-specific lysis in NCI-N87 cells (FIG. 11A-C). A summary of the *in vitro* cytotoxicity data for various leukocyte redirecting bsAbs in a variety of tumor cell lines is shown in **FIG. 12**. The various constructs showed a maximal cell lysis of up to 90% or more of the targeted tumor cells, with IC₅₀ values for cell lines expressing the targeted antiben that were generally in the low picomolar range (**FIG. 12**).

### Example 2. In vivo Studies of leukocyte Redirecting DNL^{™} Complex

One potential limitation of small (<60 kDa) scFv-based constructs, such as BITE^{®} and DART^{™}, is the requirement for administration by long-term continuous infusion, due to their toxicity and rapid clearance from circulation. Because the molecular size of DNL^{™} bsAbs is above the threshold typically associated with renal clearance, it should exhibit slower clearance from circulation. We measured the pharmacokinetic parameters in mice following a single bolus i.v. injection of 5 mg/kg of the (19)-3s bsAb (data not shown). A biphasic clearance was observed with a t½α and t½β of 1.1 and 5.1 h, respectively, resulting in an area under the curve of 1880 pmol*h/mL (data not shown), which was nearly 6-fold greater than that reported for MT 103 (anti-CD19xanti-CD3 BITE^{®}) administered at the same molar concentration (US Patent US2010/0303827A1). The major difference is apparently a longer t½α for (19)-3s (data not shown). Because of the potentially advantageous properties of (19)-3s, we evaluated the possibility of using less frequent dosing schedules rather than daily dosing, which is typically used for BITE^{®} in animal studies.

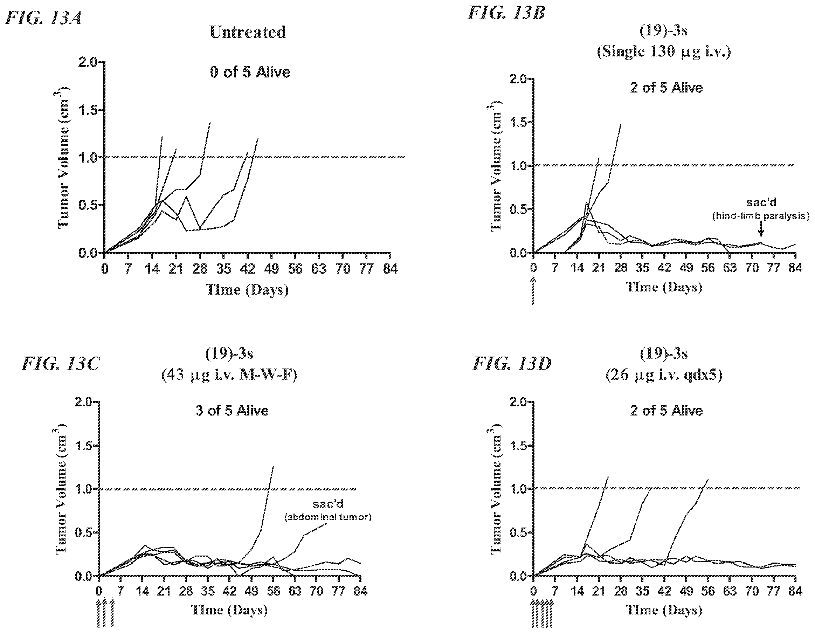

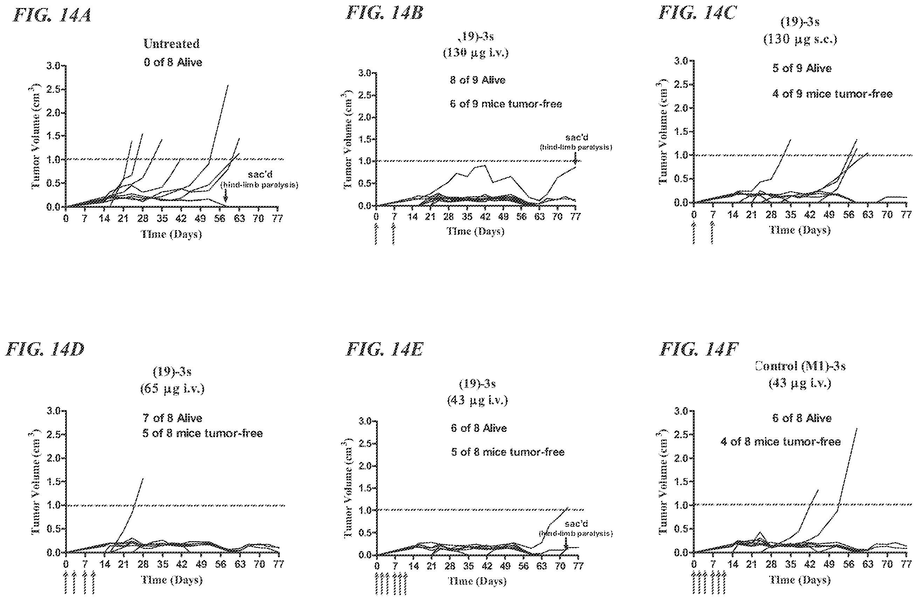

A pilot study was performed using Raji human Burkitt lymphoma xenografts in NOD/SCID mice reconstituted with human PBMCs **(****FIG. 13**, **FIG. 14****).** Raji cells (1 × 10⁶ cells/mouse) were combined with freshly isolated PBMCs (5 × 10⁶ cells/mouse) from a single healthy donor, mixed 1:1 with matrigel, and injected SC into all of the animals in the study on Day 0. Groups of 5 mice received i.v. injections of (19)-3s totaling 130 µg as a single dose on Day 0 (**FIG. 13B**), three doses of 43 µg (Days 0, 2 and 4) (**FIG. 13C**) or five daily doses of 26 µg (Days 0 - 5) (**FIG. 13D**). The untreated group (**FIG. 13A**), which was inoculated with the same cell mixture but did not receive (19)-3s, had a median survival time (MST) of 31 days. Each therapy regimen improved survival (*P* ≤ 0.05), with the three dose (every other day) schedule providing the greatest survival benefit (MST = 91 days; *P* = 0.0018 by log-rank analysis).

A follow-up study was begun to determine the efficacy of less frequent dosing (**FIG. 14**). Groups of 9 NOD/SCID mice were inoculated with Raji and PBMCs in a similar fashion as above. In this study, therapy was extended to two weeks, compared to one week in the first study. Groups received i.v. injections of (19)-3s totaling 360 µg as 2 × 130µg (**FIG. 14B**), 4 × 65-µg (**FIG. 14D**) or 6 × 43-µg doses over two weeks (**FIG. 14E**). An additional group was administered 2 × 130-µg doses SC, instead of i.v. (**FIG. 14C**). For comparison, control groups of untreated mice (**FIG. 14A**) or mice treated with non-targeting (M1)-3s antibody (**FIG. 14F**) were prepared. As of Day 28, each of the (19)-3s treatment groups had significantly smaller AUC than the untreated control (*P* < 0.05). Surprisingly, two weekly doses via the SC route was apparently as effective as greater frequency i.v. dosing.

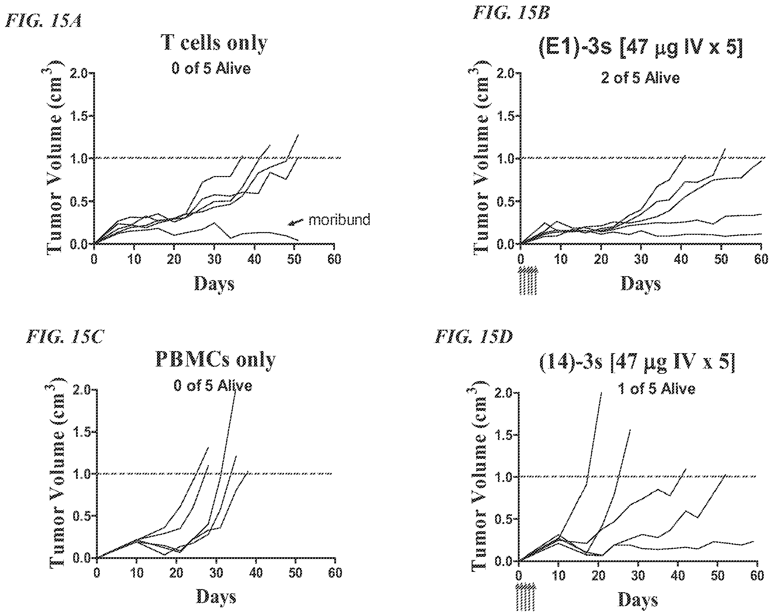

*In vivo* studies were also performed using solid tumors (**FIG. 15**). NOD/SCID mouse xenografts were prepared as described above, for the LS174T colon adenocarcinoma (**FIG. 15A, FIG. 15B**) or Capan-1 pancreatic carcinoma (**FIG. 15C, FIG. 15D**). In each case, mice administered the targeting (E1)-3s (**FIG. 15B**) or (14)-3s (**FIG. 15D**) bsAb DNL^{™} constructs showed improved survival compared to controls.

In conclusion, the leukocyte-retargeting bsAbs, including (19)-3s, (E1)-3s and (M1)-3s DNL^{™} constructs, mediated synapse formation between T cells and B cells, colon adenocarcinoma or pancreatic carcinoma cells, respectively, via monovalent and bivalent binding to CD3 and CD19, respectively. T-cell activation, proliferation and target cell killing were induced by the DNL^{™} bsAbs at pM concentrations in an *ex vivo* setting. Advantageous properties of the DNL^{™} bsAbs, including bivalent tumor binding and slower clearance, would allow for less frequent dosing and possibly SC administration, compared to BITE^{®} or DART^{™} constructs, which are administered i.v. daily in animal models and as a continuous infusion in the clinic. The modular nature of the DNL^{™} method allows the rapid production of a large number of related conjugates for redirected leukocyte killing of various malignancies, without the need for additional recombinant engineering and protein production.

The person of ordinary skill in the art will realize that other antibodies that bind to CD3 or other leukocyte antigens, as well as other antibodies that bind to Trop-2 or other disease-associated antigens are known in the art and any such antibody can be used to make F(ab)₂, scFv or other antibody fragments using techniques well known in the art. Such alternative antibodies or fragments thereof may be utilized in the instant methods and compositions. As discussed below, methods of making DOCK-AND-LOCK^{™} (DNL^{™}) complexes may be applied to incorporate any known antibodies or antibody fragments into a stable, physiologically active complex.

### Example 3. Interferon-α Enhances the Cytotoxic Effect of Anti-Trop-2 x anti-CD3 Bispecific Antibodies

The therapeutic efficacy of an anti-human Trop-2 × anti-human CD3 bispecific antibody ((E1)-3s), made from hRS7 and OKT3 as a DNL^{™} complex, was tested for its ability to delay tumor outgrowth of Capan-1 human pancreatic adenocarcinoma tumor cells when mixed with human T-cells and injected into mice. The effect of interferon-α (either in the form of E1*-2b or PEGASYS^{®}) when combined with this therapy was also evaluated.

### Methods

Five week-old female NOD/SCID mice were injected s.c. with a mixture of Capan-1 (5×10⁶) and human T-cells (2.5×10⁶ cells) mixed 1:1 with matrigel (E:T ratio of 1:2). There were six different treatment groups of 8 mice each. Treatment consisted of one group receiving 47 µg (E1)-3s i.v. every day for five days starting 1 hour after the administration of the Capan-1/T-cell mixture. Two groups were treated with equimolar amounts of IFN, one received the DNL molecule made from IFN-α2b-DDD2-CK-hRS7 IgG1 (E1*-2b; 2.5 µg s.c. weekly × 4 wks) while another received PEGASYS^{®} (Roche; 0.6 µg s.c. weekly × 4 wks). Two other groups received a combination of (E1)-3s *plus* E1*2b or (E1)*-*3s *plus* PEGASYS^{®}. The final group control group remained untreated. **Table 8** summarizes the various treatment groups.

**Table 8. Treatment Groups for (E1)-3s Therapy**

| (E1)-3s Therapy of a Human Pancreatic Carcinoma Xenograft (Capan-1) in NOD/SCID Mice | | | |
|---|---|---|---|
| Group | (N) | Amount Injected | Schedule |
| 1 | 8 | Untreated | N.A. |
| 2 | 8 | (E1)-3s (47 µg i.v.) | qdx5 |
| 3 | 8 | E1 *-2b (2.5 µg s.c.) | qwkx4 |
| 4 | 8 | PEGASYS^{®} (0.6 µg s.c.) | qwkx4 |
| 5 | 8 | (E1)-3s + E1 *-2b | qdx5 + qwkx4 |
| 6 | 8 | (E1)-3s + PEGASYS | qdx5 + qwkx4 |

Mice were monitored daily for signs of tumor out-growth. All animals had their tumors measured twice weekly once tumors began to come up. Mice were euthanized for disease progression if their tumor volumes exceeded 1.0 cm³ in size.

### Results

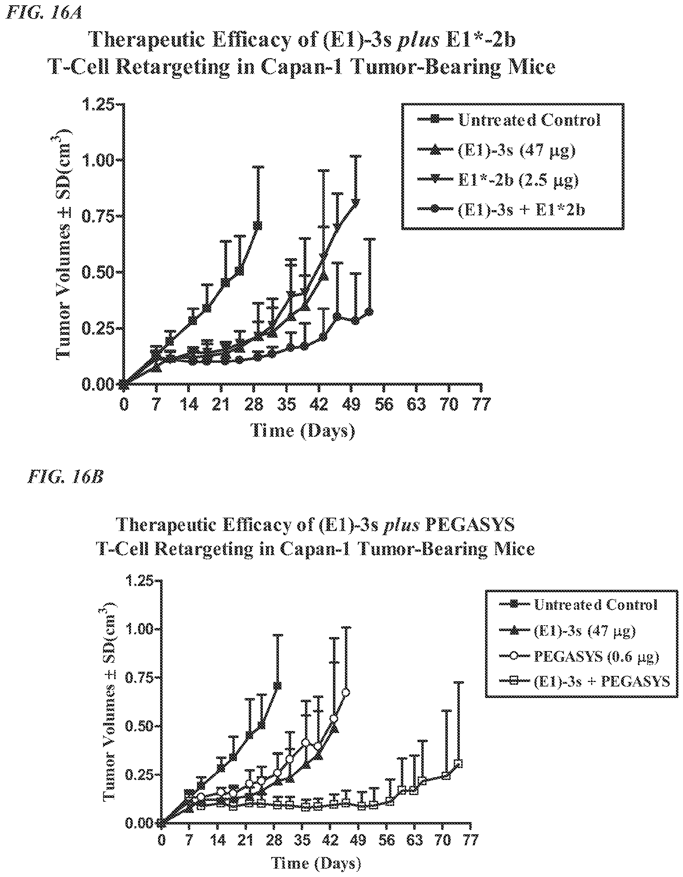

Mean tumor volumes for the various groups are shown in **FIG. 16**. The data containing PEGASYS^{®} groups (**FIG. 16B**) are shown on a separate graph from the E1*2b groups (**FIG. 16A**) for clarity. All treatments were significantly better at controlling tumor growth in terms of area-under-the-curve (AUC) when compared to the untreated mice out to day 29, which was when the first mouse in the untreated group was euthanized for disease progression (*P*<0.0009; AUC_{29 days}). Combining (E1)-3s with PEGASYS^{®} resulted in the best anti-tumor response overall in terms of tumor out-growth (**FIG. 16B**). This treatment was significantly better than any of the individual treatments (*P*<0.042; AUC) as well as superior to the combination of (E1)-3s *plus* E1*-2b (*P*=0.0312; AUC_{53 days}) (**FIG. 16A**). The combination of (E1)-3s *plus* E1*2b could significantly control tumor growth when compared to E1*2b or PEGASYS^{®} alone (*P*<0.0073; AUC_{46 days}) but not (E1)-3s alone (FIG. 16A-B). There were no significant differences between mice treated with (E1)-3s, PEGASYS^{®}, or E1*-2b (FIG. 16A-B).

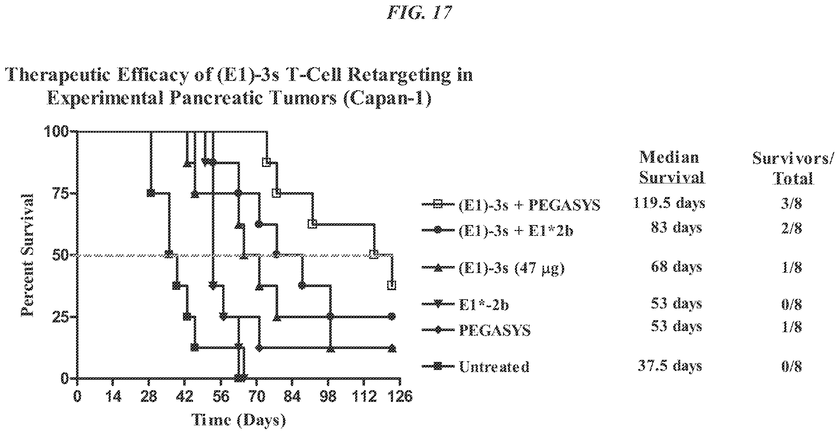

In terms of survival, all treatments provide a significant survival benefit when compared to the untreated mice (*P*<0.0112; log-rank) (**FIG. 17**). As of day 81, there was no significant difference in median survival times (MST) between mice treated with the combination of (E1)-3s*plus* E1*-2b and those treated (E1)-3s *plus* PEGASYS^{®} (MST=79.5 and >81 days, respectively) (**FIG. 17**). The mice treated with (E1)-3s *plus* PEGASYS^{®} had a significantly improved survival outcome than any of the individual treatments (P<0.0237) (**FIG. 17**). Mice treated with (E1)-3s *plus* E1*2b had a survival benefit when compared to mice treated with E1*-2b alone (MST=53 days; *P*<0.0311) but not when compared to mice treated with just (E1)-3s or PEGASYS^{®} alone (MST=68 and 53 days, respectively) (**FIG. 17**). Treatment with (E1)-3s provided a significant improvement in survival when compared to mice treated with E1*-2b (*P*=0.0406) but not when compared to mice treated with PEGASYS^{®} alone (**FIG. 17**). There was no significant differences between mice treated with only E1*2b and those treated with PEGASYS^{®} alone (**FIG. 17**).

The results demonstrate that addition of interferon-α provides a substantial increase in survival and decrease in tumor growth when combined with a leukocyte redirecting bsAb. The person of ordinary skill will realize that the improved efficacy observed with addition of type I or type III interferons (interferon-α, interferon-β, or interferon-λ) is not limited to the specific (E1)-3s bsAb, but will be observed with other leukocyte redirecting bsAbs, made either as DNL^{™} complexes or in other forms, such as BITE^{®} or DART^{™}.

### Example 4. Further Studies on Interferon-α Combination Therapy With Leukocyte-Redirecting Bispecific Antibodies

In the Example above, the combination of (E1)-3s *plus* PEGASYS^{®} proved to be a very effective treatment in the control of tumor growth. In order to confirm these results and extend them, a study was performed in which two new groups were added. First, a control group for (E1)-3s was included in which an equimolar amount of TF12 was administered to animals. TF12 consists of two hRS7-Fab molecules linked to one non-targeting 679 Fab (anti-HSG). Additionally, since Capan-1 is sensitive to IFN, another group was added in which the effect of PEGASYS^{®} on Capan-1 tumor growth was assessed without the benefit of T cells.

After the mice (40) were injected with the Capan-1/T-cell mixture, they were randomized into five treatment groups. One hour later, one group of 11 mice received 47 µg (E1)-3s i.v. every day starting 1 h post-tumor cell injection and continued for four more consecutive days (qdx5). One group of 7 animals received interferon in the form of PEGASYS^{®} s.c. on a weekly basis for four weeks. Another group received a combination of (E1)-3s i.v. *plus* PEGASYS^{®} s.c. Untreated control animals receive Capan-1/T cells but no treatment. A further control group received TF12 at amounts equivalent to the (E1)-3s in terms of moles (57 µg qdx5). Group 6 mice (8 animals) received a separate injection of only Capan-1 cells (i.e., no T cells) and was treated with PEGASYS^{®}. All therapy injections were in a volume of 100 µL. **Table 9** summarizes the various groups

**Table 9. Treatment Groups for (E1)-3s and TF12 Therapy**

| (E1)-3s Therapy of a Human Pancreatic Carcinoma Xenograft (Capan-1) in NOD/SCID Mice | | | |
|---|---|---|---|
| Group | (N) | Amount Injected | Schedule |
| 1 | 7 | Untreated (Capan-1 + T cells only) | N.A. |
| 2 | 11 | (E1)-3s (47 µg i.v.) | qdx5 |
| 3 | 7 | TF12 (57 µg i.v.) | qdx5 |
| 4 | 7 | PEGASYS^{®} (0.6 µg s.c.) | qwkx4 |
| 5 | 8 | (E1)-3s + PEGASYS^{®} | qdx5 + qwkx4 |
| 6 | 8 | PEGASYS^{®} (0.6 µg s.c.) | qwkx4 |
| | | (Capan-1 cells only) | |

Mice were monitored daily for signs of tumor out-growth. All animals had their tumors measured twice weekly once tumors began to come up. Mice were euthanized for disease progression if their tumor volumes exceeded 1.0 cm³ in size.

### Results

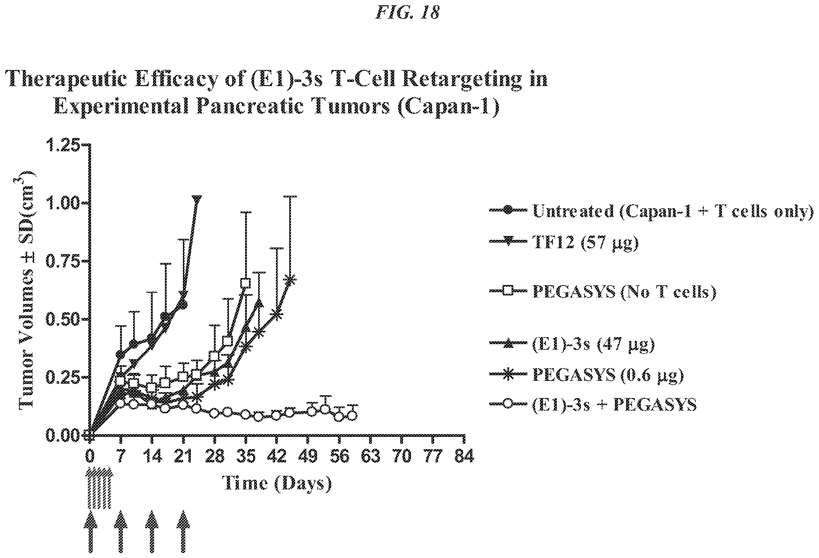

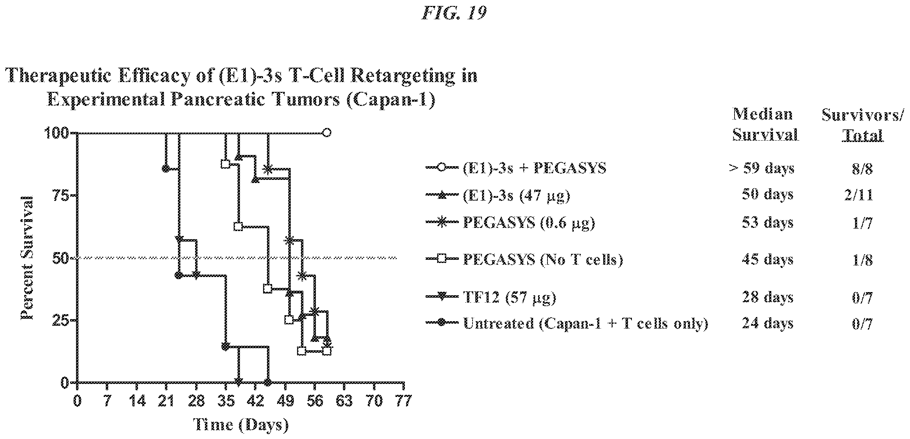

Mean tumor growth (**FIG. 18**) and survival curves (**FIG. 19**) are shown. While not different from each other, mice treated with (E1)-3s, PEGASYS^{®}, or PEGASYS^{®} (without T cells), demonstrated significant anti-tumor effects when compared to TF12 and untreated control groups (*P*<0.0102; AUC). On the day this experiment ended (day 59), the mean tumor volume for the mice treated with the combination of (E1)-3s *plus* PEGASYS^{®} was 0.083 ± 0.048 cm³. Overall, this treatment group demonstrated a significant anti-tumor effect when compared to all the other treatment groups (*P*<0.0072; AUC).

Each individual treatment (PEGASYS^{®}, PEGASYS^{®} without T cells, and (E1)-3s) significantly improved survival in comparison to both the TF12 and untreated control groups (*P*<0.0059; log-rank) (**FIG. 18****,** **FIG. 19**). All the groups except the combination of (E1)-3s *plus* PEGASYS^{®} reached their respective MSTs. No animals were euthanized for disease progression (TV>1.0 cm³) in this combination group. Importantly, the combination of (E1)-3s *plus* PEGASYS^{®} provided a significant survival benefit when compared to all other treatments (*P*<0.0007; log-rank) (**FIG. 18****,** **FIG. 19**).

### Example 5. Effect of Interferon-α Combination Therapy With T-Cell-RedirectingBispecific Antibodies in Human Gastric Cancer

The methods and compositions disclosed in the preceding two Examples were used to study the effects of leukocyte redirecting bsAbs alone or in combination with interferon-α (PEGASYS^{®}) in the IFN-refractory NCI-N87 human gastric tumor line. Groups of mice (N=8 each group) were injected s.c. with 5×10⁶ NCI-N87 cells + 2.5×10⁶ T Cells (1:2 E:T ratio) mixed with matrigel and therapy started 1 h later. The treatment groups are shown in **Table 10.**

**Table 10. Treatment Groups for (E1)-3s and TF12 Therapy**

| (E1)-3s Therapy of a Human Gastric Carcinoma Xenograft (NCI-N87) in NOD-SCID Mice | | | |
|---|---|---|---|
| Group | Amount Injected | | Schedule |
| 1 | | **Untreated** (NCI-N87 + T cells only) | N.A. |
| 2 | | **(E1)-3s** (47 µg i.v.) | qdx5 |
| 3 | | **TF12** (57 µg i.v.) | qdx5 |
| 4 | | **PEGASYS^{®}** (0.6 µg s.c.) | qwkx4 |
| 5 | | **TF12 + PEGASYS^{®}** | qdx5 + qwkx4 |
| 6 | | **(E1)-3s** + **PEGASYS^{®}** | qdx5 + qwkx4 |

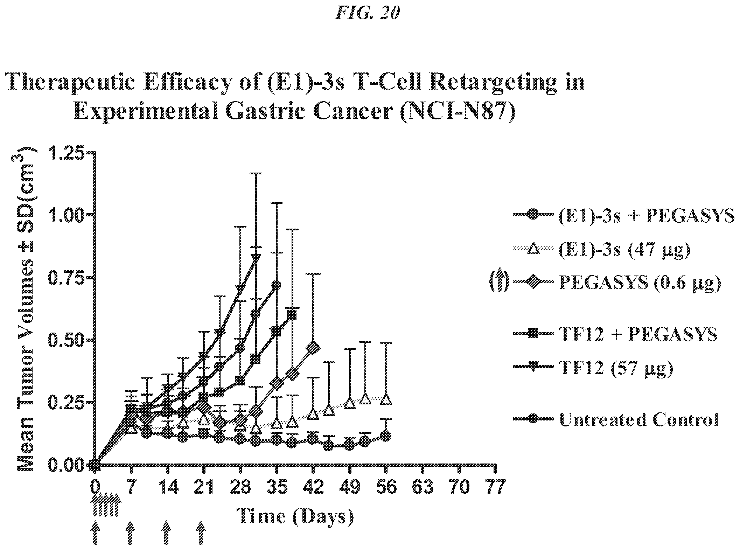

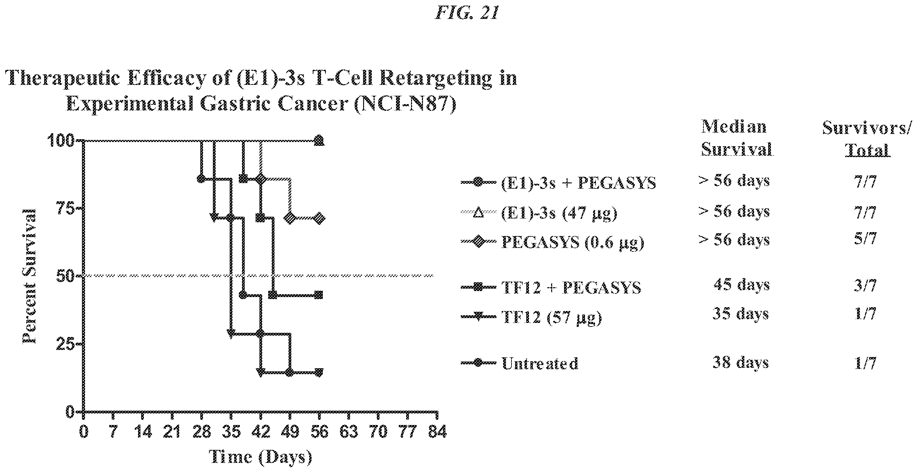

The effects of leukocyte redirecting bsAb (E1)-3s alone or in combination with interferon are shown in **FIG. 20** and **FIG. 21**. The (E1)-3s bsAb was effective to reduce tumor growth and increase survival in gastric cancer. Significantly, the combination with interferon-α enhanced the effect of leukocyte redirecting bsAb, even in an interferon resistant tumor. The combination therapy was more effective than either agent added alone. Controls with mice treated with TF12 bsAb alone or in combination with interferon-α showed little effect on tumor growth or mortality, compared to untreated animals.

### Example 6. In vivo Therapeutic Use of Antibody-Drug Conjugates (ADCs) in Preclinical Models of Human Pancreatic or Colon Carcinoma

CL2A-SN-38-antibody conjugates were prepared as previously described (see, e.g., U.S. Patent Nos. 7,999,083 and 8,080,250). Immune-compromised athymic nude mice (female), bearing subcutaneous human pancreatic or colon tumor xenografts were treated with either specific CL2A-SN-38 conjugate or control conjugate or were left untreated. The therapeutic efficacies of the specific conjugates were observed. In a Capan 1 pancreatic tumor model, specific CL2A-SN-38 conjugates of hRS7 (anti-TROP2), hPAM4 (anti-MUC5ac), and hMN-14 (anti-CEACAM5) antibodies showed better efficacies than control hA20-CL2A-SN-38 conjugate (anti-CD20) and untreated control (not shown). Similarly, in a BXPC3 model of human pancreatic cancer, the specific hRS7-CL2A-SN-38 showed better therapeutic efficacy than control treatments (not shown). Likewise, in an aggressive LS174T model of human colon carcinoma, treatment with specific hMN-14-CL2A-SN-38 was more efficacious than non-treatment (not shown).

### Example 7. In vivo therapy of lung metastases of GW-39 human colonic tumors in nude mice using ADC hMN-14-[CL2-SN-38], IMMU-130

A lung metastatic model of colonic carcinoma was established in nude mice by i.v. injection of GW-39 human colonic tumor suspension, and therapy was initiated 14 days later. Specific anti-CEACAM5 antibody conjugate, hMN14-CL2-SN-38, as well as nontargeting anti-CD22 MAb control conjugate, hLL2-CL2-SN-38 and equidose mixtures of hMN14 and SN-38 were injected at a dose schedule of q4d×8, using different doses. Selective therapeutic effects were observed with the hMN-14 ADC (not shown). At a dosage of 250 µg, the mice treated with hMN14-CL2-SN-38 showed a median survival of greater than 107 days. Mice treated with the control conjugated antibody hLL2-CL2-SN-38, which does not specifically target lung cancer cells, showed median survival of 77 days, while mice treated with unconjugated hMN14 IgG and free SN-38 showed a median survival of 45 days, comparable to the untreated saline control of 43.5 days. A significant and surprising increase in effectiveness of the conjugated, cancer cell targeted antibody-SN-38 conjugate, which was substantially more effective than unconjugated antibody and free chemotherapeutic agent alone, was clearly seen (not shown). The dose-responsiveness of therapeutic effect of conjugated antibody was also observed (not shown). These results demonstrate the clear superiority of the SN-38-antibody conjugate compared to the combined effect of both unconjugated antibody and free SN-38 in the same *in vivo* human lung cancer system.

### Example 8. Use of ADC (IMMU-132 or hRS7-SN-38) to Treat Therapy-Refractive Metastatic Colonic Cancer (mCRC)

The patient was a 62-year-old woman with mCRC who originally presented with metastatic disease in January 2012. She had laparoscopic ileal transverse colectomy as the first therapy a couple of weeks after diagnosis, and then received 4 cycles of FOLFOX (leucovorin, 5-fluorouracil, oxaliplatin) chemotherapy in a neoadjuvant setting prior to right hepatectomy in March 2012 for removal of metastatic lesions in the right lobe of the liver. This was followed by an adjuvant FOLFOX regimen that resumed in June, 2012, for a total of 12 cycles of FOLFOX. In August, oxaliplatin was dropped from the regimen due to worsening neurotoxicity. Her last cycle of 5-FU was on 09/25/12.

CT done in Jan 2013 showed metastases to liver. She was then assessed as a good candidate for enrollment to IMMU-132 (hRS7-SN-38) investigational study. Comorbidities in her medical history include asthma, diabetes mellitus, hypertension, hypercholesteremia, heart murmur, hiatal hernia, hypothyroidism, carpel tunnel syndrome, glaucoma, depression, restless leg syndrome, and neuropathy. Her surgical history includes tubo-ligation (1975), thyroidectomy (1983), cholescystectomy (2001), carpel tunnel release (2008), and glaucoma surgery.

At the time of entry into this therapy, her target lesion was a 3.1-cm tumor in the left lobe of the liver. Non-target lesions included several hypo-attenuated masses in the liver. Her baseline CEA was 781 ng/mL.

IMMU-132 was given on a once-weekly schedule by infusion for 2 consecutive weeks, then a rest of one week, this constituting a treatment cycle. These cycles were repeated as tolerated. The first infusion of IMMU-132 (8mg/kg) was started on Feb 15, 2013, and completed without notable events. She experienced nausea (Grade 2) and fatigue (Grade 2) during the course of the first cycle and has been continuing the treatment since then without major adverse events. She reported alopecia and constipation in March 2013. The first response assessment done (after 6 doses) on 04/08/2013 showed a shrinkage of target lesion by 29% by computed tomography (CT). Her CEA level decreased to 230 ng/mL on March 25, 2013. In the second response assessment (after 10 doses) on May 23, 2013, the target lesion shrank by 39%, thus constituting a partial response by RECIST criteria. She has been continuing treatment, receiving 6 cycles constituting 12 doses of hRS7-SN-38 (IMMU-132) at 8 mg/kg. Her overall health and clinical symptoms improved considerably since starting this investigational treatment.

### Example 9. ADC Therapy With IMMU-132 For Metastatic Solid Cancers

IMMU-132 is an ADC comprising the active metabolite of CPT-11, SN-38, conjugated by a pH-sensitive linker (average drug-antibody ratio = 7.6) to the hRS7 anti-Trop-2 humanized monoclonal antibody, which exhibits rapid internalization when bound to Trop-2. IMMU-132 targets Trop-2, a type I transmembrane protein expressed in high prevalence and specificity by many carcinomas. This Example reports a Phase I clinical trial of 25 patients with different metastatic cancers (pancreatic, 7; triple-negative breast [TNBC], 4; colorectal [CRC], 3; gastric, 3, esophageal, prostatic, ovarian, non-small-cell lung, small-cell lung [SCLC], renal, tonsillar, urinary bladder, 1 each) after failing a median of 3 prior treatments (some including topoisomerase-I and -II inhibiting drugs).

IMMU-132 was administered in repeated 21-day cycles, with each treatment given on days 1 and 8. Dosing started at 8 mg/kg/dose (i.e., 16 mg/kg/cycle), and escalated to 18 mg/kg before encountering dose-limiting neutropenia, in a 3+3 trial design. Fatigue, alopecia, and occasional mild to moderate diarrhea were some of the more common non-hematological toxicities, with 2 patients also reporting a rash. Over 80% of 24 assessable patients had stable disease or tumor shrinkage (SD and PR) among the various metastatic cancers as best response by CT. Three patients (CRC, TNBC, SCLC) have PRs by RECIST; median TTP for all patients, excluding those with pancreatic cancer, is >18 weeks. Neutropenia has been controlled by dose reduction to 8-10 mg/kg/dose (16-20 mg/kg/cycle).

Immunohistochemistry showed strong expression of Trop-2 in most archived patient tumors, but it is not detected in serum. Corresponding reductions in blood tumor marker titers (e.g., CEA, CA19-9) reflected tumor responses. No anti-antibody or anti-SN-38 antibodies have been detected despite repeated dosing. Peak and trough assessments of IMMU-132 concentrations in the serum show that the conjugate clears completely within 7 days, an expected finding based on *in vitro* studies showing 50% of the SN-38 is released in the serum every day. These results indicate that this novel ADC, given in doses ranging from 16-24 mg/kg per cycle, shows a high therapeutic index in diverse metastatic solid cancers.

### Example 10. IMMU-130, an SN-38 ADC That Targets CEACAM5, Is Therapeutically Active In Metastatic Colorectal Cancer (mCRC)

IMMU-130, an ADC of SN-38 conjugated by a pH-sensitive linker (7.6 average drug-antibody ratio) to the humanized anti-CEACAM5 antibody (labetuzumab), is completing two Phase I trials. In both, eligible patients with advanced mCRC were required to have failed/relapsed standard treatments, one being the topoisomerase-I inhibiting drug, CPT-11 (irinotecan), and an elevated plasma CEA (>5 ng/mL).

IMMU-130 was administered every 14 days (EOW) at doses starting from 2.0 mg/kg in the first protocol (IMMU-130-01). Febrile neutropenia occurred in 2 of 3 patients at 24 mg/kg; otherwise at ≤16 mg/kg, neutropenia (≥ Grade 2) was observed in 7 patients, with one also experiencing thrombocytopenia. One patient [of 8 who received ≥ 4 doses (2 cycles)] showed a 40.6% decrease in liver (starting at 7 cm) and lung target lesions (PR by RECIST) for 4.7 months, with no major toxicity, tolerating a total of 18 doses at 16 mg/kg. The study is continuing at 12 mg/kg EOW.

Since SN-38 is most effective in S-phase cells, a more protracted exposure could improve efficacy. Thus, in a second Phase I trial (IMMU-130-02), dosing was intensified to twice-weekly, starting at 6 mg/kg/dose for 2 weeks (4 doses) with 1 week off, as a treatment cycle, in a 3+3 trial design. Neutropenia and manageable diarrhea were the major side effects, until dose reduction to 4.0 mg/kg twice-weekly, with early results indicating multiple cycles are well-tolerated. Currently, tumor shrinkage occurred in 3 patients, with 1 in continuing PR (-46%) by RECIST, among 6 patients who completed ≥4 doses (1 cycle). In both trials, CEA blood titers correlated with tumor response, and high levels did not interfere with therapy. There have been no anti-antibody or anti-SN-38 antibody reactions, based on ELISA tests. In each study, the ADC was cleared by 50% within the first 24 h, which is much longer exposure than with typical doses of the parental molecule, CPT-11. These results indicate that this novel ADC, given in different regimens averaging ~16-24 mg/kg/cycle, shows a high therapeutic index in advanced mCRC patients. Since CEACAM5 has elevated expression in breast and lung cancers, as well as other epithelial tumors, it may be a useful target in other cancers as well.

### Example 11. Antitumor Activity of Checkpoint Inhibitor Antibody Alone or Combined with Anti-Trop-2 × anti-CD3 bsAb, IFN-α or Anti-Trop-2 ADC

To determine if the antitumor activity of the exemplary checkpoint inhibitor antibody, ipilimumab (anti-CTLA4) is synergistic with or inhibited by the addition of other therapeutic agents, CTLA4 mAb is evaluated alone or in combination with the exemplary T-cell redirecting bsAb (E1)-3s, with interferon-α (PEGINTERFERON^{®}), or with the exemplary ADC hRS7-SN-38 (IMMU-132) in murine tumor models. M109 lung carcinoma, SA1N fibrosarcoma, and CT26 colon carcinoma models are chosen based on different sensitivity to the various agents and CTLA4 blockade. Human T cells are co-administered with the antibodies.

All compounds are tested at their optimal dose and schedule. When used in combination, CTLA4 mAb is initiated one day after the first dose of IMMU-132, (E1)-3s or interferon-α. Percent tumor growth inhibition and number of days to reach target tumor size are used to evaluate efficacy. Antitumor activity is scored as: complete regression (CR; non-palpable tumor) or partial regression (PR; 50% reduction in tumor volume). Synergy is defined as antitumor activity significantly superior (p<0.05) to the activity of monotherapy with each agent.

In the SA1N fibrosarcoma tumor model, which is sensitive to CTLA4 blockade and modestly sensitive to (E1)-3s, interferon-α, and IMMU-132, borderline synergy is evident with the combination of CTLA4 mAb and (E1)-3s, whereas no effect is observed with interferon-α. IMMU-132 monotherapy does not produce significant SA1N antitumor activity. However, combining IMMU-132 with CTLA4 mAb results in synergy. In the M109 lung metastasis model and CT26 colon carcinoma model, synergy is detected for CTLA4 mAb combined with each of IMMU-132, (E1)-3s and interferon-α.

In summary, addition of CTLA4 mAb to interferon-α, IMMU-132, or (E1)-3s results in model-dependent synergistic activities. Synergy is observed regardless of the immunogenicity of the tumor and only when at least one of the therapies is active. All combination regimens are well-tolerated and the combination therapies do not appear to inhibit CTLA4 mAb activity. Synergy is observed in tumors unresponsive to CTLA4 mAb alone, suggesting that the other therapeutic agents might induce immunogenic cell death.

### Example 12. Combination Therapy with Anti-Trop-2 ADC (IMMU-132) and Interferon-α (PEGINTERFERONO) to Treat Refractory, Metastatic, Non-Small Cell Lung Cancer

The patient is a 60-year-old man diagnosed with non-small cell lung cancer. The patient is given chemotherapy regimens of carboplatin, bevacizumab for 6 months and shows a response, and then after progressing, receives further courses of chemotherapy with carboplatin, etoposide, TAXOTERE^{®}, gemcitabine over the next 2 years, with occasional responses lasting no more than 2 months. The patient then presents with a left mediastinal mass measuring 6.5 × 4 cm and pleural effusion.

After signing informed consent, the patient is given IMMU-132 at a dose of 18 mg/kg every other week. After the first week of treatment, the patient is given combination therapy with IMMU-132 and PEGINTERFERON^{®}. During the first two injections, brief periods of neutropenia and diarrhea are experienced, with 4 bowel movements within 4 hours, but these resolve or respond to symptomatic medications within 2 days. After a total of 6 infusions of IMMU-132 and 5 infusions of PEGINTERFERON^{®}, CT evaluation of the index lesion shows a 22% reduction, just below a partial response but definite tumor shrinkage. The patient continues with this therapy for another two months, when a partial response of 45% tumor shrinkage of the sum of the diameters of the index lesion is noted by CT, thus constituting a partial response by RECIST criteria. The combination therapy appears to provide a synergistic response, compared to the two agents administered separately.

### Example 13. Combination Therapy with ADC (IMMU-130) and T-Cell Redirecting bsAb (MT100) to Treat Advanced Colonic Cancer

The patient is a 75-year-old woman initially diagnosed with metastatic colonic cancer (Stage IV). She has a right partial hemicolectomy and resection of her small intestine and then receives FOLFOX, FOLFOX + bevacizumab, FOLFIRI + ramucirumab, and FOLFIRI + cetuximab therapies for a year and a half, when she shows progression of disease, with spread of disease to the posterior cul-de-sac, omentum, with ascites in her pelvis and a pleural effusion on the right side of her chest cavity. Her baseline CEA titer just before this therapy is 15 ng/mL. She is given 6 mg/kg IMMU-130 (anti-CEACAM5-SN-38) twice weekly for 2 consecutive weeks, and then one week rest (3-week cycle). After the first cycle, the patient is given combination therapy with IMMU-132 and the leukocyte redirecting bsAb MT110, which is administered by continuous infusion on the same 3-week cycle. After 5 cycles, which are tolerated very well, without any major hematological or non-hematological toxicities, her plasma CEA titer shrinks modestly to 1.3 ng/mL, but at the 8-week evaluation she shows a 21% shrinkage of the index tumor lesions, which increases to a 27% shrinkage at 13 weeks. Surprisingly, the patient's ascites and pleural effusion both decrease (with the latter disappearing) at this time, thus improving the patient's overall status remarkably. The combination therapy appears to provide a synergistic response, compared to the two agents administered separately.

### Example 14. Combination Therapy With ADC (IMMU-130), Anti-Trop-2 × Anti-CD3 bsAb ((E1)-3s) and Interferon-α to Treat Gastric Cancer Patient with Stage IV Metastatic Disease

The patient is a 52-year-old male who sought medical attention because of gastric discomfort and pain related to eating for about 6 years, and with weight loss during the past 12 months. Palpation of the stomach area reveals a firm lump which is then gastroscoped, revealing an ulcerous mass at the lower part of his stomach. This is biopsied and diagnosed as a gastric adenocarcinoma. Laboratory testing reveals no specific abnormal changes, except that liver function tests, LDH, and CEA are elevated, the latter being 10.2 ng/mL. The patent then undergoes a total-body PET scan, which discloses, in addition to the gastric tumor, metastatic disease in the left axilla and in the right lobe of the liver (2 small metastases). The patient has his gastric tumor resected, and then has baseline CT measurements of his metastatic tumors. Four weeks after surgery, he receives 3 courses of combination chemotherapy consisting of a regimen of cisplatin and 5-fluorouracil (CF), but does not tolerate this well, so is switched to treatment with docetaxel. It appears that the disease is stabilized for about 4 months, based on CT scans, but then the patient's complaints of further weight loss, abdominal pain, loss of appetite, and extreme fatigue cause repeated CT studies, which show increase in size of the metastases by a sum of 20% and a suspicious lesion at the site of the original gastric resection.

The patient is then given experimental therapy with IMMU-130 (anti-CEACAM5-SN-38) on a weekly schedule of 8 mg/kg. After the first week, combination therapy with IMMU-130, (E1)-3s and interferon-α is initiated. The patient exhibits no evidence of diarrhea or neutropenia over the following 4 weeks. The patient then undergoes a CT study to measure his metastatic tumor sizes and to view the original area of gastric resection. The radiologist measures, according to RECIST criteria, a decrease of the sum of the metastatic lesions, compared to baseline prior to therapy, of 23%. There does not seem to be any clear lesion in the area of the original gastric resection. The patient's CEA titer at this time is 7.2 ng/mL, which is much reduced from the baseline value of 14.5 ng/mL. The patient continues on weekly combination therapy, and after a total of 13 infusions, his CT studies show that one liver metastasis has disappeared and the sum of all metastatic lesions is decreased by 41%, constituting a partial response by RECIST. The patient's general condition improves and he resumes his usual activities while continuing to receive maintenance therapy every third week. At the last measurement of blood CEA, the value is 4.8 ng/mL, which is within the normal range for a smoker, which is the case for this patient.

### Example 15. General Techniques for Dock-and-Lock^{™}

The general techniques discussed below may be used to generate DNL^{™} complexes with AD or DDD moieties attached to any antibodies or antigen-binding antibody fragments, using the disclosed methdods and compositions.

### Expression Vectors

The plasmid vector pdHL2 has been used to produce a number of antibodies and antibody-based constructs. See Gillies et al., J Immunol Methods (1989), 125:191-202; Losman et al., Cancer (Phila) (1997), 80:2660-6. The di-cistronic mammalian expression vector directs the synthesis of the heavy and light chains of IgG. The vector sequences are mostly identical for many different IgG-pdHL2 constructs, with the only differences existing in the variable domain (V_{H} and V_{L}) sequences. Using molecular biology tools known to those skilled in the art, these IgG expression vectors can be converted into Fab-DDD or Fab-AD expression vectors.

To generate Fab-DDD expression vectors, the coding sequences for the hinge, CH2 and CH3 domains of the heavy chain were replaced with a sequence encoding the first 4 residues of the hinge, a 14 residue linker and a DDD moiety, such as the first 44 residues of human RIIα (referred to as DDD1, SEQ ID NO:1). To generate Fab-AD expression vectors, the sequences for the hinge, CH2 and CH3 domains of IgG were replaced with a sequence encoding the first 4 residues of the hinge, a 15 residue linker and an AD moiety, such as a 17 residue synthetic AD called AKAP-*IS* (referred to as AD1, SEQ ID NO:3), which was generated using bioinformatics and peptide array technology and shown to bind RIIα dimers with a very high affinity (0.4 nM). See Alto, et al. Proc. Natl. Acad. Sci., U.S.A (2003), 100:4445-50. Two shuttle vectors were designed to facilitate the conversion of IgG-pdHL2 vectors to either Fab-DDD1 or Fab-AD1 expression vectors, as described below. Preparation of CH1

The CH1 domain was amplified by PCR using the pdHL2 plasmid vector as a template. The left PCR primer consisted of the upstream (5') end of the CH1 domain and a SacII restriction endonuclease site, which is 5' of the CH1 coding sequence. The right primer consisted of the sequence coding for the first 4 residues of the hinge (PKSC, SEQ ID NO:102) followed by four glycines and a serine, with the final two codons (GS) comprising a Barn HI restriction site. The 410 bp PCR amplimer was cloned into the PGEMT^{®} PCR cloning vector (PROMEGA^{®}, Inc.) and clones were screened for inserts in the T7 (5') orientation.

A duplex oligonucleotide was synthesized to code for the amino acid sequence of DDD 1 preceded by 11 residues of the linker peptide, with the first two codons comprising a BamHI restriction site. A stop codon and an EagI restriction site are appended to the 3'end. The encoded polypeptide sequence is shown below.
GSGGGGSGGGGSHIQIPPGLTELLQGYTVEVLRQQPPDLVEFAVEYFTRLREARA (SEQ ID NO:103)

Two oligonucleotides, designated RIIA1-44 top and RIIA1-44 bottom, which overlap by 30 base pairs on their 3' ends, were synthesized and combined to comprise the central 154 base pairs of the 174 bp DDD 1 sequence. The oligonucleotides were annealed and subjected to a primer extension reaction with Taq polymerase. Following primer extension, the duplex was amplified by PCR. The amplimer was cloned into PGEMT^{®} and screened for inserts in the T7 (5') orientation.

A duplex oligonucleotide was synthesized to code for the amino acid sequence of AD1 preceded by 11 residues of the linker peptide with the first two codons comprising a BamHI restriction site. A stop codon and an EagI restriction site are appended to the 3'end. The encoded polypeptide sequence is shown below.
GSGGGGSGGGGSQIEYLAKQIVDNAIQQA (SEQ ID NO:104)

Two complimentary overlapping oligonucleotides encoding the above peptide sequence, designated AKAP-IS Top and AKAP-IS Bottom, were synthesized and annealed. The duplex was amplified by PCR. The amplimer was cloned into the PGEMT^{®} vector and screened for inserts in the T7 (5') orientation.

### Ligating DDD1 with CH1

A 190 bp fragment encoding the DDD1 sequence was excised from PGEMT^{®} with BamHI and NotI restriction enzymes and then ligated into the same sites in CH1-PGEMT^{®} to generate the shuttle vector CH1-DDD1-PGEMT^{®}.

### Ligating AD1 with CH1

A 110 bp fragment containing the AD1 sequence was excised from PGEMT^{®} with BamHI and NotI and then ligated into the same sites in CH1-PGEMT^{®} to generate the shuttle vector CH1-AD1-PGEMT^{®}.

With this modular design either CH1-DDD1 or CH1-AD1 can be incorporated into any IgG construct in the pdHL2 vector. The entire heavy chain constant domain is replaced with one of the above constructs by removing the SacII/EagI restriction fragment (CH1-CH3) from pdHL2 and replacing it with the SacII/EagI fragment of CH1-DDD1 or CH1-AD1, which is excised from the respective PGEMT^{®} shuttle vector.

### C-DDD2-Fd-hMN-14-pdHL2

C-DDD2-Fd-hMN-14-pdHL2 is an expression vector for production of C-DDD2-Fab-hMN-14, which possesses a dimerization and docking domain sequence of DDD2 (SEQ ID NO:2) appended to the carboxyl terminus of the Fd of hMN-14 via a 14 amino acid residue Gly/Ser peptide linker. The fusion protein secreted is composed of two identical copies of hMN-14 Fab held together by non-covalent interaction of the DDD2 domains.

The expression vector was engineered as follows. Two overlapping, complimentary oligonucleotides, which comprise the coding sequence for part of the linker peptide and residues 1-13 of DDD2, were made synthetically. The oligonucleotides were annealed and phosphorylated with T4 PNK, resulting in overhangs on the 5' and 3' ends that are compatible for ligation with DNA digested with the restriction endonucleases BamHI and PstI, respectively.

The duplex DNA was ligated with the shuttle vector CH1-DDD1-PGEMT^{®}, which was prepared by digestion with BamHI and PstI, to generate the shuttle vector CH1-DDD2-PGEMT^{®}. A 507 bp fragment was excised from CH1-DDD2-PGEMT^{®} with SacII and EagI and ligated with the IgG expression vector hMN-14(I)-pdHL2, which was prepared by digestion with SacII and EagI. The final expression construct was designated C-DDD2-Fd-hMN-14-pdHL2. Similar techniques have been utilized to generated DDD2-fusion proteins of the Fab fragments of a number of different humanized antibodies.

### h679-Fd-AD2-pdHL2

h679-Fab-AD2, was designed to pair to C-DDD2-Fab-hMN-14. h679-Fd-AD2-pdHL2 is an expression vector for the production of h679-Fab-AD2, which possesses an anchoring domain sequence of AD2 (SEQ ID NO:4) appended to the carboxyl terminal end of the CH1 domain via a 14 amino acid residue Gly/Ser peptide linker. AD2 has one cysteine residue preceding and another one following the anchor domain sequence of AD1.

The expression vector was engineered as follows. Two overlapping, complimentary oligonucleotides (AD2 Top and AD2 Bottom), which comprise the coding sequence for AD2 and part of the linker sequence, were made synthetically. The oligonucleotides were annealed and phosphorylated with T4 PNK, resulting in overhangs on the 5' and 3' ends that are compatible for ligation with DNA digested with the restriction endonucleases BamHI and SpeI, respectively.

The duplex DNA was ligated into the shuttle vector CH1-AD1-PGEMT^{®}, which was prepared by digestion with BamHI and SpeI, to generate the shuttle vector CH1-AD2-PGEMT^{®}. A 429 base pair fragment containing CH1 and AD2 coding sequences was excised from the shuttle vector with SacII and EagI restriction enzymes and ligated into h679-pdHL2 vector that prepared by digestion with those same enzymes. The final expression vector is h679-Fd-AD2-pdHL2.

### Generation of TF2 DNL^{™} Construct

A trimeric DNL^{™} construct designated TF2 was obtained by reacting C-DDD2-Fab-hMN-14 with h679-Fab-AD2. A pilot batch of TF2 was generated with >90% yield as follows. Protein L-purified C-DDD2-Fab-hMN-14 (200 mg) was mixed with h679-Fab-AD2 (60 mg) at a 1.4:1 molar ratio. The total protein concentration was 1.5 mg/ml in PBS containing 1 mM EDTA. Subsequent steps involved TCEP reduction, HIC chromatography, DMSO oxidation, and IMP 291 affinity chromatography. Before the addition of TCEP, SE-HPLC did not show any evidence of a₂b formation. Addition of 5 mM TCEP rapidly resulted in the formation of a₂b complex consistent with a 157 kDa protein expected for the binary structure. TF2 was purified to near homogeneity by IMP 291 affinity chromatography (not shown). IMP 291 is a synthetic peptide containing the HSG hapten to which the 679 Fab binds (Rossi et al., 2005, Clin Cancer Res 11:7122s-29s). SE-HPLC analysis of the IMP 291 unbound fraction demonstrated the removal of a₄, a₂ and free kappa chains from the product (not shown).

The functionality of TF2 was determined by BIACORE^{®} assay. TF2, C-DDD1-hMN-14+h679-AD1 (used as a control sample of noncovalent a₂b complex), or C-DDD2-hMN-14+h679-AD2 (used as a control sample of unreduced a₂ and b components) were diluted to 1 µg/ml (total protein) and passed over a sensorchip immobilized with HSG. The response for TF2 was approximately two-fold that of the two control samples, indicating that only the h679-Fab-AD component in the control samples would bind to and remain on the sensorchip. Subsequent injections of WI2 IgG, an anti-idiotype antibody for hMN-14, demonstrated that only TF2 had a DDD-Fab-hMN-14 component that was tightly associated with h679-Fab-AD as indicated by an additional signal response. The additional increase of response units resulting from the binding of WI2 to TF2 immobilized on the sensorchip corresponded to two fully functional binding sites, each contributed by one subunit of C-DDD2-Fab-hMN-14. This was confirmed by the ability of TF2 to bind two Fab fragments of WI2 (not shown).

### Production of TF10 DNL^{™} Construct

A similar protocol was used to generate a trimeric TF10 DNL^{™} construct, comprising two copies of a C-DDD2-Fab-hPAM4 and one copy of C-AD2-Fab-679. The TF10 bispecific ([hPAM4]₂ × h679) antibody was produced using the method disclosed for production of the (anti CEA)₂ × anti HSG bsAb TF2, as described above. The TF10 construct bears two humanized PAM4 Fabs and one humanized 679 Fab.

The two fusion proteins (hPAM4-DDD2 and h679-AD2) were expressed independently in stably transfected myeloma cells. The tissue culture supernatant fluids were combined, resulting in a two-fold molar excess of hPAM4-DDD2. The reaction mixture was incubated at room temperature for 24 hours under mild reducing conditions using 1 mM reduced glutathione. Following reduction, the reaction was completed by mild oxidation using 2 mM oxidized glutathione. TF10 was isolated by affinity chromatography using IMP291-affigel resin, which binds with high specificity to the h679 Fab.

### Example 16. Production of AD- and DDD-linked Fab and IgG Fusion Proteins from Multiple Antibodies

Using the techniques described in the preceding Example, the IgG and Fab fusion proteins shown in **Table 11** were constructed and incorporated into DNL^{™} constructs. The fusion proteins retained the antigen-binding characteristics of the parent antibodies and the DNL^{™} constructs exhibited the antigen-binding activities of the incorporated antibodies or antibody fragments.

**Table 11. Fusion proteins comprising IgG or Fab**

| **Fusion Protein** | **Binding Specificity** |
|---|---|
| **C-AD1-Fab-h679** | **HSG** |
| **C-AD2-Fab-h679** | **HSG** |
| **C-(AD)₂-Fab-h679** | **HSG** |
| **C-AD2-Fab-h734** | **Indium-DTPA** |
| **C-AD2-Fab-hA20** | **CD20** |
| **C-AD2-Fab-hA20L** | **CD20** |
| **C-AD2-Fab-hL243** | **HLA-DR** |
| **C-AD2-Fab-hLL2** | **CD22** |
| **N-AD2-Fab-hLL2** | **CD22** |
| **C-AD2-IgG-hMN-14** | **CEACAM5** |
| **C-AD2-IgG-hR1** | **IGF-1R** |
| **C-AD2-IgG-hRS7** | **EGP-1** |
| **C-AD2-IgG-hPAM4** | **MUC** |
| **C-AD2-IgG-hLL1** | **CD74** |
| | |
| **C-DDD1-Fab-hMN-14** | **CEACAM5** |
| **C-DDD2-Fab-hMN-14** | **CEACAM5** |
| **C-DDD2-Fab-h679** | **HSG** |
| **C-DDD2-Fab-hA19** | **CD19** |
| **C-DDD2-Fab-hA20** | **CD20** |
| **C-DDD2-Fab-hAFP** | **AFP** |
| **C-DDD2-Fab-hL243** | **HLA-DR** |
| **C-DDD2-Fab-hLL1** | **CD74** |
| **C-DDD2-Fab-hLL2** | **CD22** |
| **C-DDD2-Fab-hMN-3** | **CEACAM6** |
| **C-DDD2-Fab-hMN-15** | **CEACAM6** |
| **C-DDD2-Fab-hPAM4** | **MUC** |
| **C-DDD2-Fab-hR1** | **IGF-1R** |
| **C-DDD2-Fab-hRS7** | **EGP-1** |
| **N-DDD2-Fab-hMN-14** | **CEACAM5** |

### Example 17. Use of NK-Targeted Leukocyte-Redirecting bsAbs

The use of bsAbs to retarget leukocytes is not limited to antibodies against T cells. In alternative embodiments, bsAbs that bind to monocytes, NK cells or neutrophils may also be used for retargeting purposes.

CD16 is an activating low-affinity Fc-γ receptor for IgG, which is highly expressed by the CD56^{dim} subset of NK cells (Gleason et al., 2012, Mol Cancer Ther 11:2674-84). In addition to their use in NK cell retargeting, bsAbs comprising an anti-CD16 antibody component have the ability to activate NK-mediated cytotoxicity through direct signaling of CD16, inducing directed secretion of lytic granules and target cell death (Gleason et al., 2012).

A CD16/CD19 bispecific killer cell engager (BiKE) and a CD16/CD19/CD22 trispecific killer cell engager (TriKe) are prepared according to (Gleason et al., 2012, Mol Cancer Ther 11:2674-84), using DNA shuffling and ligation techniques as previously reported (Vallera et al., 2005, Clin Cancer Res 11:3879-88). The expressed BiKE and TriKE are purified by sequential ion exchange and size-exclusion column chromatography. Resting PBMCs are exposed to primary ALL and CLL tumor cells in the presence of CD16/CD19 BiKE or CD16/CD19/CD22 TriKE (10 µg/mL). A significant increase in cytotoxicity to tumor cells is observed in the presence of the BiKE or TriKE, compared to cells without retargeting antibody. No effect is observed on tumor cells exposed to BiKE or TriKE in the absence of PBMCs. The TriKE has a greater effect on tumor cell toxicity relative to the BiKE, indicating that binding to an additional tumor cell antigen may enhance the retargeting effect. Similar results are obtained using purified NK cells instead of PBMCs.

A CD16/CD33 BiKE is prepared as disclosed in Wiernik et al. (2013, Clin Cancer Res 19:3844-55. The BiKE is administered to nude mice injected with human HL60 promyelocytic leukemia xenograft cells, co-administered with human PBMCs. The BiKE treated mice show a decreased mortality and tumor growth rate compared to mice treated with control bsAbs. Addition of an anti-CD33-SN-38 ADC further enhances the cytotoxic effect of the BiKE.

### Example 18. Trivalent Antibodies for Therapeutic Use

A trivalent, trispecific cell targeting construct is made as described in patent EP1309795B1 comprising: (i) chimerizing or humanizing a mouse anti-CD16 mab as described in patent US 618728 from which the Fab of Claim 1 of EP1309795 is derived; (ii) constructing a single chain antibody comprised of the Fv of the humanized anti-Trop-2 antibody described in US 7,238,785, and joining the scFv by a linker to the carboxyl terminal of the light chain of the anti-CD16 Fab of (i); and (iii) constructing a single chain of the Fv of the humanized anti-CD19 described in US8486395 and joining the scFv by a linker to the carboxyl terminal of the CH1 of the anti-CD16 Fab of (ii).

The trivalent construct is administered to a subject with metastatic pancreatic cancer, in combination with IMMU-132. A partial response is observed and the tumor shows a regression in size that lasts for 12 months.

### Example 19. Anti-Trop-2 × Anti-CD3 Bispecific Antibody

A bispecific antibody (bsAb) was produced as a tandem single-chain variable fragment (scFv) for redirecting T cells via CD3 binding to tumor cells, particularly carcinomas, via Trop-2 targeting. Trop-2 is a tumor-associated antigen (TAA) that could be highly effective for targeting various epithelial cancers. However, it has yet to be investigated in any bsAb format for T-cell-redirected therapy. Trop-2 is a 35 kDa transmembrane glycoprotein that is overexpressed relative to normal tissues in a variety of human cancers, including pancreatic and gastric carcinomas, where increased expression correlates with poor prognosis (Fong et al., 2008, Br J Cancer 99:1290-5; Iacobuzio-Donahue et al., 2002, Am J Pathol 160:1239-49; Kapoor, 2013, Tumour Biol 34:1967-8; Muhlmann et al., 2009, J Clin Pathol 62:152-8; Stein et al., 1993, Int J Cancer 55:938-46; Stein et al., 1993, Int J Cancer 55:938-46). Variable domains (VH and VK) derived from hRS7, the humanized version of the original murine anti-Trop-2 mAb, RS7, were combined with the variable domains of the murine anti-CD3 mAb, Okt3, to generate the E1-3 bsAb.

### Construction of a plasmid vector for expression of E1-3 in mammalian cells

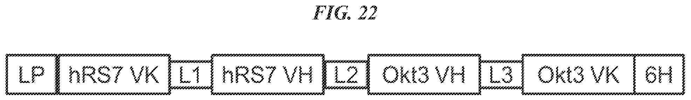

A double stranded DNA sequence (SEQ ID NO:106) was synthesized and assembled into the pUC57 plasmid vector. SEQ ID NO:106 was excised from pUC57 by digestion with Xba I and Eag I restriction endonucleases, and ligated into the pdHL2 mammalian expression vector, which was prepared by digestion with the same enzymes. The coding sequence directs the synthesis of a single polypeptide (SEQ ID NO:107) comprising a leader peptide, hRS7VK (SEQ ID NO:108), L1 (SEQ ID NO:109), hRS7VH (SEQ ID NO:110), L2 (SEQ ID NO:111), Okt3VH (SEQ ID NO:112), L3 (SEQ ID NO:113), Okt3VK (SEQ ID NO:114), and 6-His (SEQ ID NO:105). A schematic representation of the tandem scFv E1-3 is shown in **FIG. 22****.**

### Synthetic DNA sequence comprising El-3 insert

tctagacacaggccgccatcatgggatggagctgtatcatcctcttcttggtagcaacagctacaggtgtccactccgacattcagctga
cccagtctccatcctccctgtctgcatctgtaggagacagagtcagcatcacctgcaaggccagtcaggatgtgagtattgctgtagcct
ggtatcagcagaaaccagggaaagcccctaagctcctgatctactcggcatcctaccggtacactggagtccctgataggttcagtgg
cagtggatctgggacagatttcactctcaccatcagcagtctgcaacctgaagattttgcagtttattactgtcagcaacattatattactcc
gctcacgttcggtgctgggaccaaggtggagatcaaaggtggaggagggtccggtggaggagggtctggtggaggagggagcca
ggtccagctgcagcaatctgggtctgagttgaagaagcctggggcctcagtgaaggtttcctgcaaggcttctggatacaccttcacaa
actatggaatgaactgggtgaagcaggcccctggacaagggcttaaatggatgggctggataaacacctacactggagagccaacat
atactgatgacttcaagggacggtttgccttctccttggacacctctgtcagcacggcatatctccagatcagcagcctaaaggctgacg
acactgccgtgtatttctgtgcaagagggggggttcggtagtagctactggtacttcgatgtctggggccaagggtccctggtcaccgtct

cctcaggtggcggagggtccgatatcaagctgcagcagtctggagcagagctcgctcgaccaggagctagtgtgaagatgtcatgta
aaacaagtggctatactttcacccggtacactatgcactgggtcaagcagcgcccaggacagggtctggaatggatcggctacattaa
ccccagcaggggatataccaactacaatcagaagttcaaggataaagccaccctgactaccgacaagtcctctagtacagcttatatgc
agctgtcaagcctcacttccgaggactctgcagtgtattactgcgccagatattacgacgatcattattgtctggattactggggccaggg
aacaactctcacagtgtcctctgtcgaaggtggcagtggagggtcaggtggcagcggagggtccggtggagtggacgatatccagc
tgacccagtctcctgccattatgagcgcttccccaggcgagaaggtgacaatgacttgccgggccagttcaagcgtcagctatatgaat
tggtatcagcagaagtctggaaccagtcctaaacgatggatctatgacacatctaaagtggcaagcggggtcccatacaggttctctgg
gagtggttcaggcactagctattccctgaccatttcctctatggaggccgaagatgcagccacctattactgtcagcagtggagttcaaa
tccactcaccttcggagcaggcactaaactggaactcaagcaccaccaccaccaccactaaggcggccg (SEQ ID NO:106)

### Deduced amino acid sequence of E1-3

DIQLTQSPSSLSASVGDRVSITCKASQDVSIAVAWYQQKPGKAPKLLIYSASYRYTGV
PDRFSGSGSGTDFTLTISSLQPEDFAVYYCQQHYITPLTFGAGTKVEIKGGGGSGGGG
SGGGGSQVQLQQSGSELKKPGASVKVSCKASGYTFTNYGMNWVKQAPGQGLKWM
GWINTYTGEPTYTDDFKGRFAFSLDTSVSTAYLQISSLKADDTAVYFCARGGFGSSY
WYFDVWGQGSLVTVSSGGGGSDIKLQQSGAELARPGASVKMSCKTSGYTFTRYTM
HWVKQRPGQGLEWIGYINPSRGYTNYNQKFKDKATLTTDKSSSTAYMQLSSLTSED
SAVYYCARYYDDHYCLDYWGQGTTLTVSSVEGGSGGSGGSGGSGGVDDIQLTQSP
AIMSASPGEKVTMTCRASSSVSYMNWYQQKSGTSPKRWIYDTSKVASGVPYRFSGS
GSGTSYSLTISSMEAEDAATYYCQQWSSNPLTFGAGTKLELKHHHHHH (SEQ ID
NO:107)

### Amino acid sequence of hRS7 VK

DIQLTQSPSSLSASVGDRVSITCKASQDVSIAVAWYQQKPGKAPKLLIYSASYRYTGV
PDRFSGSGSGTDFTLTISSLQPEDFAVYYCQQHYITPLTFGAGTKVEIK (SEQ ID
NO:108)

### Amino acid sequence of linker L1

GGGGSGGGGSGGGGS (SEQ ID NO: 109)

### Amino acid sequence hRS7 VH

QVQLQQSGSELKKPGASVKVSCKASGYTFTNYGMNWVKQAPGQGLKWMGWINTY
TGEPTYTDDFKGRFAFSLDTSVSTAYLQISSLKADDTAVYFCARGGFGSSYWYFDV
WGQGSLVTVSS (SEQ ID NO:110)

### Amino acid sequence of linker L2

GGGGS (SEQ ID NO:111)

### Amino acid sequence of Okt3 VH

DIKLQQSGAELARPGASVKMSCKTSGYTFTRYTMHWVKQRPGQGLEWIGYINPSRG
YTNYNQKFKDKATLTTDKSSSTAYMQLSSLTSEDSAVYYCARYYDDHYCLDYWGQ
GTTLTVSS (SEQ ID NO:112)

### Amino acid sequence of linker L3

VEGGSGGSGGSGGSGGVD (SEQ ID NO:113)

### Amino acid sequence of Okt3 VK

DIQLTQSPAIMSASPGEKVTMTCRASSSVSYMNWYQQKSGTSPKRWIYDTSKVASG
VPYRFSGSGSGTSYSLTISSMEAEDAATYYCQQWSSNPLTFGAGTKLE (SEQ ID
NO:114)

### Development of a stable production clone in SpESF myeloma cells

The E1-3-pdHL2 vector was linearized by digestion with Sal I restriction endonuclease and 30 µg was used to stably transfect 1 × 10⁷ SpESFX myeloma cells (Rossi et al., 2011, Biotechnol Prog 27:766-75) by electroporation using two pulses at 850 V and 10 µF. Selection and production media was supplemented with 0.2 µM methotrexate (MTX). Transfectant clones were selected in 96-well tissue culture plates and screened for E1-3 expression by ELISA using Ni-NTA 96-well plates. The E1-3 protein was purified from the culture broth of roller bottle cultures by immobilized metal affinity chromatography (IMAC) using Nickel-SEPHAROSE^{®} resin, followed by size exclusion high performance liquid chromatography (SE-HPLC). The purified product resolved as a single SE-HPLC peak (not shown) and a single polypeptide band by SDS-PAGE (not shown), with relative mobilities consistent with its calculated molecular size of 53,423 Da.

### Example 20. Redirected T cell Killing of Trop-2-Expressing Solid Tumor Cells Ex Vivo

Peripheral blood mononuclear cells (PBMCs) were prepared from the buffy coat of blood specimens of two healthy donors (Blood Center of NJ), and used for the isolation of CD8⁺ T cells (Miltenyi). Capan-1 (pancreatic cancer, 157,000 Trop-2/cell), BxPC3 (pancreatic cancer, 500,000 Trop-2/cell) and NCI-N87 (gastric cancer, 247,000 Trop-2/cell) cell lines (ATCC) were used as target cells expressing low-, high- and mid-levels of Trop-2. BxPC3 and NCI-N87 were maintained in RPMI1640 media supplemented with 10% FBS, while Capan-1 cells were maintained in 20% FBS/RPMI1640. CD8⁺ T cells (1.2 × 10⁵ cells/well) were combined with target cells (2 × 10⁴ cells/well) at a 6:1 ratio in 96-well tissue culture plates. Titrations of E1-3 and (E1)-3s were added to the assay plates. Following a 48-hour incubation at 37°C, plates were washed twice with PBS to remove the T cells, and then 150 µL of fresh media supplemented with 30% MTS reagent (CELLTITER 96^{®} Aqueous One Solution, Promega) was added to each well. Absorbance at 490 nm was measured after 1-2 h at 37°C with an ENVISION plate reader^{®}.

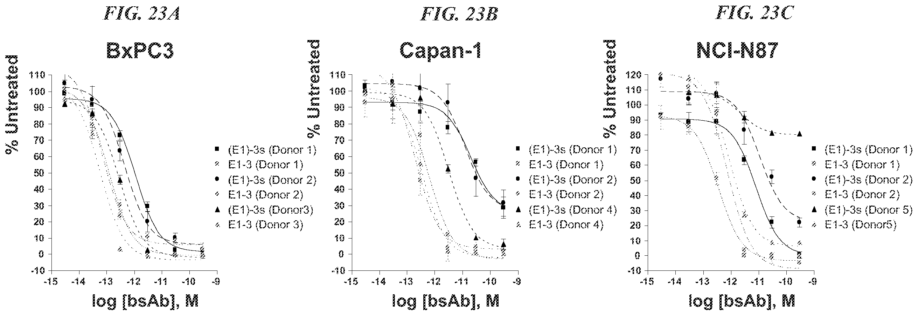

The *in vitro* potency of the E1-3 bispecific antibody was compared with that of the equivalent DNL construct, (E1)-3s, in three Trop-2-expressing cell lines (BxPC3, Capan-1 and NCI-N87) using T cells from three donors for each cell line (**FIG. 23**). Based on the IC₅₀ values (**Table 12**), E1-3 is at least 5-fold more potent than (E1)-3s in all three cell lines, whose relative sensitivity to E1-3 appears to correlate with the Trop-2-antigen density, when compared with T cells from the same donor. However, potency was varied among the donor T cells used. *In vitro,* E1-3 mediated a highly potent T-cell lysis of BxPC3 [IC₅₀=0.09(±0.04) pM], Capan-1 [IC₅₀=1.2(±1.1) pM] and NCI-N87 [IC₅₀=1.2(±1.2) pM] target cells.

**Table 12. IC₅₀ values for ex vivo T cell redirected killing of Trop-2⁺ cancer cell lines with E1-3 and (E1)-3s.**

| | BxPC3 | | | Capan-1 | | | NCI-N87 | | |
|---|---|---|---|---|---|---|---|---|---|
| Trop-2 | 500,000/cell | | | 157,000/cell | | | 247,000/cell | | |

| | Donor 1 | Donor 2 | Donor 3 | Donor 1 | Donor 2 | Donor 4 | Donor 1 | Donor 2 | Donor 5 |
|---|---|---|---|---|---|---|---|---|---|
| E1-3 | 0.12 | 0.10 | 0.05 | 0.58 | 2.7 | 0.47 | 0.29 | 0.76 | 2.50 |
| (E1)-3s | 1.06 | 0.56 | 0.32 | 35.6 | 248 | 8.51 | 6.76 | 34 | NA* |

| | | | | | | | | | |
|---|---|---|---|---|---|---|---|---|---|
| IC₅₀ values = pM concentration resulting in 50% killing. *Did not achieve 50% killing. Donors 1 and 2 were the same for each donor. Donors 3, 4 and 5 were independent donors. | | | | | | | | | |

### Example 21. In vivo Therapy of Solid Tumors With E1-3 vs. (E1)-3s

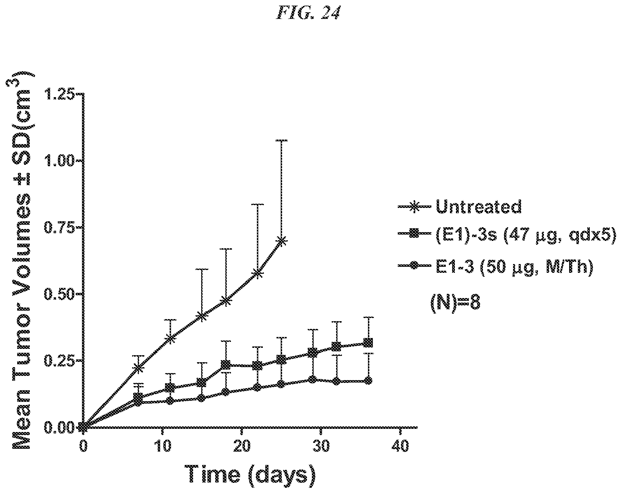

Female 4-8-week old NOD/SCID mice were administered subcutaneous injections of a mixture of PBMCs and NCI-N87 (2:1) mixed with an equal volume of MATRIGEL^{®}. Therapy consisted of i.v. injections of 50 µg of E1-3 on days 1 and 4, or daily injections with 47 µg of (E1)-3s on days 1 through 5. The untreated group received the mixture of NCI-N87 and PBMCs without bsAb. Tumor volume (TV) was determined twice weekly by measurements in two dimensions using calipers, with volumes defined as: L × W²/2, where L is the longest dimension of the tumor and W the shortest (**FIG. 24**). Statistical analysis of tumor growth was based on area under the curve (AUC). Profiles of individual tumor growth were obtained through linear-curve modeling. An F-test was employed to determine equality of variance between groups prior to statistical analysis of growth curves. A Critical Z test on the survival data identified any outliers within a given treatment group with P<0.05 censored from the final data analysis. A two-tailed t-test was used to assess statistical significance between the various treatment groups and controls, except for the untreated control, where a one-tailed t-test was used. Additionally, efficacy was determined by log-rank using Prism software on Kaplan-Meier curves using survival surrogate endpoints as time for tumor progression (TTP) to 1.0 cm3. Significance was considered at P≤0.05 for all comparisons.

Both E1-3 (P) and (E1)-3s delayed growth of NCI-N87 tumors significantly (*P*≤0.001; AUC_{day25}) (**FIG. 24**). The E1-3 was superior to (E1)-3s (*P*=0.0324, AUC_{day36}) (**FIG. 24**). *In vivo,* two 50-µg doses of E1-3 given three days apart cured all of the mice (N=8) bearing NCI-N87 xenografts (*P*=0.0005; Log-Rank). Tumors in the control group (PBMCs only) reached the endpoint (TV>1 cm³) at 39.5 days. All mice were tumor-free in the E1-3 group after 78 days.

### Example 22. Trogocytosis Induced by Anti-CD3 Anti-Trop-2 Bispecific Antibodies

Trop-2 has limited presence on normal tissues but is highly expressed in diverse epithelial cancers. As discussed in the Examples above, (E1)-3s is a T-cell-redirecting trivalent bispecific antibody (bsAb) DNL^{®} complex, comprising an anti-CD3 scFv covalently linked to a stabilized dimer of a Trop-2-targeting Fab. We show herein for the first time that bsAb-mediated bidirectional trogocytosis occurs between target cells and T cells and involves formation of immunologic synapses.

### Methods

BxPC3 cells were detached with trypsin (which does not affect Trop-2) and mixed with purified T cells. Cell mixtures were treated with 0.1 nmol/L bsAbs at 37°C for 1 hour. Cells were stained with either: (i) anti-Trop-2 MABC518 followed by GAM-FITC, or (ii) anti-Trop-2-PE clone MR54 and anti-CD4-APC. Single BxPC3 and T cells were gated from cell conjugates by forward and side scattering, as well as Trop-2 and CD4 fluorescence.

### Results

(E1)-3s induces the formation of immunologic synapses between T cells and target cells. This was shown using Capan-1 pancreatic carcinoma cells (Rossi et al., 2013, MAbs 6:381-91). Here, addition of 0.1 µg/mL (E1)-3s to a mixture of purified CD8⁺ T cells and NCI-N87 gastric carcinoma cells, which were membrane-labeled with red and green fluorescence, respectively, resulted in the formation of conjugates evident by fluorescence microscopy (not shown). No conjugates were observed in the presence of (19)-3s (not shown) or TF12 (not shown), which bind only T cells or NCI-N87, respectively. Dunking the slides in saline washed off the vast majority of T cells in wells containing (19)-3s or TF12, whereas many T cells remained bound to the adherent NCI-N87 cells in the wells treated with (E1)-3s.

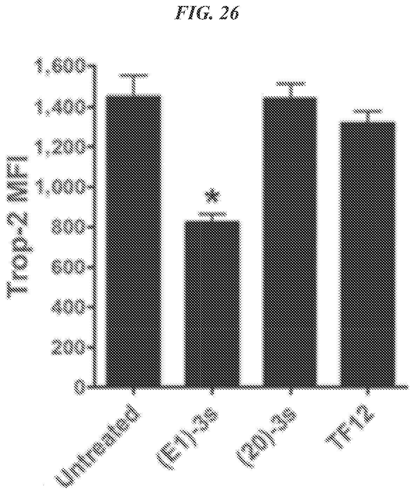

Treatment of BxPC3 (500,000 Trop-2/cell) and purified T-cell mixtures with (E1)-3s specifically induced trogocytosis,whereby Trop-2 was transferred from BxPC3 to T cells (**FIG. 25**). Whereas (E1)-3s treatment resulted in 40% Trop-2⁺ T cells, <5% of the T cells were counted in the Trop-2⁺ gate following treatment with control bsAbs binding only Trop-2 (TF12) or CD3 [(20)-3s], or with (E1)-3s in the absence of BxPC3 cells. The uptake of Trop-2 by T cells coincided with its reduction on BcPC3 cells (**FIG. 26**). During the short incubation time, the T cells (97.5% live) and BxPC3 (94.5% live) remained at high viability, indicating that the T cells acquired the tumor antigens by trogocytosis and not by adhering to membrane fragments of dead cells (not shown). Trogocytosis mediated by (E1)-3s was bidirectional, because T-cell membrane components were transferred to BxPC3 cells, as demonstrated for CD4 (data not shown).

### Example 23. Bispecific Anti-CD3 × Anti-Trop-2 Antibodies and Cytokine Release

As discussed in the Examples above, we studied the effects of interferon-α (IFNα) on (E1)-3s-mediated T-cell killing of human gastric and pancreatic cancer cell lines. T-cell activation, cytokine induction, and cytotoxicity were evaluated ex vivo using peripheral blood mononuclear cells (PBMC) or T cells with NCI-N87 gastric cancer as target cells. In the presence of target cells and PBMCs, (E1)-3s did not cause excess cytokine production. When combined with (E1)-3s, peginterferonalfa-2a-which alone did not increase T-cell activation or raise cytokine levels over baseline-increased CD69 expression but did not significantly increase cytokine induction. IFNα enhanced the therapeutic efficacy of (E1)-3s without increasing the production of other cytokines to levels that could induce cytokine release syndrome (CRS). Adjuvant therapy with IFNα, or other cytokines, might be universally applicable for enhanced efficacy of T-cell immunotherapy.

### Methods

Cytokine release was measured ex vivo using 5 × 10⁵ cells/0.5 mL/well of either NCI-N87, which were allowed to attach overnight, or Raji. Freshly isolated PBMCs (5 × 10⁶ cells/0.4 mL/well) were added to each well. Treatments (100 µL, 10x) comprising (19)-3s, 19-3 BiTE, (E1)-3s, peginterferonalfa-2a, or (E1)-3s plus peginterferonalfa-2a were added to 0.1 nmol/L for each reagent. Alternatively, titrations ranging from 1 pmol/L to 10 nmol/L were used for dose-response studies. Following a 20-hour incubation at 37°C with gentle shaking, the supernatant fluid was diluted 1:2 (or greater when necessary) and the concentrations of TNFα, IFNg, IL2, IL6, and IL10 measured using Single-Analyte ELISArray kits (Qiagen), following the manufacturer's protocol.

### Results

A Trop-2 × CD3 BiTE (or equivalent) was not available for comparison with (E1)-3s. However, the availability of both (19)-3s, which has the same (X)-3s molecular configuration as (E1)-3s, and 19-3 BiTE, which has the identical amino acid sequence as the CD19 × CD3 BiTE, blinatumomab, enabled a head-to-head comparison to evaluate the relative cytokine-inducing potency of the two bsAb formats.

Initially, titrations of (19)-3s and 19-3 BiTE were added to mixtures of PBMCs (two independent donors), and Raji NHL cells and the levels of TNFα, IFNγ, and IL6 were measured after 20 hours (not shown). Minimal cytokine levels were detected from PBMCs alone, even with the addition of a bsAb. However, because of a mixed lymphocyte reaction occurring between Raji and the donor PBMCs (stronger for donor A), cytokine levels in untreated cell mixtures were elevated for each of TNFα (200 and 50 pg/mL), IFNγ (600 and 200 pg/mL), and IL6 (190 and 220 pg/mL). The levels of TNFα and IL6 were increased above those of untreated only at ≥1 nmol/L (19)-3 s. Apparently, (19)-3s inhibited TNFα and IL6 production at lower concentrations. In comparison, TNFα and IL6 were elevated to >1,000 pg/mL at all concentrations of 19-3 BiTE tested (≥1 pmol/L). The levels of IFNγ were not increased significantly by (19)-3s, whereas 19-3 BiTE showed a dose-dependent increase to >2,000 pg/mL.

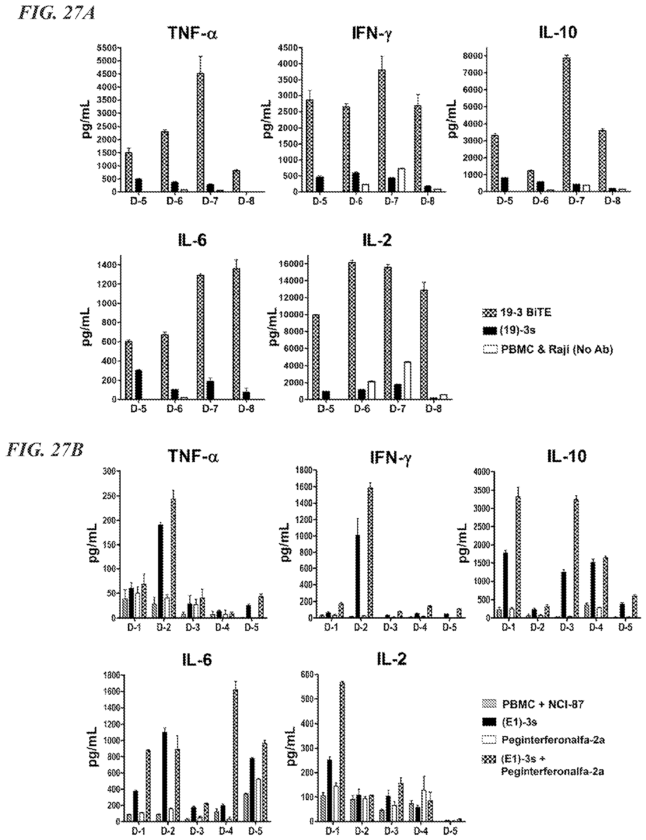

For all further comparisons, agents were tested at 0.1 nmol/L, which is approximately what has been used in similar studies with BiTE (Brandl et al., Cancer Immunol Immunother 2007, 56:1551-63). We compared the levels of TNFα, IPNγ, IL2, IL6, and IL10 induced by 0.1 nmol/L (19)-3s or 19-3 BiTE from Raji mixed with PBMCs, using 4 different donors (**FIG. 27A**). With each of the 4 donors, the levels of each of the five cytokines were significantly higher with 19-3 BiTE, compared with (19)-3s. The mean TNFα concentration with 19-3 BiTE (2,284 ± 1,483 pg/mL) was 8-fold higher (P=0.0001) than that with (19)-3s (280 ± 188 pg/mL). Treatment with 19-3 BiTE, compared with (19)-3s, resulted in levels of IFNγ (3,002 ± 560 pg/mL vs. 416 ± 169 pg/mL), IL2 (13,635 ± 2,601 pg/mL vs. 1,024 ± 598 pg/mL), IL6 (981 ± 364 pg/mL vs. 168 ± 96 pg/mL), and IL10 (4,006 ± 2,520 pg/mL vs. 493 ± 242 pg/mL) that were 7-, 13-, 6-, and 8-fold higher for 19-3 BiTE, respectively (P < 0.0001 for each). These results indicate that the (X)-3s bsAb format is a considerably less potent inducer of cytokine release, compared with the BiTE format.

In general, (E1)-3s in the presence of PBMCs and target cells caused even less cytokine production than (19)-3s,because there is no mixed lymphocyte reaction to elevate the baseline levels (**FIG. 27B**). Levels remained low for the proinflammatory cytokines IFNγ (<100 pg/mL), TNFα (<100 pg/mL), and IL2 (<250 pg/mL) with 4 of 5 donors. IL6 was low (<400 pg/mL) in 3 of 5 donors, and moderate (800-1,100 pg/mL) in donors D-2 and D-5. Donor D-2 also responded to the (E1)-3s more than the others for IFNγ (1,000 pg/mL) and TNFα (190 pg/mL). IL10, an anti-inflammatory cytokine, was significantly (P < 0.0001) elevated by (E1)-3s to >1,200 pg/mL in 3 of 5 donors. Of note, donor D-2, who had a uniquely potent proinflammatory response, produced relatively low levels of IL10 (230 pg/mL) after treatmentwith (E1)-3s. Peginterferonalfa-2a alone did not increase the level of any cytokine over background. Addition of peginterferonalfa-2a to (E1)-3s consistently increased IFNγ (~1.5-3-fold) over (E1)-3s alone. For the rest of the cytokines, there was an apparent trend for a
moderately increased production with the combination; however, a consistent effect was not observed.

### Example 24. In Vitro Cytotoxicity Induced by Bispecific Anti-CD3 × Anti-Trop-2 Antibodies

Further studies were performed to examine *in vitro* cytotoxicity induced by anti-CD3 × anti-Trop-2 bispecific antibodies.

### Methods

Freshly-isolated CD8⁺ T cells were incubated for 24 h with 0.1 nM peginterferonalfa-2a, 0.1 nM 20*-2b, or media only. Treated or untreated T cells and PKH67 green fluorescent-labeled NCI-N87 cells were combined at a 5:1 ratio (5 × 10⁴ target cells and 2.5 × 10⁵ effector cells/well) in 48-well plates containing serial dilutions of (E1)-3s in triplicate. Peginterferonalfa-2a or 20*-2b were maintained at 0.1 nM in the appropriate cell mixtures. Plates were incubated for 48 h at 37°C. Suspension cells were removed and the attached cells were detached with trypsin-EDTA and combined with the corresponding suspension. Cells were washed and resuspended in 1% BSA-PBS containing 30,000 COUNTBRIGHT^{™} Absolute Counting Beads (Life Technologies) and 1 µg/mL of 7-AAD. Total live target cells (7-AAD⁻/PKH67⁺) were counted by flow cytometry. For each sample, 8,000 COUNTBRIGHT^{™} beads were counted as a normalized reference. The specific lysis (%) was calculated using the formula: [1-(A₁/A₂)] × 100, where A₁ and A₂ represent the number of viable target cells in the test and untreated samples, respectively. Statistical significance (*P*≤0.05) was determined for IC₅₀ (the concentration resulting in 50% lysis), EC₅₀ (50% effective concentration) and lysis^{max} (maximal target cell lysis) by F-test on non-linear regression (sigmoidal dose-response) curves with Prism software.

### Results

To evaluate redirected T-cell killing of Trop-2-expressing tumor cells, CD8⁺ T cells were mixed with NCI-N87 cells in the presence or absence of IFN-α2 (0.1 nM peginterferonalfa-2a or 20*-2b) along with titrations of (E1)-3s (**FIG. 28**). Considerable variability in T-cell potency was observed among donors (**FIG. 28A, FIG. 28B**). With a donor of very active T cells, (E1)-3s mediated a highly potent (IC₅₀=0.37 pM; lysis^{max}=77.1%) T-cell lysis of NCI-N87 cells, and inclusion of peginterferonalfa-2a enhanced its activity, improving the IC₅₀ (0.14 pM; *P*=0.0001) by more than 2.5 fold and increasing lysis^{max} (84.0%; *P*<0.0001) (**FIG. 28A**). NCI-N87 was only weakly sensitive to the direct actions of IFN-α (peginterferonalfa-2a IC₅₀ = >10 nM, data not shown), and inhibited <10% by 0.1 nM peginterferonalfa-2a in the absence of (E1)-3s. The more potent form of IFNα, 20*-2b, consisting of 4 IFN-α molecules fused to a bivalent anti-CD20 mAb by DNL^{®}, enhanced the potency of (E1)-3s by more than 7-fold (IC₅₀=0.05 pM; P<0.0001). At 0.1 nM, 20*-2b inhibited NCI-N87 by 12.6% in the absence of (E1)-3s. The 20*-2b was included only to show enhanced activity with another (more potent) form IFN-α, and that the effect is not restricted to peginterferonalfa-2a. The anti-CD20 mAb moiety is not functional in this experiment. In a similar assay using very weak donor T cells, (E1)-3s was considerably less potent (EC₅₀=39 pM; lysis^{max}=21%); however, addition of peginterferonalfa-2a enhanced the potency by >25 fold (EC₅₀=1.4 pM; P=0.0008) (**FIG**. **28B**). Potent (E1)-3s-mediated T-cell killing also was observed for the human pancreatic cancer line, BxPC3 (IC₅₀=0.4 pM); however, the effect of adding IFN-α was not evaluated with this cell line (not shown).

### Example 25. Dose-Response Curves for T Cell Activation by Anti-CD3 × Anti-Trop-2 Bispecific Antibodies

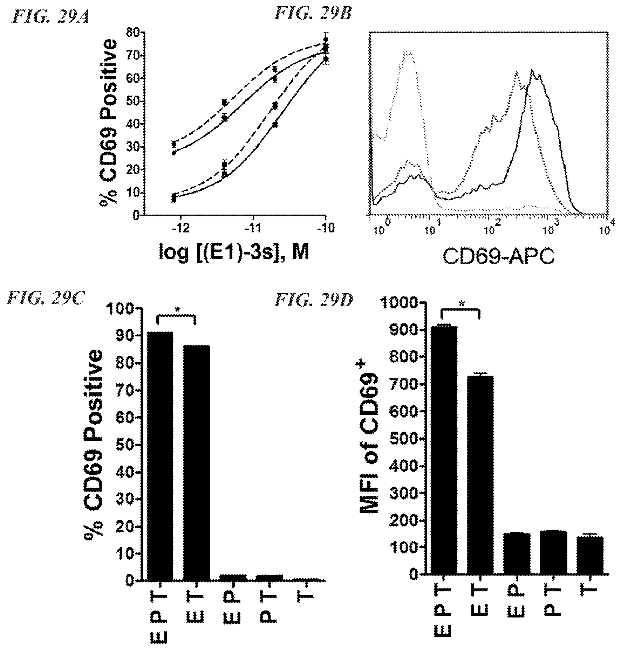

Addition of 0.1 nM peginterferonalfa-2a increased CD69 upregulation on T cells treated with (E1)-3s moderately, but significantly. For (E1)-3s dose-response experiments measuring %CD69⁺ T cells, the EC₅₀ was lowered from 26 pM to 16 pM (P<0.0001) for CD4⁺ T cells, and from 11 pM to 6 pM (*P*=0.0204) for CD8⁺ T cells in the presence of IFN-α (**FIG. 29A**). Peginterferonalfa-2a combined with (E1)-3s resulted in more CD69⁺ cells (**FIG. 29B, FIG. 29C****,** *P*<0.0001), and also, the activated cells had significantly higher CD69 expression with IFN-α (**FIG. 29B, FIG. 29D****;** MFI= 907 *vs* 726; *P*<0.0001). Peginterferonalfa-2a induced minimal CD69 expression in the absence of (E1)-3s. Likewise, (E1)-3, either alone or in combination with peginterferonalfa-2a, did not activate T cells in the absence of target cells.

### Example 26. Extended In Vivo Survival With (E1)-3s is Augmented with IFN-α

The preliminary data on *in vivo* survival reported in Example 3 above were further extended to as long as 126 days. As shown below, the combination of (E1)-3s with IFN-α provided the greatest benefit for animals bearing Trop-2⁺ xenograft tumors.

### Methods

Female 4-8-week old NOD/SCID mice (Charles River, Wilmington, MA) were injected s.c. with a mixture of 5 × 10⁶ tumor cells (Capan-1 or NCI-N87) and T cells (2.5 × 10⁶) combined with an equal volume of matrigel. Therapy began 1 h later by i.v. injection, as per the BiTE methodology (Dreier et al., 2003, J Immunol 170:4397-402). Treatment regimens, dosages, and number of animals in each experiment are described in the figure legends. Tumor volume was determined twice weekly by measurements in two dimensions using calipers, with volumes defined as: *L* × *w*²/2, where *L* is the longest dimension of the tumor and *w* the shortest.

Statistical analysis of tumor growth was based on area under the curve (AUC). Profiles of individual tumor growth were obtained through linear-curve modeling. An *F*-test was employed to determine equality of variance between groups prior to statistical analysis of growth curves. A Critical Z test on the survival data identified any outliers within a given treatment group with *P*≤0.05 censored from the final data analysis. A two-tailed *t*-test was used to assess statistical significance between the various treatment groups and controls, except for the untreated control, where a one-tailed *t*-test was used. Additionally, efficacy was determined by log-rank using Prism software on Kaplan-Meier curves using survival surrogate endpoints as time for tumor progression to 1.0 cm³. Significance was considered at *P*≤0.05 for all comparisons.

### Results

*In vivo* efficacy with human pancreatic cancer was evaluated with Capan-1 xenografts. In the first study, treatment with a combination of (E1)-3s and peginterferonalfa-2a [median survival time (MST) >59 days] was superior to all other treatments (*P*<0.0007, log-rank), including (E1)-3s (MST = 50 days) or peginterferonalfa-2a (MST = 53 days) alone (**FIG. 30A**). Even with the omission of T cells, peginterferonalfa-2a extended survival (MST = 45 days, *P*=0.0059 *vs* saline, log-rank), indicating direct action on the tumor cells. However, peginterferonalfa-2a was more effective in the presence of T cells (*P*=0.0260, AUC), suggesting stimulation of T cells by IFN-α. TF12, which binds target but not T cells, did not affect tumor growth or survival. A repeat experiment, using T cells from a different donor, confirmed the results of the first study (**FIG. 30B**). The second study continued until all groups reached their MST. As in the initial experiment, the combination of (E1)-3s and peginterferonalfa-2a (MST = 119.5 days) was superior to all other groups in terms of both tumor growth inhibition and overall survival (*P*=0.0475 *vs* (E1)-3s alone; *P*<0.0001 *vs* all other groups; log-rank). (E1)-3s (MST = 68 days) was superior (*P*=0.0373, AUC over 29 days) to peginterferonalfa-2a with T cells (MST = 53 days) and to T cells alone (MST = 37.5 days; *P*=0.0014 log-rank).

For the NCI-N87 gastric cancer xenograft model (**FIG. 30C**), the combination of (E1)-3s and peginterferonalfa-2a (MST>88 days) was superior to (E1)-3s alone (MST=49 days; *P*=0.0007, log-rank). Compared to the control group with only T cells (MST=32 days), peginterferonalfa-2a alone with T cells provided only a minor, but significant, survival advantage (MST=35 days; *P*=0.0276). (E1)-3s plus peginterferonalfa-2a without T cells did not improve survival significantly.

The antigen density measured for NCI-N87 [247,000(±65,000) Trop-2/cell] and Capan-1 [157,000 (±37,000) Trop-2/cell] was not significantly different. Compared to NCI-N87, Capan-1 cells were >5-fold more sensitive (IC₅₀=2 nM vs. >10 nM) to direct inhibition by peginterferonalfa-2a *in vitro* (not shown). (E1)-3s does not cross-react with mouse Trop-2 or CD3 (not shown), and NOD-SCID mice are T-cell deficient.

### Discussion

This section discusses results presented in Examples 23-26. We described in Examples 1 and 2 above the use of the (X)-3s bsAb format for redirecting T cell-mediated therapy of both hematopoietic and solid tumors using several example constructs, including (E1)-3s, (19)-3s and (20)-3s. In one *in vivo* experiment from that study, where Capan-1 xenografts were treated with (E1)-3s, we included groups with peginterferonalfa-2a, because prior (unpublished) data showed that Capan-1 was inhibited by IFN-α. The striking enhancement observed with the addition of IFN-α spurred further investigation, leading to the current studies. The results of studies with T cell redirecting bispecific antibodies, in combination with peginterferonalfa-2a are reported herein. The studies were extended until all groups reached their MST, confirming that IFN-α can enhance the *in-vivo* efficacy of T-cell killing of an IFN-α-sensitive cell line. IFN-α also can enhance T-cell-mediated killing of a cell line that is weakly sensitive to the direct actions of IFN-α. These *in vivo* studies were performed following methods, including dosing and schedules, typically used with BiTE constructs.

Flieger and colleagues demonstrated that *in-vitro* killing by CD3⁺CD56⁺ NK-T cells, which were expanded *ex vivo* and redirected with an EpCAMxCD3 BiTE (MT110), was enhanced with either IFN-α or IL-2 (Flieger et al., 2000, Cancer Immunol Immunother 49:441-8). However, even in the absence of the bsAb, IFN-α significantly inhibited the target cells. Since a control to evaluate potential direct effects of IFN-α on target cells was lacking, the extent to which the enhanced cytotoxicity was due to IFN-α stimulating NK-T cells, compared to direct inhibition of target cells, could not be determined. Therefore, we measured the sensitivity to IFN-α for both target cells and included groups with peginterferonalfa-2a only, both in the presence and absence of pan-T cells. For Capan-1 tumors, which were more sensitive to IFN-α *in vitro,* peginterferonalfa-2a improved survival in the absence of T cells, and even more so in the presence of T cells, indicating that IFN-α acted on both Capan-1 as well as T cells in this model. In the absence of T cells, peginterferonalfa-2a did not improve survival of mice bearing NCI-N87 xenografts, which were weakly sensitive to IFN-α *in vitro,* indicating that the enhancement with IFN-α was due primarily to its actions on T cells. The mechanism of the observed T-cell enhancement by IFN-α is unclear. The increase in CD69 expression attributed to IFN-α was moderate, but significant, suggesting that the cytokine may potentiate T-cell activation induced with the bsAb. Additionally, IFN-α specifically increased (up to 3-fold) the release of IFN-γ, which is considered the chief cytotoxic cytokine produced by cytotoxic T cells, whereas none of the other cytokines measured increased consistently.

Combination therapy with IFN-α and a T-cell-redirecting bsAb has not been investigated clinically, or even in animal models. However, IL-2 was combined with a F(ab')₂ fragment of an anti-CD3/EpCAM quadroma in a clinical trial (Kroesen et al., 1997, Cancer Immunol Immunother 45:203-6), but treatment was limited due to considerable toxicity most likely caused by induction of secondary cytokines, known as CRS or cytokine storm. Systemic administration of IL-2 is known to induce a cytokine storm (Panelli et al., 2004, J Transl Med 2:17), and the severity of adverse events associated with CRS, such as with the TGN1412 catastrophic trial, are correlated with IL-2 release (Eastwood et al., 2013, Br J Clin Pharmacol 76:299-315). Although it is not without side effects, immunotherapy with IFN-α, which is not produced by T cells, is not typically associated with cytokine storm.

CRS is a risk associated with immunotherapy using any T-cell directed mAb (e.g., Okt3) or bsAb, including BiTE (Klinger et al., 2012, Blood 119:6226-33). However, not all bsAb formats necessarily have the same risk. Brandl *et al.* reported cytokine induction with blinatumomab, where response levels of IL-2, IL-6, IFN-γ, and TNF-α were variable among donors and typically peaked at > 1 ng/mL, with some donors reaching levels as high as 5 ng/mL (Brandl et al., 2007, Cancer Immunol Immunother 56:1551-63). We lacked a suitable BiTE, or equivalent construct, for direct comparison with (E1)-3s. However, we were able to compare the relative cytokine-inducing potency between the (X)-3s and BiTE formats, using a CD19XCD3 BiTE (identical sequence as blinatumomab) and (19)-3s made by DNL^{®}. The 19-3 BiTE induced similar cytokine levels as reported by Brandl and colleagues under similar conditions. The levels of the five cytokines measured were 7-13-fold higher for 19-3 BiTE, compared to those of (19)-3s. The use of foreign lymphoma cells (Raji) caused a mixed lymphocyte reaction, which increased the baseline cytokine levels, particularly for IL-2. BiTE, but not (19)-3s, increased the cytokine levels well above the mixed lymphocyte baseline level. Using NCI-N87 gastric carcinoma cells as the target for (E1)-3s did not increase baseline cytokine levels. We observed an expected variability in donor response to (E1)-3s; however, the resulting cytokine levels were even lower than those induced by (19)-3s, particularly for TNF-α and IFN-γ, which were <100 pg/mL. Nevertheless, one of five donors had elevated levels (~1 ng/mL) of IFN-γ and IL-6. Addition of IFN-α (peginterferonalfa-2a) to (E1)-3s increased IFN-γ 2-3-fold, but did not consistently affect the levels of the other cytokines. These results suggest that compared to other constructs, such as BiTE, the (X)-3s bsAb format is less likely to induce CRS, and the addition of IFN-α to a therapeutic regimen does not increase this risk.

We observed considerable variability in the potency of donor T cells. The *in vitro* results shown in **FIG. 28** represent the most and least active T cells that we have tested, with a 100-fold difference in potency (IC₅₀ = 0.37 pM *vs.* 39 pM) for killing NCI-N87; however, an IC₅₀ = 1-5 pM is most representative (>10 donors) and the low-activity T cells was atypical. Notably, lysis with the weaker T cells was augmented by IFN-α more than with the potent T cells.

EpCAM is a widely exploited TAA that is overexpressed in many carcinomas. However, the heterogeneous expression of EpCAM in carcinomas and the fact that EpCAM is not tumor-specific, since it is expressed on most normal epithelia, raise concerns that immunotherapy directed towards EpCAM could have severe side effects (Balzar et al., 1999, J Mol Med (Berl) 77:699-712; Momburg et al., 1987, Cancer Res 47:2883-91). Like EpCAM, Trop-2 is highly expressed in diverse carcinomas, but its expression in normal tissues is under debate. Several reports indicate that, in contrast to tumor cells, somatic adult tissues show little or no Trop-2 expression, which is invariably upregulated in tumors, regardless of baseline expression in normal tissues (Wang et al., 2008, Mol Cancer Ther 7:280-5; Zhang et al., 1997, Science 276:1268-72). However, recent evidence indicates expression of Trop-2 on epithelia of several normal tissues (Trerotola et al., 2013, Oncogene 32:222-33). Nonetheless, expression of Trop-2 in Cynomolgus monkeys did not result in toxicities after administrations of reasonably high doses of hRS7 (humanized anti-Trop-2) conjugated with SN-38 as an antibody-drug conjugate (ADC) (Cardillo et al., 2011, Clin Cancer Res 17:3157-69). Further, in clinical studies with this anti-Trop-2 ADC, no increased normal organ toxicity other than manageable neutropenia and diarrhea, expected from the drug (a metabolite of irinotecan), was observed at therapeutic doses (Starodub et al., Proceedings of the 105th Annual Meeting of the American Association for Cancer Research. 2014 (abstr CT206)). Thus, immunotherapy, including T-cell-redirected therapy, using Trop-2 for tumor targeting, is expected to have a similar, or greater, therapeutic index compared to similar regimens targeting EpCAM.

This is the first report of trogocytosis between target tumor and T cells mediated by a bsAb. This finding demonstrates that the target/T-cell conjugates induced with (E1)-3s have functional immunologic synapses. We observed a similar bi-directional trogocytosis between B cells and T cells, which was mediated by (19)-3s (unpublished data), and believe this is likely a common phenomenon with T-cell redirecting bsAbs.

### Example 27. Further Studies With E1-3 Bispecific Antibodies

### Summary

A T-cell redirecting bispecific tandem scFv, E1-3, was produced as described in Example 19 above, using the variable domains of hRS7 (humanized anti-Trop-2 mAb) and Okt-3 (anti-CD3 mAb). The studies reported in this Example continue and expand on the results shown in Examples 20-25. Any discrepancies between the instant reported results and those shown in Examples 20-25 are based on the collection of additional data. T-cell activation, proliferation, cytokine induction and cytotoxicity were evaluated *ex vivo* using PBMCs or purified T cells with human pancreatic (Capan-1 and BxPC-3) and gastric (NCI-N87) cancer cell lines as target cells. *In vivo* activity was assayed with NCI-N87 xenografts that were inoculated s.c. in a mixture with twice the number of human PBMCs and matrigel.

### Results

In the presence of target cells and PBMCs, E1-3 potently induced T-cell activation, proliferation and a dose-dependent cytokine production of IL-2 (>2 ng/mL), IL-6 (>1 ng/mL), IL-10 (>7 ng/mL), TNF-α (>1 ng/mL) and IFN-γ (>50 ng/mL). Using 3 - 5 different T cell donors, E1-3 mediated a highly potent T-cell lysis of BxPC-3 [IC₅₀=0.09(±0.04) pM], Capan-1 [IC₅₀=1.2(±1.1) pM] and NCI-N87 [IC₅₀=1.2(±1.2) pM] target cells *in vitro. In vivo,* two 50-µg doses of E1-3 given three days apart cured 6 of 8 mice bearing NCI-N87 xenografts (*P*<0.0001; Log-Rank). Tumors in the control group (PBMCs only) reached the endpoint (TV>1 cm³) with a median of 39.5 days. Seven of 8 animals had not reached the endpoint, with six of the mice remaining tumor-free in the E1-3 group when the experiment was terminated after 176 days.

T-cell activation and proliferation - Purified CD8⁺ T cells were mixed 5:1 with NCI-N87 cells, treated for 18 h with 0.01 nM E1-3 and analyzed by flow cytometry. CD69 was upregulated by E1-3 in the presence of target cells (not shown). Treatments with omission of E1-3 or NCI-N87 target cells did not induce CD69 expression (not shown). Additionally, T cells experienced an increase in forward (FSC) and side scattering (SSC) after culture in the presence of E1-3 and target cells (not shown). T-cell proliferation was evident after three days (*P*<0.005, data not shown).

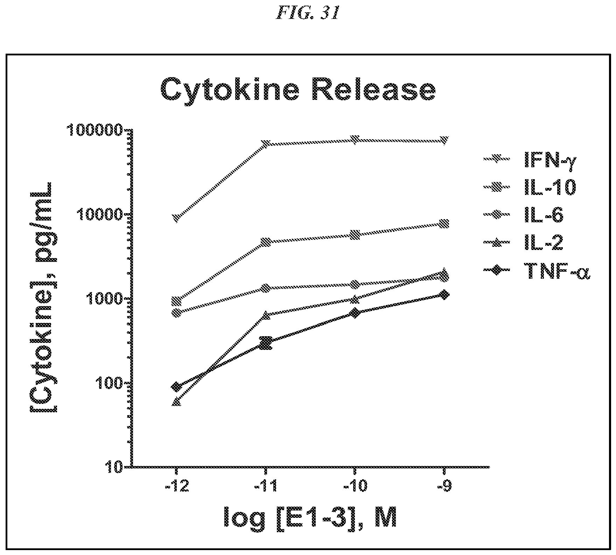

Cytokine release - The ability of E1-3 bispecific tandem scFv to induce release of cytokines IFN-γ, TNF-α, IL-2, IL-6 and IL-10 as a function of dosage was determined. As shown in **FIG. 31****,** the E1-3 bispecific antibody effectively induced cytokine release in the picomolar concentration range.

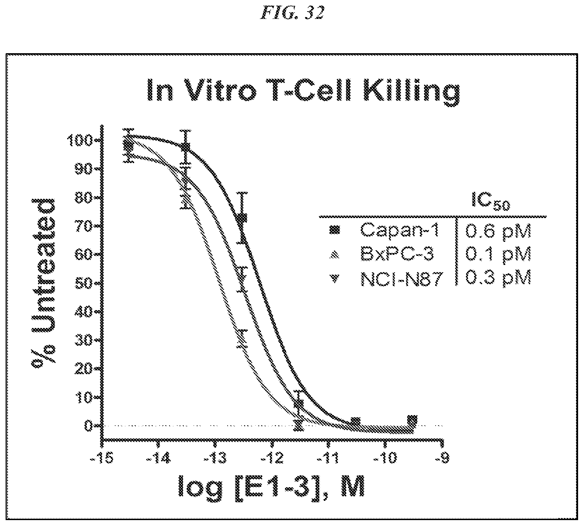

In vitro T-cell mediated killing - The ability of E1-3 to induce T-cell mediated killing of target pancreatic and gastric cancer cells was determined in the presence of purified CD8⁺ T cells (1.2 × 10⁵/well). An exemplary dose-response curve using T-cells from a representative donor are shown in **FIG. 32****.** In this experiment, the IC₅₀ values for E1-3 were 0.6 pM for Capan-1, 0.1 pM for BxPC-3 and 0.3 pM for NCI-N87.

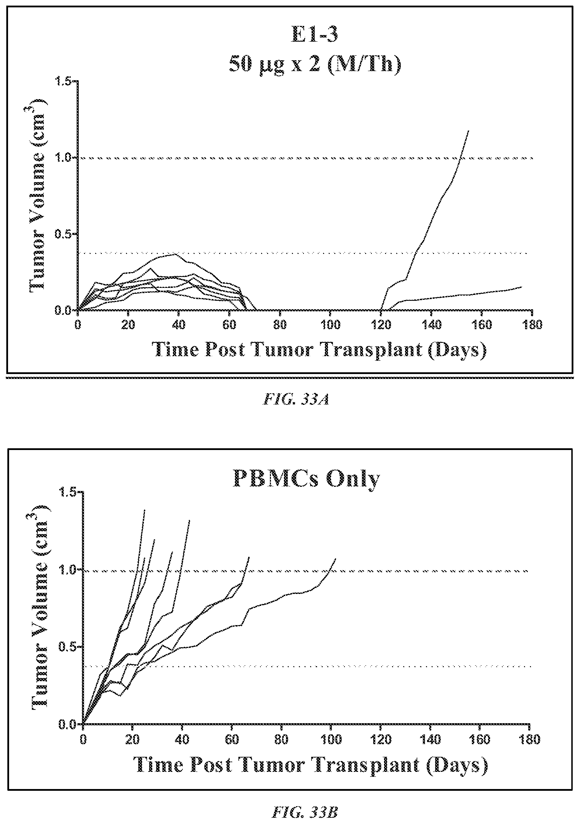

In vivo anti-tumor effects of E1-3 - Nude mice bearing NCI-N87 xenografts were treated with two 50-µg doses of E1-3 given three days apart. The treatment (**FIG. 33A**) cured 6 of 8 mice bearing the human gastric cancer xenografts (*P*<0.0001; Log-Rank). In comparison with tumors in the control group (treated with PBMCs only) reached the endpoint (TV>1 cm³) with a median of 39.5 days (**FIG. 33B**). When the study was terminated after 176 days, seven of eight animals in the E1-3 group had not reached the endpoint.

### Conclusions

The studies above show that Trop-2 is an attractive target for T-cell-mediated killing of pancreatic, gastric and other epithelial cancers. The E1-3 anti-Trop-2 × anti-CD3 bispecific antibody induced potent T-cell activation and cytokine production. E1-3 was highly effective at killing solid tumors *in vitro* and *in vivo.*

## Claims

1. A bispecific antibody for use in a method of treatment of Trop-2 expressing cancer by inducing an immune response to a Trop-2 expressing cancer, wherein the method comprises administering the bispecific antibody and a checkpoint inhibitor antibody to a subject with a Trop-2 expressing cancer, wherein the bispecific antibody comprises a first antibody fragment and a second antibody fragment, wherein both the first antibody fragment and the second antibody fragment are each an scFv and, wherein the bispecific antibody comprises one binding site for Trop-2 and one binding site for CD3, and wherein the bispecific antibody comprises the amino acid sequence of SEQ ID NO: 107 binding to Trop-2.

2. The bispecific antibody for use according to claim 1, wherein the method further comprises administering to the subject at least one therapeutic agent selected from the group consisting of (i) an interferon selected from the group consisting of interferon-α, interferon-β, interferon-λ1, interferon-λ2 and interferon-λ3; and (ii) an antibody-drug conjugate (ADC).

3. The bispecific antibody for use according to claim 2, wherein the interferon is interferon-α.

4. The bispecific antibody for use according to claim 1, wherein the checkpoint inhibitor antibody is selected from the group consisting of lambrolizumab (MK-3475), nivolumab (BMS-936558), pidilizumab (CT-011), AMP-224, MDX-1105, MEDI4736, MPDL3280A, BMS-936559, ipilimumab, lirlumab, IPH2101 and tremelimumab.

5. The bispecific antibody for use according to claim 1, wherein the checkpoint inhibitor antibody binds to an antigen selected from the group consisting of CTLA4, PD1, PD-L1, LAG3, B7-H3, B7-H4, KIR and TIM3.

6. The bispecific antibody for use according to claim 2, wherein the antibody-drug conjugate is selected from the group consisting of hLL1-doxorubicin, hRS7-SN-38, hMN-14-SN-38, hLL2-SN-38, hA20-SN-38, hPAM4-SN-38, hLL1-SN-38, hRS7-Pro-2-P-Dox, hMN-14-Pro-2-P-Dox, hLL2-Pro-2-P-Dox, hA20-Pro-2-P-Dox, hPAM4-Pro-2-P-Dox, hLL1-Pro-2-P-Dox, P4/D10-doxorubicin, gemtuzumab ozogamicin, brentuximab vedotin, trastuzumab emtansine, inotuzumab ozogamicin, glembatumomab vedotin, SAR3419, SAR566658, BIIB015, BT062, SGN-75, SGN-CD19A, AMG-172, AMG-595, BAY-94-9343, ASG-5ME, ASG-22ME, ASG-16M8F, MDX-1203, MLN-0264, anti-PSMA ADC, RG-7450, RG-7458, RG-7593, RG-7596, RG-7598, RG-7599, RG-7600, RG-7636, ABT-414, IMGN-853, IMGN-529, vorsetuzumab mafodotin, and lorvotuzumab mertansine.

7. The bispecific antibody for use according to claim 2, wherein an ADC is administered prior to any other agent.

8. The bispecific antibody for use according to claim 3, wherein the interferon is administered as free interferon, PEGylated interferon, an interferon fusion protein or interferon conjugated to an antibody.

9. The bispecific antibody for use according to claim 1, wherein the Trop-2 expressing cancer is a carcinoma of the esophagus, pancreas, lung, stomach, colon, rectum, urinary bladder, breast, ovary, uterus, kidney or prostate.

10. The bispecific antibody for use according to claim 1, wherein the method further comprises administering to the subject a therapeutic agent selected from the group consisting of a second antibody or antigen-binding fragment thereof, a drug, a toxin, an enzyme, a cytotoxic agent, an anti-angiogenic agent, a pro-apoptotic agent, an antibiotic, a hormone, an immunomodulator, a cytokine, a chemokine, an antisense oligonucleotide, a small interfering RNA (siRNA), a boron compound and a radioisotope.

11. The bispecific antibody for use according to claim 1, wherein the bispecific antibody induces an immune response to a Trop-2 expressing cancer without increasing cytokine production to levels capable of inducing cytokine release syndrome (CRS).

## Patentansprüche

1. Bispezifischer Antikörper zur Verwendung in einem Verfahren zum Behandeln von Trop-2-exprimierenden Krebs durch Induzieren einer Immunantwort gegen einen Trop-2-exprimierenden Krebs, wobei das Verfahren umfasst Verabreichen des bispezifischen Antikörpers und eines Checkpoint-Inhibitor-Antikörpers an ein Lebewesen mit einem Trop-2-exprimierenden Krebs, wobei der bispezifische Antikörper ein erstes Antikörperfragment und ein zweites Antikörperfragment umfasst, wobei sowohl das erste Antikörperfragment als auch das zweite Antikörperfragment jeweils ein scFv ist und wobei der bispezifische Antikörper eine Bindungsstelle für Trop-2 und eine Bindungsstelle für CD3 umfasst, und wobei der bispezifische Antikörper die an Trop-2 bindende Aminosäuresequenz gemäß SEQ ID NO: 107 umfasst.

2. Bispezifischer Antikörper zur Verwendung nach Anspruch 1, wobei das Verfahren weiter umfasst Verabreichen an das Lebewesen von wenigstens einem therapeutischen Agens, das ausgewählt ist aus der Gruppe bestehend aus (i) einem Interferon, das ausgewählt ist aus der Gruppe bestehend aus Interferon-α, Interferon-β, Interferon-λ1, Interferon-λ2 und Interferon-λ3; und (ii) einem Antikörper-Wirkstoff-Konjugat (ADC).

3. Bispezifischer Antikörper zur Verwendung nach Anspruch 2, wobei das Interferon Interferon-α ist.

4. Bispezifischer Antikörper zur Verwendung nach Anspruch 1, wobei der Checkpoint-Inhibitor-Antikörper ausgewählt ist aus der Gruppe bestehend aus Lambrolizumab (MK-3475), Nivolumab (BMS-936558), Pidilizumab (CT-011), AMP-224, MDX-1105, MEDI4736, MPDL3280A, BMS-936559, Ipilimumab, Lirlumab, IPH2101 und Tremelimumab.

5. Bispezifischer Antikörper zur Verwendung nach Anspruch 1, wobei der Checkpoint-Inhibitor-Antikörper an ein Antigen bindet, das ausgewählt ist aus der Gruppe bestehend aus CTLA4, PD1, PD-L1, LAG3, B7-H3, B7-H4, KIR und TIM3.

6. Bispezifischer Antikörper zur Verwendung nach Anspruch 2, wobei das Antikörper-Wirkstoff-Konjugat ausgewählt ist aus der Gruppe bestehend aus hLL1-Doxorubicin, hRS7-SN-38, hMN-14-SN-38, hLL2-SN-38, hA20-SN-38, hPAM4-SN-38, hLL1-SN-38, hRS7-Pro-2-P-Dox, hMN-14-Pro-2-P-Dox, hLL2-Pro-2-P-Dox, hA20-Pro-2-P-Dox, hPAM4-Pro-2-P-Dox, hLL1-Pro-2-P-Dox, P4/D10-Doxorubicin, Gemtuzumab-Ozogamicin, Brentuximab-Vedotin, Trastuzumab-Emtansin, Inotuzumab-Ozogamicin, Glembatumomab-Vedotin, SAR3419, SAR566658, BIIB015, BT062, SGN-75, SGN-CD19A, AMG-172, AMG-595, BAY-94-9343, ASG-5ME, ASG-22ME, ASG-16M8F, MDX-1203, MLN-0264, anti-PSMA ADC, RG-7450, RG-7458, RG-7593, RG-7596, RG-7598, RG-7599, RG-7600, RG-7636, ABT-414, IMGN-853, IMGN-529, Vorsetuzumab-Mafodotin und Lorvotuzumab-Mertansin.

7. Bispezifischer Antikörper zur Verwendung nach Anspruch 2, wobei ein ADC vor einem jeglichen anderen Agens verabreicht wird.

8. Bispezifischer Antikörper zur Verwendung nach Anspruch 3, wobei das Interferon als freies Interferon, PEGyliertes Interferon, ein Interferon-Fusionsprotein oder an einen Antikörper konjugiertes Interferon verabreicht wird.

9. Bispezifischer Antikörper zur Verwendung nach Anspruch 1, wobei der Trop-2-exprimierende Krebs ein Karzinom des Ösophagus, des Pankreas, der Lunge, des Magens, des Kolons, des Rektums, der Harnblase, der Brust, des Eierstocks, der Gebärmutter, der Niere oder der Prostata ist.

10. Bispezifischer Antikörper zur Verwendung nach Anspruch 1, wobei das Verfahren weiter umfasst Verabreichen an das Lebewesen eines therapeutischen Agens, das ausgewählt ist aus der Gruppe bestehend aus einem zweiten Antikörper oder einem Antigen-bindenden Fragment davon, einem Wirkstoff, einem Toxin, einem Enzym, einem cytotoxischen Agens, einem anti-angiogenen Agens, einem pro-apoptotischen Agens, einem Antibiotikum, einem Hormon, einem Immunmodulator, einem Cytokin, einem Chemokin, einem Antisinn-Oligonukleotid, einer kleinen interferierenden RNA (siRNA), einer Borverbindung und einem Radioisotop.

11. Bispezifischer Antikörper zur Verwendung nach Anspruch 1, wobei der bispezifische Antikörper eine Immunantwort gegen einen Trop-2-exprimierenden Krebs ohne Erhöhen der Cytokinproduktion auf Niveaus induziert, die in der Lage sind, Cytokinfreisetzungssyndrom (CRS) zu induzieren.

## Revendications

1. Anticorps bispécifique pour son utilisation dans un procédé de traitement d'un cancer exprimant le Trop-2 par induction d'une réponse immunitaire à un cancer exprimant le Trop-2, dans lequel le procédé comprend l'administration de l'anticorps bispécifique et d'un anticorps inhibiteur de point de contrôle à un sujet présentant un cancer exprimant le Trop-2, dans lequel l'anticorps bispécifique comprend un premier fragment d'anticorps et un second fragment d'anticorps, dans lequel le premier fragment d'anticorps et le second fragment d'anticorps sont chacun un scFv et, dans lequel l'anticorps bispécifique comprend un site de liaison pour le Trop-2 et un site de liaison pour le CD3, et dans lequel l'anticorps bispécifique comprend la séquence d'acides aminés de SEQ ID NO : 107 se liant au Trop-2.

2. Anticorps bispécifique pour son utilisation selon la revendication 1, dans lequel le procédé comprend en outre l'administration au sujet d'au moins un agent thérapeutique choisi dans le groupe constitué de (i) un interféron choisi dans le groupe constitué de l'interféron-α, de l'interféron-β, de l'interféron-λ1, de l'interférant et de l'interféron-λ3 ; et (ii) un conjugué anticorps-médicament (ADC).

3. Anticorps bispécifique pour son utilisation selon la revendication 2, dans lequel l'interféron est l'interféron-α.

4. Anticorps bispécifique pour son utilisation selon la revendication 1, dans lequel l'anticorps inhibiteur de point de contrôle est choisi dans le groupe constitué du lambrolizumab (MK-3475), du nivolumab (BMS-936558), du pidilizumab (CT-011), de l'AMP-224, du MDX-1105, du MEDI4736, du MPDL3280A, du BMS-936559, de l'ipilimumab, du lirlumab, de l'IPH2101 et du trémélimumab.

5. Anticorps bispécifique pour son utilisation selon la revendication 1, dans lequel l'anticorps inhibiteur de point de contrôle se lie à un antigène choisi dans le groupe constitué de CTLA4, PD1, PD-L1, LAG3, B7-H3, B7-H4, KIR et TIM3.

6. Anticorps bispécifique pour son utilisation selon la revendication 2, dans lequel le conjugué anticorps-médicament est choisi dans le groupe constitué de hLL1-doxorubicine, hRS7-SN-38, hMN-14-SN-38, hLL2-SN-38, hA20-SN-38, hPAM4-SN-38, hLL1-SN-38, hRS7-Pro-2-P-Dox, hMN-14-Pro-2-P-Dox, hLL2-Pro-2-P-Dox, hA20-Pro-2-P-Dox, hPAM4-Pro-2-P-Dox, hLL1-Pro-2-P-Dox, P4/D10-doxorubicine, gemtuzumab ozogamicine, brentuximab védotine, trastuzumab emtansine, inotuzumab ozogamicine, glembatumomab védotine, SAR3419, SAR566658, BIIB015, BT062, SGN-75, SGN-CD19A, AMG-172, AMG-595, BAY-94-9343, ASG-5ME, ASG-22ME, ASG-16M8F, MDX-1203, MLN-0264, anti-PSMA ADC, RG-7450, RG-7458, RG-7593, RG-7596, RG-7598, RG-7599, RG-7600, RG-7636, ABT-414, IMGN-853, IMGN-529, vorsetuzumab mafodotine, et lorvotuzumab mertansine.

7. Anticorps bispécifique pour son utilisation selon la revendication 2, dans lequel un ADC est administré avant tout autre agent.

8. Anticorps bispécifique pour son utilisation selon la revendication 3, dans lequel l'interféron est administré sous forme d'un interféron libre, d'un interféron PEGylé, d'une protéine de fusion d'interféron ou d'un interféron conjugué à un anticorps.

9. Anticorps bispécifique pour son utilisation selon la revendication 1, dans lequel le cancer exprimant le Trop-2 est un carcinome de l'oesophage, du pancréas, du poumon, de l'estomac, du côlon, du rectum, de la vessie, du sein, de l'ovaire, de l'utérus, du rein ou de la prostate.

10. Anticorps bispécifique pour son utilisation selon la revendication 1, dans lequel le procédé comprend en outre l'administration au sujet d'un agent thérapeutique choisi dans le groupe constitué d'un second anticorps ou d'un fragment de liaison à l'antigène de celui-ci, d'un médicament, d'une toxine, d'une enzyme, d'un agent cytotoxique, d'un agent anti-angiogénique, d'un agent pro-apoptotique, d'un antibiotique, d'une hormone, d'un immunomodulateur, d'une cytokine, d'une chimiokine, d'un oligonucléotide antisens, d'un petit ARN interférent (siRNA), d'un composé de bore et d'un radio-isotope.

11. Anticorps bispécifique pour son utilisation selon la revendication 1, dans lequel l'anticorps bispécifique induit une réponse immunitaire contre un cancer exprimant le Trop-2 sans augmenter la production de cytokines à des niveaux capables d'induire le syndrome de libération de cytokines (CRS).
